# EUROPEAN PATENT APPLICATION

(11) **EP 4 450 512 A1**
(43) Date of publication of application: **23.10.2024**
(21) Application number: 22907560.1
(22) Date of filing: 16.12.2022
(51) Int. Cl.: C07H 21/04, C07H 19/067, C07H 19/073, C12N 15/10

(54) **OLIGONUCLEOTIDE PRODUCTION METHOD**

(30) Priority: 17.12.2021 JP 2021205491
(71) Applicant: LIID Pharmaceuticals, Inc., Suita-shi, Osaka 564-8565 (JP)
(72) Inventor: HARI, Yoshiyuki, Tokushima-shi, Tokushima 770-8514 (JP); FUCHI, Yasufumi, Tokushima-shi, Tokushima 770-8514 (JP); ITO, Yuta, Tokushima-shi, Tokushima 770-8514 (JP); YAMAMOTO, Kazuki, Tokushima-shi, Tokushima 770-8514 (JP)
(74) Representative: Weickmann & Weickmann PartmbB
(86) International application number: PCT/JP2022/046549
(87) International publication number: WO 2023/113038

(57) **Abstract**

As a solid phase synthesis method of an oligonucleotide that enables cleavage of a synthesized oligonucleotide even under mild conditions, a solid phase synthesis method of an oligonucleotide, including a step of carrying a nucleotide derivative represented by the formula (I): 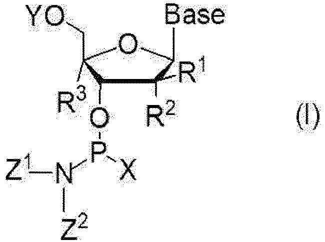 wherein each symbol is as described in the specification,
on a solid phase support via a universal linker, and the like are disclosed.

## Description

### [Technical Field]

The present invention relates to a solid phase synthesis method of an oligonucleotide that enables cleavage of a synthesized oligonucleotide even under mild conditions by using a nucleoside phosphoramidite with a specific structure, and the like.

### [Background Art]

Solid phase synthesis methods using the phosphoramidite method have been widely used for chemical synthesis of oligonucleotides (nucleic acids) such as DNA and RNA. At the first stage of the synthesis cycle, however, the 3'-terminal nucleoside of the oligonucleotide (nucleic acid) sequence to be synthesized needs to be carried on a solid phase support.

In this case, there is a method including preparing, depending on the nucleoside that becomes the 3'-terminus of the oligonucleotide to be synthesized, a solid phase support on which the nucleoside is carried in advance and performing the subsequent nucleotide elongation reaction. However, it is complicated to prepare each time a solid phase support on which the 3'-terminal nucleoside of the oligonucleotide to be synthesized is carried in advance. On the other hand, when a solid phase support carrying a universal linker capable of coupling with any nucleoside (e.g., Patent Literature 1, Patent Literature 2) is used, the nucleoside at the 3'-terminal of oligonucleotide can be freely introduced as the first nucleoside in the step of elongating the oligonucleotide onto the universal linker, regardless of the sequence of the oligonucleotide to be synthesized, and an oligonucleotides with any sequence can be effectively synthesized regardless of the nucleoside at the 3'-terminal. Therefore, such universal linkers are widely used in solid phase synthesis.

More specifically, an universal linker is carried in advance on a solid phase support via a cleavable linker (spacer) such as succinyl group and the like, and any nucleoside at the 3'-terminus is coupled to the universal linker, after which an oligonucleotide elongation reaction generally including the following steps:
(1) a step of deprotecting the 5'-OH group of the protected nucleoside with an acid such as a trichloroacetic acid/dichloromethane solution;
(2) a step of coupling a nucleoside phosphoramidite (to be also denoted as "nucleic acid monomer") to the deprotected 5'-OH group in the presence of an activator (tetrazole, etc.);
(3) a step of capping an unreacted 5'-OH group with acetic anhydride or the like; and
(4) a step of oxidizing phosphite with aqueous iodine, or sulfurizing phosphite with 3-((N,N-dimethylaminomethylidene)amino)-3H-1,2,4-dithiazole-5-thione and the like:
   is performed in a reaction column, according to the synthesis program of an automatic nucleic acid synthesizer.

By repeating the above synthesis cycle and proceeding with the elongation reaction of the oligonucleotide from the 3'-terminus to the 5'-terminus, an oligonucleotide (nucleic acid) having the desired sequence is synthesized.

Finally, the cleavable linker is hydrolyzed with aqueous ammonia, methylamine solution, and the like, and the synthesized oligonucleotide (nucleic acid) is cleaved from the solid phase support and the universal linker.

### [Citation List]

### [Patent Literature]

[Patent Literature 1]
   US Patent No. 8541599
[Patent Literature 2]
   US Patent Application Publication No. 2004/0152905

### [Summary of Invention]

### [Technical Problem]

The nucleoside phosphoramidites (nucleic acid monomers) widely used in the above-mentioned conventional solid phase phosphoramidite methods are a group of nucleoside phosphoramidites represented by a compound shown by the following formula (A): 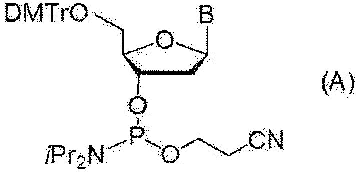 wherein B is an optionally modified nucleobase. The feature here is that the hydroxyl group on the phosphorus atom is protected by a "cyanoethyl group".

However, when a widely used nucleoside phosphoramidite is used for coupling with a universal linker, in the final step of solid phase synthesis in which the cleavable linker is hydrolyzed with aqueous ammonia or methylamine solution and the synthesized oligonucleotide is separated from the solid phase support and the universal linker, strongly basic conditions such as "treatment with concentrated aqueous ammonia, 55°C, 8 hr" are often required. It has thus been difficult to use modified nucleic acids and functional molecules, that are vulnerable to basic conditions, in oligonucleotide (nucleic acid) synthesis. For this reason, the development of a solid phase synthesis method of oligonucleotides (nucleic acids) that enables cleavage of synthesized oligonucleotides (nucleic acids) even under milder conditions is awaited.

### [Solution to Problem]

The present inventors have conducted intensive studies in an attempt to solve the aforementioned problems and found that the above-mentioned problems can be solved by converting the "cyanoethyl group" used as a hydroxy-protecting group on the phosphorus atom for the widely-used nucleoside phosphoramidite to an alkoxy group and the like, and created a solid phase oligonucleotide (nucleic acid) synthesis method that enables cleavage of synthesized nucleic acid even under mild conditions.

Furthermore, they have also found that, in the solid phase oligonucleotide (nucleic acid) synthesis method of the present invention, a linker with a simpler structure can also be used as a universal linker in place of the linkers widely used at present.

That is, one embodiment of the present invention is, in a solid phase synthesis of an oligonucleotide by using a universal linker, a solid phase synthesis method of an oligonucleotide, including coupling, as a nucleoside phosphoramidite for introducing a 3'-terminal nucleoside, a nucleotide derivative represented by the following formula (I): 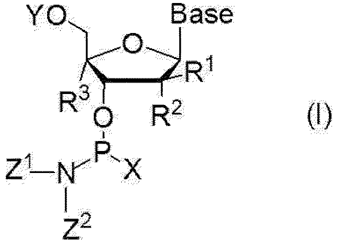
wherein X is an optionally substituted alkoxy group (excluding a cyanoethoxy group),
an optionally substituted cyclic group-oxy group,
an optionally substituted alkyl group,
an optionally substituted alkenyl group, or
an optionally substituted aryl group;
Y is a hydrogen atom or a hydroxy-protecting group;
Z¹ and Z² are each independently an optionally substituted alkyl group, or
Z¹ and Z² are bonded to each other to form, together with a nitrogen atom bonded thereto, an optionally substituted heterocycle;
R¹ is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R² is a hydrogen atom, a halogen atom, an optionally protected hydroxy group, an optionally substituted alkyl group, an optionally substituted alkoxy group, an optionally substituted alkenyloxy group, or an optionally substituted alkynyloxy group, and
R³ is a hydrogen atom, an optionally substituted alkyl group,
an optionally substituted alkoxy group, or an optionally substituted alkylthio group, or
R² and R³ are bonded to each other to form a group represented by the following formula (shown in the order of -R²-R³-) :
   -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
   in the above-mentioned formulas, Ra to Rj are each independently a hydrogen atom or an optionally substituted alkyl group; and
   Base is an optionally modified nucleobase
   (hereinafter to be also denoted as "nucleotide derivative (I)") first with a universal linker, performing a subsequent oligonucleotide elongation reaction to obtain an oligonucleotide having, at the 3'-terminus of an oligonucleotide sequence thereof, a nucleotide derivative represented by the formula (II): 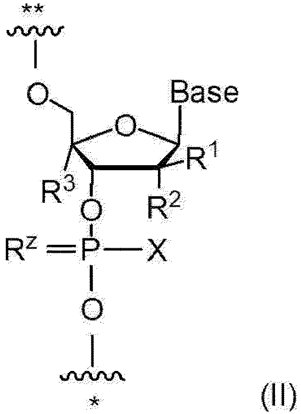
   wherein * is a bonding position to the universal linker;
   ** is a bonding position to the nucleotide or oligonucleotide; R^{z} is 0 or S; and
   X, R¹ to R³, and Base are as defined above
   (hereinafter to be also denoted as "nucleotide derivative residue (II)"), and cleaving the obtained oligonucleotide from the solid phase support and the universal linker.

More specific embodiments of the present invention are shown in the following.
[1] A solid phase synthesis method of an oligonucleotide, comprising a step of subjecting an oligonucleotide having, at the 3'-terminus of an oligonucleotide sequence thereof, a nucleotide derivative represented by the formula (II): 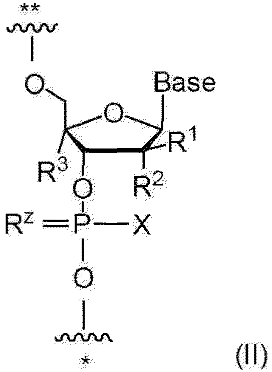
   wherein * is a bonding position to the universal linker;
   ** is a bonding position to the nucleotide or oligonucleotide; R^{z} is 0 or S; and
   X, R¹ to R³, and Base are as defined above,
   which oligonucleotide is carried on a solid phase support via a universal linker and synthesized by a nucleotide elongation reaction in a solid phase synthesis method of an oligonucleotide,
   to a reaction for cleaving the oligonucleotide from the solid phase support and the universal linker.
[2] A solid phase synthesis method of an oligonucleotide, comprising
   (1) a step of carrying a nucleotide derivative represented by the formula (I): 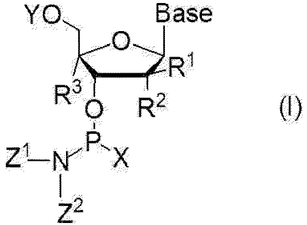
      wherein X, Y, Z¹, Z², R¹ to R³ and Base are each as defined above
      on a solid phase support via a universal linker,
   (2) a step of sequentially condensing nucleic acid monomers according to the desired oligonucleotide sequence to elongate the nucleotide (one or two or more nucleotide derivatives represented by the above-mentioned formula (I) may also be used as the nucleic acid monomers),
   (3) a step of subjecting the obtained oligonucleotide having the desired sequence to a reaction for cleaving from the solid phase support and the universal linker.
[3] The solid phase synthesis method of an oligonucleotide of the above-mentioned [1] or [2], wherein the reaction for cleaving from the solid phase support and the universal linker is performed by contacting the solid phase support, on which the oligonucleotide is carried via a universal linker, with
   1) concentrated aqueous ammonia at room temperature to under warming,
   2) a mixed solution of concentrated alkylamine aqueous solution/concentrated aqueous ammonia at room temperature to under warming,
   3) an alcohol solution of potassium carbonate at room temperature to under warming, or
   4) a mixed solution of alkylamine/water or alkylamine/water/alcohol at room temperature to under warming.
[4] The solid phase synthesis method of an oligonucleotide of the above-mentioned [3], wherein the reaction for cleaving from the solid phase support is performed by contacting the solid phase support, on which the oligonucleotide is carried via a universal linker, with
   1) 28% aqueous ammonia at room temperature,
   2) a 1:1 mixed solution of 40% methylamine aqueous solution/28% aqueous ammonia (AMA solution) at room temperature,
   3) a methanol solution of 50 mM potassium carbonate at room temperature, or
   4) a mixed solution of t-butylamine/water or t-butylamine/water/methanol under warming.
[5] A nucleotide derivative represented by the formula (Ia): 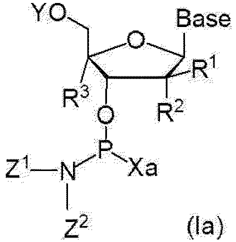
   wherein Xa is an optionally substituted (C₄-C₅)alkoxy group, and
   Y, Z¹, Z², R¹ to R³ and Base are each as defined above.
[6] Use of a 1,2-diol derivative represented by the formula (III) :

   Y¹-O-C (X¹) (X²) C (X³) (X⁴) -O-Y² (III)

   wherein X¹ to X⁴ are each independently a hydrogen atom or a chain or cyclic substituent, wherein any two groups of X¹ to X⁴ are optionally bonded to each other at a bondable position to form, together with a carbon atom bonded thereto, an optionally substituted ring; and
   Y¹ and Y² are each a hydrogen atom or a hydroxy-protecting group,
   as a universal linker for oligonucleotide solid phase synthesis.

### [Advantageous Effects of Invention]

According to the present invention, a solid phase synthesis method of an oligonucleotide that enables cleavage of a synthesized oligonucleotide (nucleic acid) even under mild conditions, a novel nucleoside phosphoramidite useful for practicing the solid phase synthesis method of an oligonucleotide of the present invention, and a universal linker with a simple structure useful for practicing the solid phase synthesis method of an oligonucleotide of the present invention are disclosed.

### [Brief Description of Drawings]

[Fig. 1]

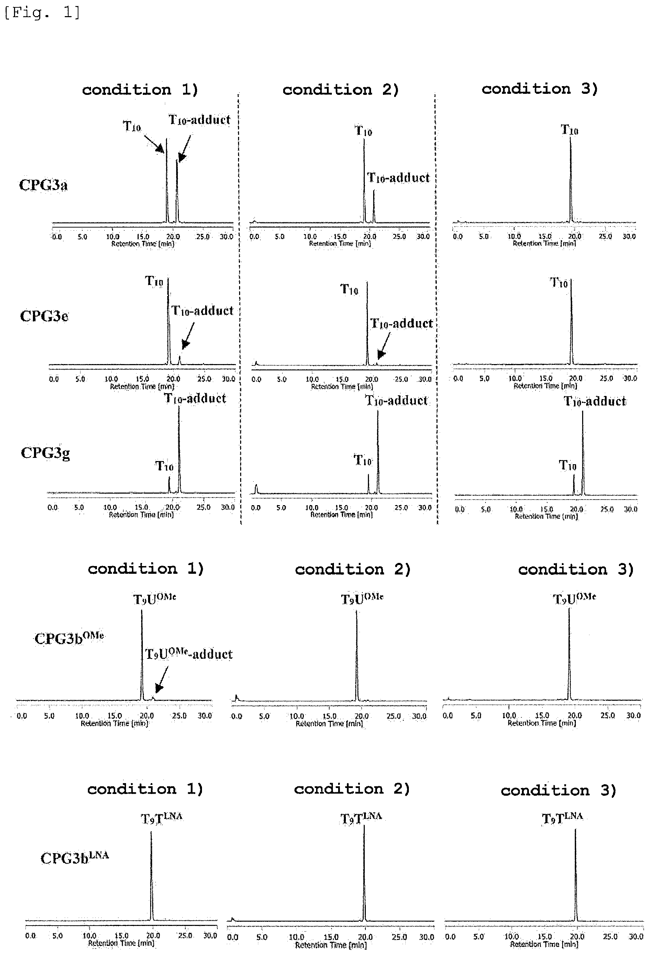

   Fig. 1 shows HPLC charts showing the results of cleavage of oligonucleotides from CPG3a, CPG3e, CPG3g, CPG3b^{OMe} and CPG3b^{LNA} (conditions 1) to 3)) in Example D1 described later.
[Fig. 2]

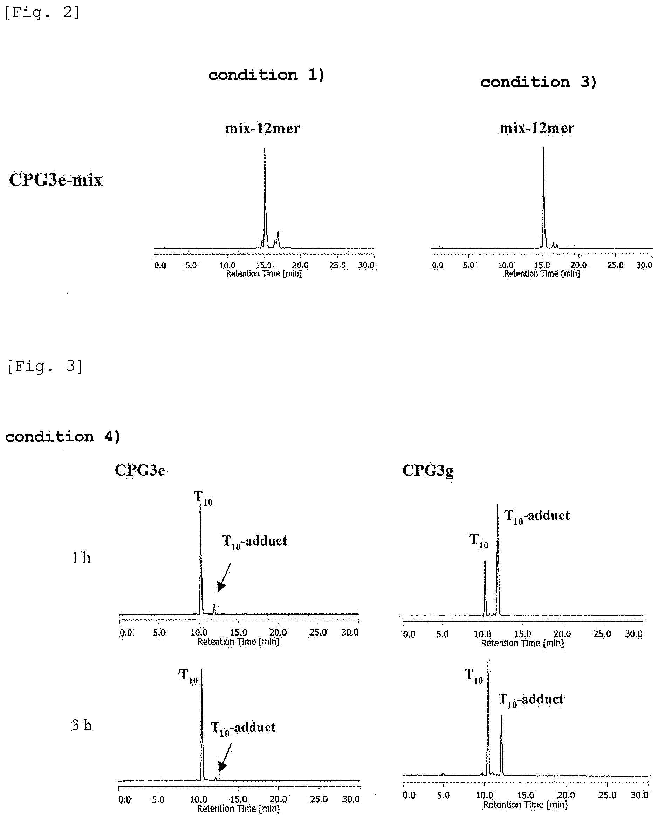

   Fig. 2 shows HPLC charts showing the results of cleavage of oligonucleotides from CPG3e-mix (condition 1) and condition 3)) in Example D1 described later.
[Fig. 3]
   Fig. 3 shows HPLC charts showing the results of cleavage of oligonucleotides from CPG3e and CPG3g (condition 4)) in Example D1 described later.
[Fig. 4]

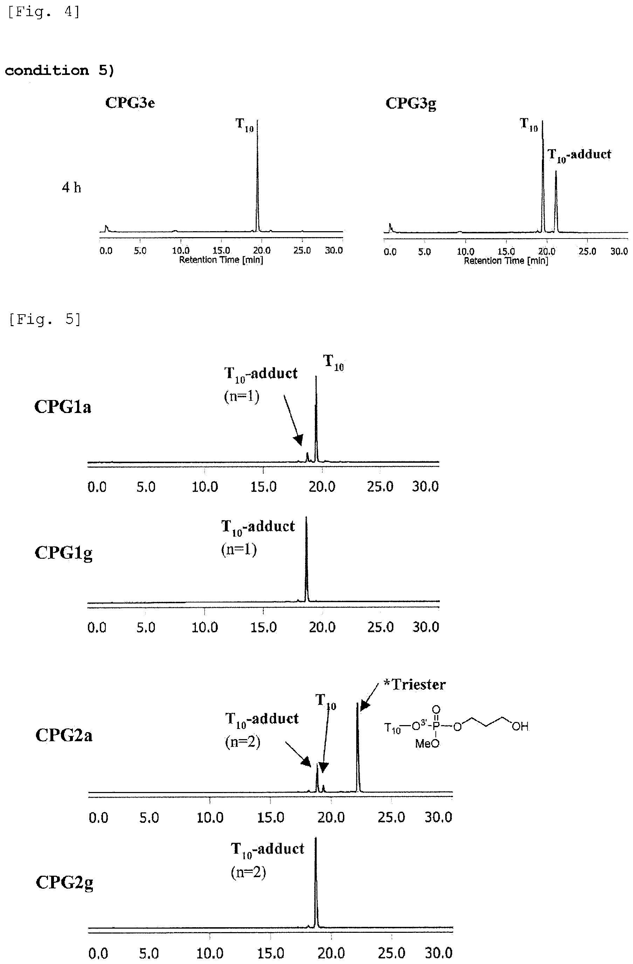

   Fig. 4 shows HPLC charts showing the results of cleavage of oligonucleotides from CPG3e and CPG3g (condition 5)) in Example D1 described later.
[Fig. 5]
   Fig. 5 shows HPLC charts showing the results of cleavage of oligonucleotides from CPG1a and CPG1g/CPG2a and CPG2g in Example D2 described later.
[Fig. 6]

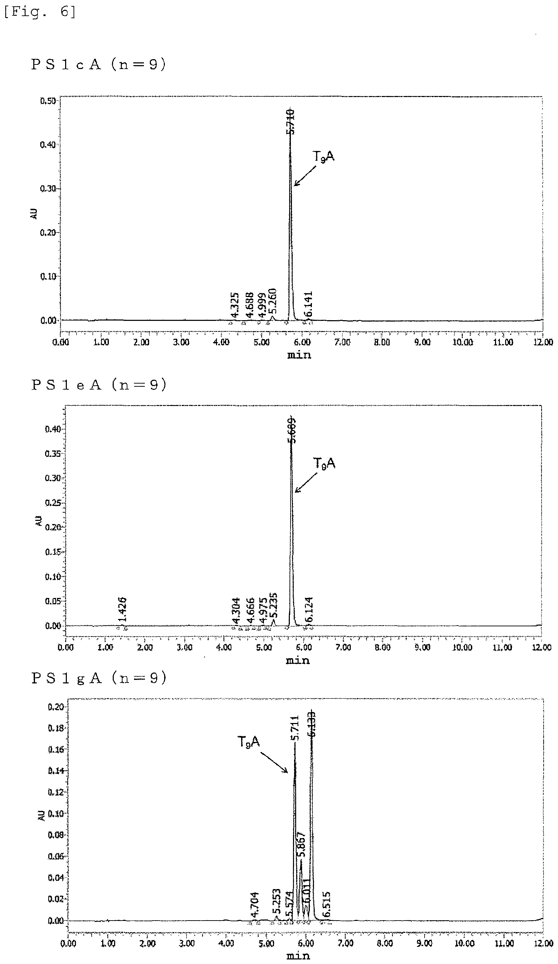

   Fig. 6 shows HPLC charts showing the results of cleavage of oligonucleotides from PS1cA (n=9), PSleA (n=9) and PS1gA (n=9) in Example G1 described later.
[Fig. 7]

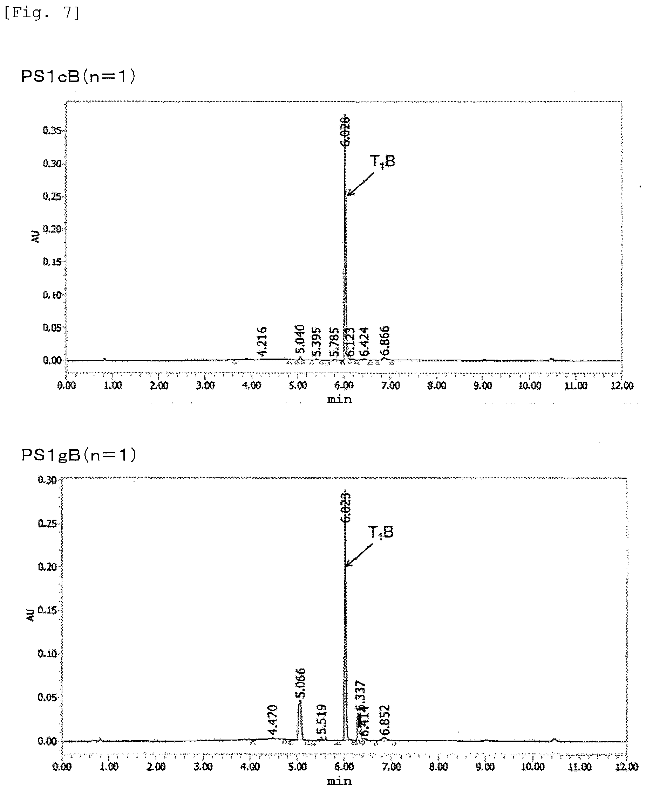

   Fig. 7 shows HPLC charts showing the results of cleavage of oligonucleotides from PS1cB (n=1) and PSlgB (n=1) in Example G2 described later.
[Fig. 8]

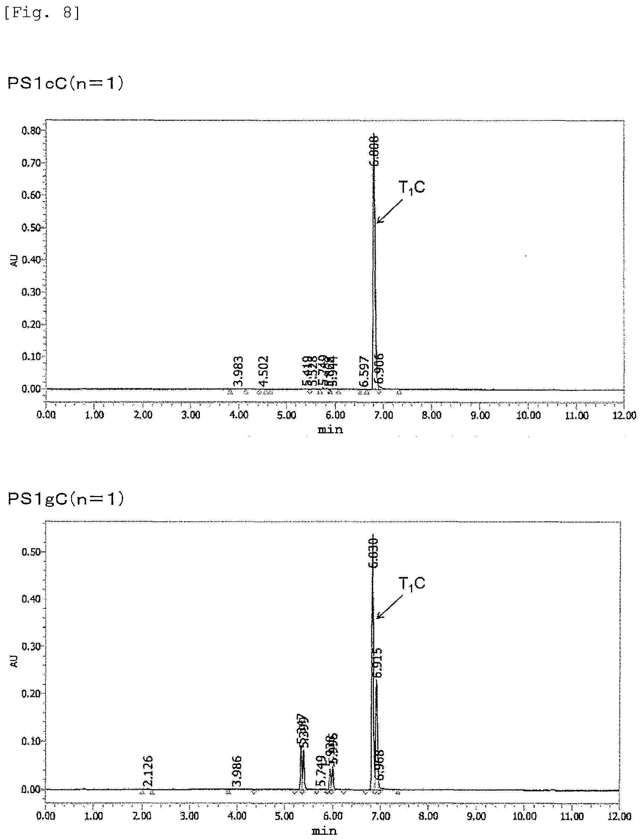

   Fig. 8 shows HPLC charts showing the results of cleavage of oligonucleotides from PS1cC (n=1) and PS1gC (n=1) in Example G2 described later.
[Fig. 9]

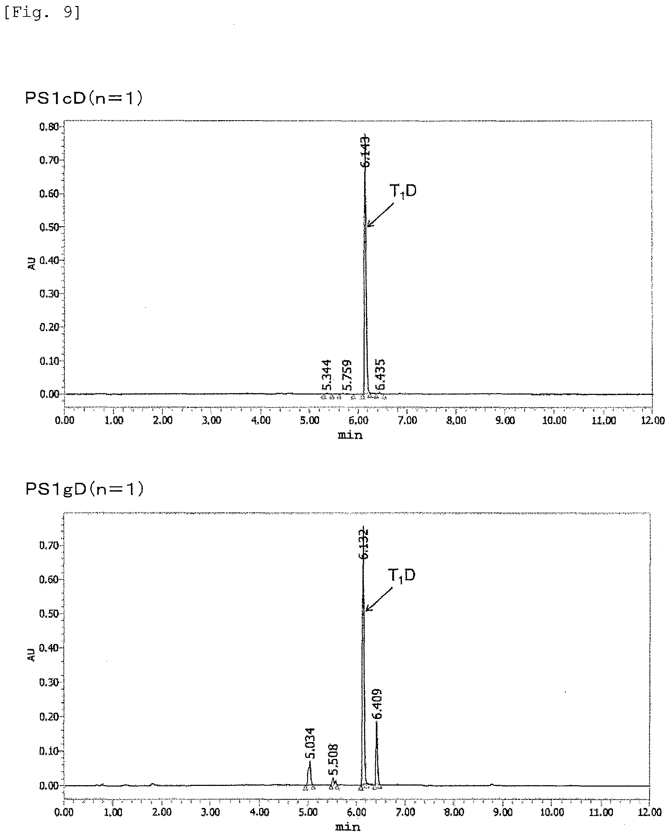

   Fig. 9 shows HPLC charts showing the results of cleavage of oligonucleotides from PS1cD (n=1) and PS1gD (n=1) in Example G2 described later.
[Fig. 10]

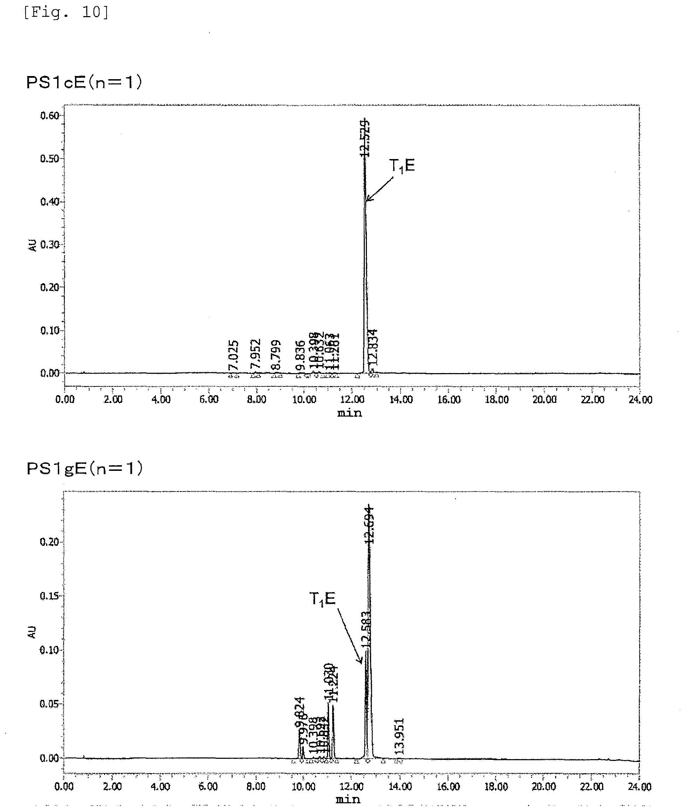

   Fig. 10 shows HPLC charts showing the results of cleavage of oligonucleotides from PS1cE (n=1) and PS1gE (n=1) in Example G2 described later.
[Fig. 11]

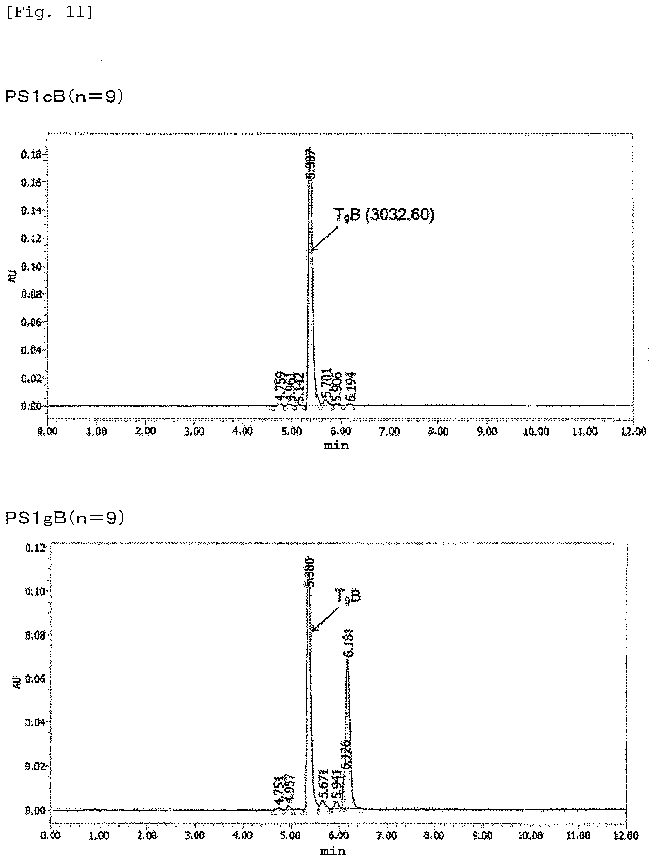

   Fig. 11 shows HPLC charts showing the results of cleavage of oligonucleotides from PS1cB (n=9) and PS1gB (n=9) in Example G2 described later.
[Fig. 12]

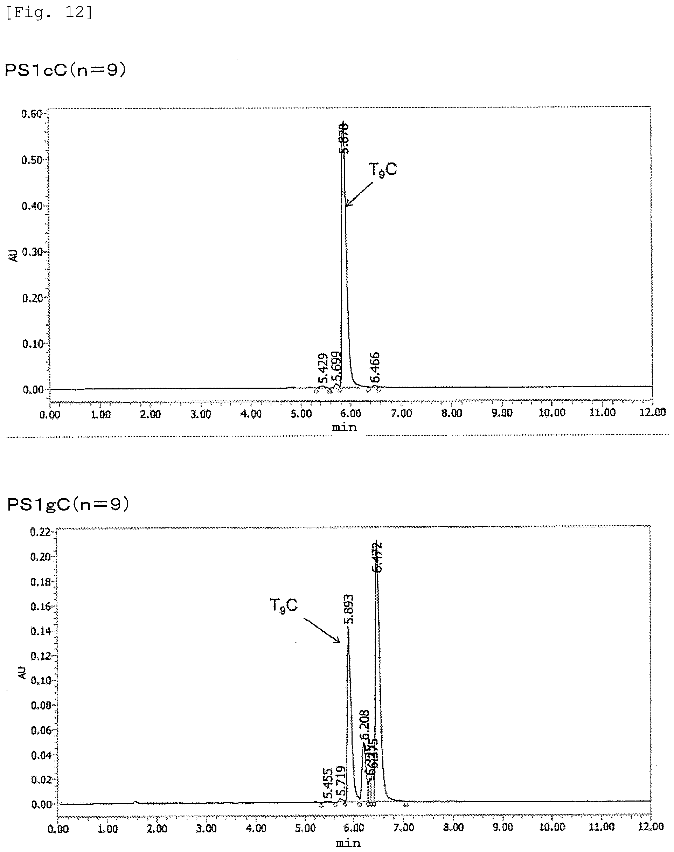

   Fig. 12 shows HPLC charts showing the results of cleavage of oligonucleotides from PS1cC (n=9) and PS1gC (n=9) in Example G2 described later.
[Fig. 13]

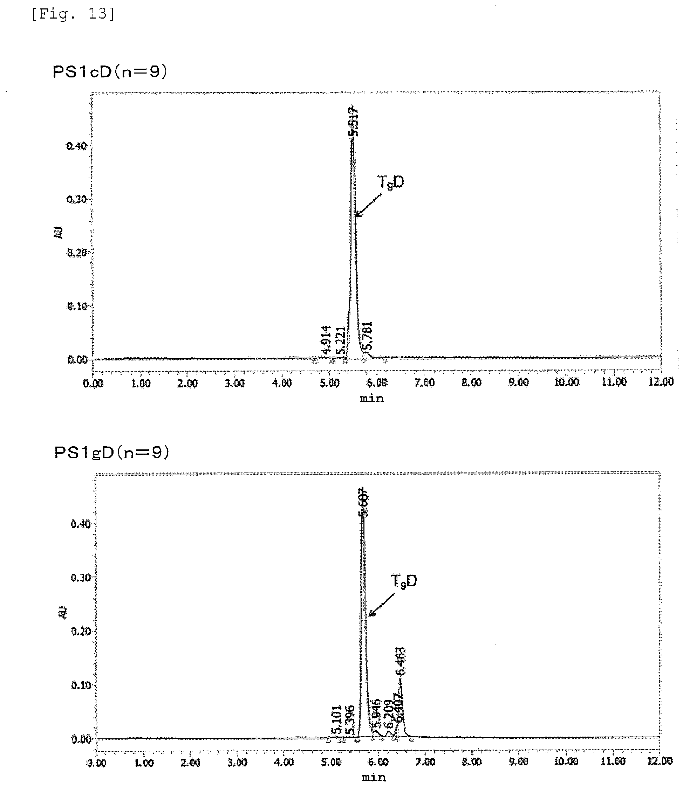

   Fig. 13 shows HPLC charts showing the results of cleavage of oligonucleotides from PS1cD (n=9) and PS1gD (n=9) in Example G2 described later.
[Fig. 14]

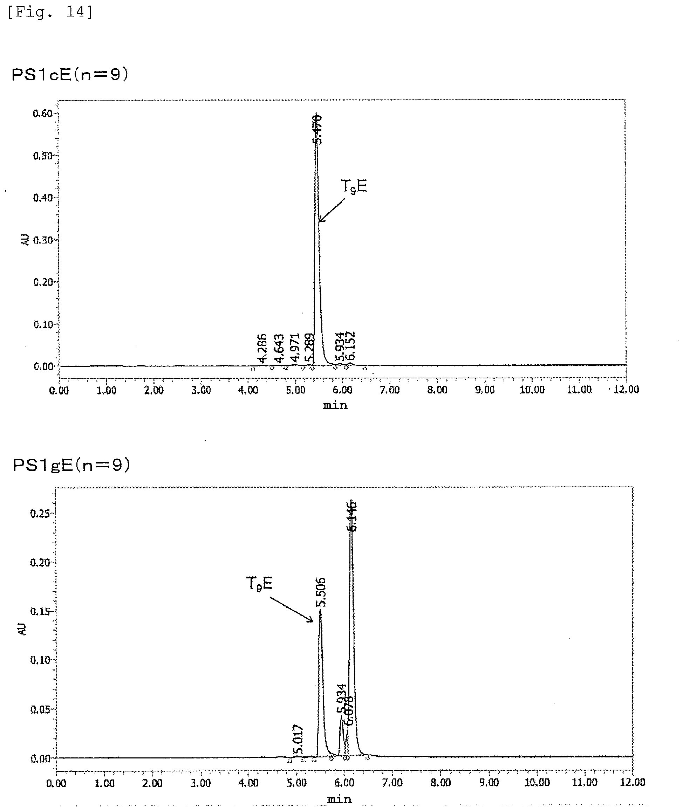

   Fig. 14 shows HPLC charts showing the results of cleavage of oligonucleotides from PS1cE (n=9) and PS1gE (n=9) in Example G2 described later.

### [Description of Embodiments]

A solid phase synthesis method of an oligonucleotide as one embodiment of the present invention is
[1] a solid phase synthesis method of an oligonucleotide, comprising a step of subjecting an oligonucleotide having, at the 3'-terminus of an oligonucleotide sequence thereof, a nucleotide derivative represented by the formula (II): 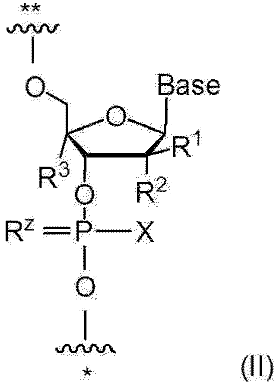
   wherein * is a bonding position to the universal linker;
   ** is a bonding position to the nucleotide or oligonucleotide; R^{z} is O or S;
   X is an optionally substituted alkoxy group (excluding a cyanoethoxy group),
   an optionally substituted cyclic group-oxy group,
   an optionally substituted alkyl group,
   an optionally substituted alkenyl group, or
   an optionally substituted aryl group;
   R¹ is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
   R² is a hydrogen atom, a halogen atom, an optionally protected hydroxy group, an optionally substituted alkyl group, an optionally substituted alkoxy group, an optionally substituted alkenyloxy group, or an optionally substituted alkynyloxy group, and
   R³ is a hydrogen atom, an optionally substituted alkyl group,
   an optionally substituted alkoxy group, or an optionally substituted alkylthio group,
      or
   R² and R³ are bonded to each other to form a group represented by the following formula (shown in the order of -R²-R³-) :
      -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
      in the above-mentioned formulas, Ra to Rj are each independently a hydrogen atom or an optionally substituted alkyl group; and
      Base is an optionally modified nucleobase,
      which oligonucleotide is carried on a solid phase support via a universal linker and synthesized by a nucleotide elongation reaction in a solid phase synthesis method of an oligonucleotide,
      to a reaction for cleaving the oligonucleotide from the solid phase support and the universal linker.
   More specifically,
[2] a solid phase synthesis method of an oligonucleotide, including
   (1) a step of carrying a nucleotide derivative represented by the formula (I): 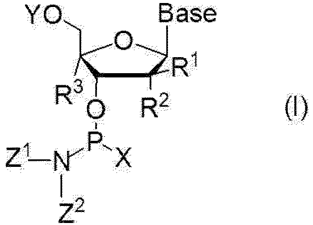
      wherein Y is a hydrogen atom or a hydroxy-protecting group;
      Z¹ and Z² are each independently an optionally substituted alkyl group, or
      Z¹ and Z² are bonded to each other to form, together with a nitrogen atom bonded thereto, an optionally substituted heterocycle;
      X, R¹ to R³, and Base are each as defined above,
      on a solid phase support via a universal linker (hereinafter to be also denoted as "step (1)"),
   (2) a step of sequentially condensing nucleic acid monomers according to the desired oligonucleotide sequence to elongate the nucleotide (one or two or more nucleotide derivatives represented by the above-mentioned formula (I) may also be used as the nucleic acid monomers) (hereinafter to be also denoted as "step (2)"),
   (3) a step of subjecting the obtained oligonucleotide having the desired sequence to a reaction for cleaving from the solid phase support and the universal linker (hereinafter to be also denoted as "step (3)").

As used herein, since the "oligonucleotide having the desired sequence" obtained in step (2) corresponds to "the oligonucleotide having, at the 3'-terminus of an oligonucleotide sequence thereof, a nucleotide derivative represented by the formula (II): 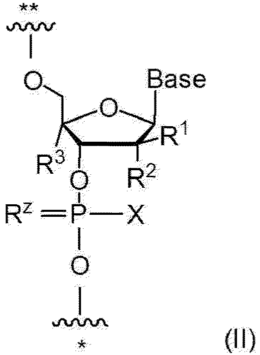 wherein each symbol is as defined above, which oligonucleotide is carried on a solid phase support via a universal linker" in the above-mentioned [1], the above-mentioned [2] can also be said to be:
[2-1] the solid phase synthesis method of an oligonucleotide of the above-mentioned [1], in which an oligonucleotide having, at the 3'-terminus of an oligonucleotide sequence thereof, a nucleotide derivative represented by the formula (II): 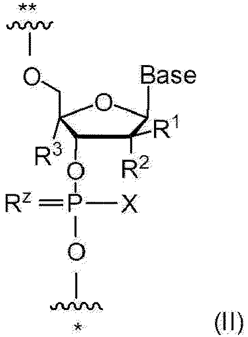
wherein each symbol is as defined above,
which oligonucleotide is carried on a solid phase support via a universal linker, is obtained by steps including
   (1) a step of carrying a nucleotide derivative represented by the formula (I): 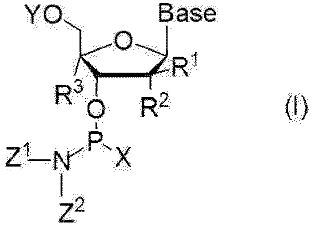
      wherein each symbol is as defined above,
      on a solid phase support via a universal linker ("step (1)"), and
   (2) a step of sequentially condensing nucleic acid monomers according to the desired oligonucleotide sequence to elongate the nucleotides (here, one or two or more nucleotide derivatives represented by the above-mentioned formula (I) may also be used as the nucleic acid monomers) ("step (2)").

The "oligonucleotide (nucleic acid)" of interest in the present invention means a chain compound in which nucleosides are linked by phosphodiester bonds, and includes DNA, RNA, and the like. The oligonucleotide (nucleic acid) may be either single-stranded or double-stranded, but is preferably single-stranded because it can be efficiently synthesized using an oligonucleotide (nucleic acid) synthesizer. In addition, the "oligonucleotide (nucleic acid)" includes not only oligonucleotides containing purine bases such as adenine (A) and guanine (G) and pyrimidine bases such as thymine (T), cytosine (C), and uracil (U), but also modified oligonucleotides (nucleic acids) containing other modified heterocyclic bases. In the present invention, nucleosides linked by phosphorothioate bonds are also included in the "modified oligonucleotide (nucleic acid)". In addition, cases where the sugar moiety of nucleoside is modified or where a crosslinked structure is formed between the atoms constituting the sugar moiety (bridged nucleotide, BNA/LNA) are also included in the "modified oligonucleotide (nucleic acid)".

### [Nucleotide derivative (I) in the present invention]

As described above, the present invention is characterized in that the desired oligonucleotide is synthesized by carrying a nucleotide derivative represented by the formula (I): 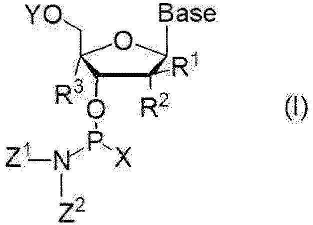
wherein each group is as defined above,
on a solid phase support via a universal linker, and sequentially condensing (coupling) nucleic acid monomers according to the desired oligonucleotide sequence, by using the nucleotide derivative (I) as a starting point, to elongate the nucleotide.

Each group in the nucleotide derivative (I) is described below.

In the present specification, examples of the "halogen atom" include fluorine, chlorine, bromine, and iodine.

In the present specification, the "alkyl group" (including "alkyl moiety" constituting the group) refers to a linear or branched chain saturated hydrocarbon group, where one having 1 to 6 carbon atoms is preferred. Examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

In the present specification, the "alkenyl group" (including "alkenyl moiety" constituting the group) refers to a linear or branched chain hydrocarbon group having one or more double bonds in a molecule, where one having 2 to 6 carbon atoms is preferred. Examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

In the present specification, the "alkynyl group" (including "alkynyl moiety" constituting the group) refers to a linear or branched chain hydrocarbon group having one or more triple bonds in a molecule, where one having 2 to 6 carbon atoms is preferred. Examples of the "C₂₋₆ alkynyl group" include ethynyl, 1-propynyl, 2-propynyl(propargyl), 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and 4-methyl-2-pentynyl.

In the present specification, the "alkoxy group" (including "alkoxy moiety" constituting the group) refers to a group having a structure in which a hydrogen atom of the hydroxy group is substituted by the aforementioned "alkyl group" where one having 1 to 6 carbon atoms is preferred. Examples of the "C₁₋₆ alkoxy group" include methoxy group, ethoxy group, n-propoxy group, isopropoxy group, n-butoxy group, isobutoxy group, sec-butoxy group, tert-butoxy group, n-pentyloxy group, isopentyloxy group, sec-pentyloxy group, tert-pentyloxy group, hexyloxy group, and the like.

In the present specification, the "alkylthio group" (including "alkylthio moiety" constituting the group) refers to a group having a structure in which a hydrogen atom of the thiol group is substituted by the aforementioned "alkyl group" where one having 1 to 6 carbon atoms is preferred. Examples of the "C₁₋₆ alkylthio group" include methylthio, ethylthio, propylthio, isopropylthio, butylthio, isobutylthio, sec-butylthio, tert-butylthio, pentylthio, isopentylthio, neopentylthio, 1-ethylpropylthio, hexylthio, isohexylthio, 1,1-dimethylbutylthio, 2,2-dimethylbutylthio, 3,3-dimethylbutylthio, and 2-ethylbutylthio.

In the present specification, the "aryl group" (including "aryl moiety" constituting the group) refers to an aromatic hydrocarbon ring group where one having 6 to 14 carbon atoms is preferred. Examples of the C₆₋₁₄ aromatic hydrocarbon ring group include phenyl, naphthyl, and the like.

In the present specification, the "cyclic group" (including "cyclic group moiety" constituting the group) refers to "hydrocarbon ring group" and "heterocyclic group".

In the present specification, examples of the "hydrocarbon ring group" include aromatic hydrocarbon ring group (preferably, C₆₋₁₄ aromatic hydrocarbon ring group having 6 to 14 carbon atoms), cycloalkyl (preferably, C₃₋₁₀ cycloalkyl having 3 to 10 carbon atoms), and cycloalkenyl (preferably, C₃₋₁₀ cycloalkenyl having 3 to 10 carbon atoms).

In the present specification, examples of the "C₆₋₁₄ aromatic hydrocarbon ring group" include phenyl and naphthyl.

In the present specification, examples of the "C₃₋₁₀ cycloalkyl" include cyclopropyl, cyclobutyl, cyclopentyl, cyclohexyl, cycloheptyl, and cyclooctyl.

In the present specification, examples of the "C₃₋₁₀ cycloalkenyl" include cyclopropenyl, cyclobutenyl, cyclopentenyl, cyclohexenyl, cycloheptenyl, and cyclooctenyl.

In the present specification, examples of the "heterocyclic group" include (i) an aromatic heterocyclic group, (ii) a non-aromatic heterocyclic group, and (iii) a 7- to 10-membered crosslinked heterocyclic group, each containing, as a ring-constituting atom besides carbon atom, 1 to 4 hetero atoms selected from a nitrogen atom, a sulfur atom, and an oxygen atom.

In the present specification, examples of the "aromatic heterocyclic group" (including "5- to 14-membered aromatic heterocyclic group") include a 5- to 14-membered (preferably, 5- to 10-membered) aromatic heterocyclic group containing, as a ring-constituting atom besides carbon atom, 1 to 4 hetero atoms selected from a nitrogen atom, a sulfur atom and an oxygen atom.

Preferred examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl, and the like; and 8- to 14-membered fused polycyclic (preferably, bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl, and the like.

In the present specification, examples of the "non-aromatic heterocyclic group" (including "3- to 14-membered non-aromatic heterocyclic group") include a 3- to 14-membered (preferably, 4- to 10-membered) non-aromatic heterocyclic group containing, as a ring-constituting atom besides carbon atom, 1 to 4 hetero atoms selected from a nitrogen atom, a sulfur atom and an oxygen atom.

Preferred examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl, and the like; and 9- to 14-membered fused polycyclic (preferably, bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl, and the like.

In the present specification, preferred examples of the "7- to 10-membered crosslinked heterocyclic group" include quinuclidinyl and 7-azabicyclo[2.2.1]heptanyl.

Examples of the "heterocycle" when Z¹ and Z² are bonded to each other to form, together with a nitrogen atom bonded thereto, an optionally substituted heterocycle include a nitrogen-containing aromatic heterocycle and a nitrogen-containing non-aromatic heterocycle, each containing, as a ring-constituting atom, 1 to 4 nitrogen atoms besides carbon atom, and further, 1 to 4 hetero atoms selected from a sulfur atom and an oxygen atom.

In the present specification, preferred examples of the "nitrogen-containing aromatic heterocycle" include 5- or 6-membered monocyclic nitrogen-containing aromatic heterocycles such as imidazole, pyrazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine, and the like; and 8- to 14-membered fused polycyclic (preferably, bi or tricyclic) nitrogen-containing aromatic heterocycles such as benzoimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, indole, isoindole, 1H-indazole, purine, isoquinoline, quinoline, and the like.

In the present specification, preferred examples of the "nitrogen-containing non-aromatic heterocycle" include 3- to 8-membered (more preferably, 5- or 6-membered) monocyclic nitrogen-containing non-aromatic heterocycles such as aziridine, pyrroline, pyrrolidine, imidazoline, imidazolidine, oxazoline, oxazolidine, pyrazoline, pyrazolidine, thiazoline, thiazolidine, tetrahydroisothiazole, tetrahydroxazole, tetrahydroisoxazole, piperidine, piperazine, tetrahydropyridine, dihydropyridine, tetrahydropyrimidine, tetrahydropyridazine, morpholine, thiomorpholine, and the like; and 9- to 14-membered fused polycyclic (preferably, bi or tricyclic) nitrogen-containing non-aromatic heterocycles such as dihydrobenzimidazole, dihydrobenzoxazole, dihydrobenzothiazole, dihydrobenzisothiazole, tetrahydroisoquinoline, tetrahydroquinoline, 4H-quinolizine, indoline, isoindoline, tetrahydrothieno[2,3-c]pyridine, tetrahydrobenzazepine, and the like.

In the present specification, the "hydroxy-protecting group" for Y is not particularly limited, and those generally used for protecting the 5'-hydroxy group in the solid phase synthesis of oligonucleotides (nucleic acids) can be used. For example, trityl-based protecting groups, silyl-based protecting groups, and acyl-based protecting groups can be mentioned.

Examples of the "trityl-based protecting group" include trityl groups optionally substituted by any substituents (e.g., substituents selected from C₁₋₆ alkoxy group, C₁₋₆ alkyl group, halogen atom, and the like (two or more substituents may be joined to form a ring)). Specifically, a trityl group (Tr), a mono-methoxytrityl group (e.g., 4-methoxytrityl group (MMTr)), a dimethoxytrityl group (e.g., 4,4'-dimethoxytrityl group (DMTr)), a 9-phenylxanthen-9-yl group (pixyl group), and the like, preferably, a 4,4'-dimethoxytrityl group (DMTr), can be mentioned.

Examples of the "silyl-based protecting group" include silyl groups tri-substituted by any substituents (e.g., substituents selected from C₁₋₆ alkoxy group, C₁₋₆ alkyl group, phenyl group, and the like). Specifically, a trimethylsilyl group, a triethylsilyl group, an isopropyldimethylsilyl group, a tert-butyldimethylsilyl group, a dimethylmethoxysilyl group, a methyldimethoxysilyl group, a tert-butyldiphenylsilyl group, and the like, preferably, a trimethylsilyl group, can be mentioned.

Examples of the "acyl-based protecting group" include an acetyl group, a t-butoxycarbonyl group, a benzoyl group, a levulinoyl group, and the like.

Among the above-mentioned hydroxy-protecting groups, a trityl-based protecting group is preferred and a 4,4'-dimethoxytrityl group (DMTr) is more preferred, because deprotection is easily performed using an acid in the case of hydroxy-protecting groups that are removed by acids.

In the present specification, "optionally substituted" means an embodiment without substitution or with substitution with 1 to 3 substituents at substitutable positions. In the case of 2 or 3 substitutions, each substituent may be the same or different.

In the present specification, "substituted" means an embodiment with substitution with 1 to 3 substituents at substitutable positions. In the case of 2 or 3 substitutions, each substituent may be the same or different.

Examples of the substituent that each group of the nucleotide derivative (I), such as "an optionally substituted alkoxy group", "an optionally substituted cyclic group-oxy group", "an optionally substituted alkyl group", "an optionally substituted alkenyl group", "an optionally substituted alkynyl group", "an optionally substituted aryl group", and the like, can have include
a halogen atom (e.g., fluorine, chlorine, bromine, iodine);
a cyano group;
an alkyl group (e.g., a C₁₋₆ alkyl group);
an alkoxy group (e.g., a C₁₋₆ alkoxy group);
an alkoxy-alkoxy group (e.g., a C₁₋₆ alkoxy-C₁₋₆ alkoxy group (e.g., methoxy-methoxy, methoxy-ethoxy, ethoxy-ethoxy, etc.)); an aralkyloxy group (e.g., a C₇₋₁₆ aralkyloxy group (e.g., benzyloxy)) ;
a protected hydroxy group (e.g., a C₁₋₆ alkyl-carbonyloxy group (e.g., acetoxy, propanoyloxy), a C₆₋₁₄ aryl-carbonyloxy group (e.g., benzoyloxy, 1-naphthoyloxy, 2-naphthoyloxy), a C₁₋₆ alkoxy-carbonyloxy group (e.g., methoxycarbonyloxy, ethoxycarbonyloxy, propoxycarbonyloxy, butoxycarbonyloxy), a mono- or di-C₁₋₆ alkyl-carbamoyloxy group (e.g., methylcarbamoyloxy, ethylcarbamoyloxy, dimethylcarbamoyloxy, diethylcarbamoyloxy), a C₆₋₁₄ aryl-carbamoyloxy group (e.g., phenylcarbamoyloxy, naphthylcarbamoyloxy) etc.); an aryl group (e.g., a C₆₋₁₄ aryl group);
a protected amino group (e.g., an acylamino group (e.g., a formylamino group, a C₁₋₁₀ alkyl-carbonylamino group (e.g., acetylamino), a C₂₋₆ alkenyl-carbonylamino group (e.g., crotonoyl amino), a C₃₋₁₀ cycloalkyl-carbonylamino group (e.g., cyclobutanecarbonylamino, cyclopentanecarbonylamino, cyclohexanecarbonylamino, cycloheptanecarbonylamino), a C₃₋₁₀ cycloalkenyl-carbonylamino group (e.g., 2-cyclohexenecarbonylamino), a C₆₋₁₄ aryl-carbonylamino group (e.g., benzoylamino), a C₇₋₁₆ aralkyl-carbonylamino group (e.g., benzylcarbonylamino), a C₆₋₁₄ aryloxy-carbonylamino group (e.g., phenyloxycarbonylamino, naphthyloxycarbonylamino), a C₇₋₁₆ aralkyloxy-carbonylamino group (e.g., benzyloxycarbonylamino, phenethyloxycarbonylamino), a carbamoylamino group, a mono- or di-C₁₋₆ alkyl-carbamoylamino group, a mono- or di-C₂₋₆ alkenyl-carbamoylamino group (e.g., diallylcarbamoylamino), a mono- or di-C₃₋₁₀ cycloalkyl-carbamoylamino group (e.g., cyclopropylcarbamoylamino), a mono- or di-C₆₋₁₄ aryl-carbamoylamino group (e.g., phenylcarbamoylamino), a mono- or di-C₇₋₁₆ aralkyl-carbamoylamino group, a 5- to 14- membered aromatic heterocyclic carbamoylamino group (e.g., pyridylcarbamoylamino), a thiocarbamoylamino group, a mono- or di-C₁₋₆ alkyl-thiocarbamoylamino group (e.g., methylthiocarbamoylamino, N-ethyl-N-methylthiocarbamoylamino), a mono- or di-C₂₋₆ alkenyl-thiocarbamoylamino group (e.g., dial lylthiocarbamoylamino), a mono- or di-C₃₋₁₀ cycloalkyl-thiocarbamoylamino group (e.g., cyclopropylthiocarbamoylamino, cyclohexylthiocarbamoylamino), a mono- or di-C₆₋₁₄ aryl-thiocarbamoylamino group (e.g., phenylthiocarbamoylamino), a mono- or di-C₇₋₁₆ aralkyl-thiocarbamoylamino group (e.g., benzylthiocarbamoylamino, phenethylthiocarbamoylamino), a 5- to 14- membered aromatic heterocyclic thiocarbamoylamino group (e.g., pyridylthiocarbamoylamino); a C₁₋₆ alkylamino group (e.g., methylamino); a N,N-di-C₁₋₆ alkylamino group (e.g., N,N-dimethylamino), a C₃₋₁₀ cycloalkylamino group (e.g., cyclopropylamino); a C₆₋₁₄ arylamino group (e.g., phenylamino), etc.),
wherein the protected amino group is more preferably an acylamino group, further preferably a formylamino group or a C₁₋₁₀ alkyl-carbonylamino group; or
an optionally substituted carbamoyl group (e.g., a carbamoyl group, a mono- or di-C₁₋₁₀ alkyl-carbamoyl group (e.g., mono- or di-methyl-carbamoyl), a mono- or di-C₂₋₆ alkenyl-carbamoyl group (e.g., mono- or di-allylcarbamoyl), a mono- or di-C₃₋₁₀ cycloalkyl-carbamoyl group (e.g., cyclopropylcarbamoyl, cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ arylcarbonyl-carbamoyl group (e.g., benzoylcarbamoyl), a 5- to 14-membered aromatic heterocyclic carbamoyl group (e.g., pyridylcarbamoyl),
wherein the optionally substituted carbamoyl group is more preferably a carbamoyl group or a mono- or di-C₁₋₁₀ alkyl-carbamoyl group, further preferably a carbamoyl group or a C₁₋₁₀ alkyl-carbamoyl group;
and the like.

In the "optionally substituted alkoxy group", "optionally substituted alkyl group", "optionally substituted alkenyloxy group", and "optionally substituted alkynyloxy group" of the nucleotide derivative (I), when the "alkoxy group", "alkyl group", "alkenyloxy group", and "alkynyloxy group" are each substituted by a "substituent" selected from a formylamino group, a C₁₋₁₀ alkyl-carbonylamino group, a carbamoyl group, and a C₁₋₁₀ alkyl-carbamoyl group, the total carbon number of the constituent moieties other than -CONH- (or -NHCO-) is preferably 1 to 10.

In the present specification, the "optionally modified nucleobase" is not particularly limited as long as it is used for the synthesis of oligonucleotides (nucleic acids). For example, cytosyl group, uracil group, thyminyl group, adenyl group, guanyl group, and modified nucleobases of these such as 8-bromoadenyl group, 8-bromoguanyl group, 5-bromocytosyl group, 5-iodocytosyl group, 5-bromouracil group, 5-iodouracil group, 5-fluorouracil group, 5-methylcytosyl group, 8-oxoguanyl group, hypoxanthinyl group, and the like can be mentioned.

Here, in the "nucleobase", for example, an amino group of the adenyl group, guanyl group and cytosyl group or modified nucleobase may be protected. The amino-protecting group is not particularly limited as long as it is used as a nucleic acid-protecting group, and specifically, for example, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and the like are optionally used for protection.

Preferred embodiments of each symbol in the nucleotide derivative (I) are described below.
X is as defined above, and is preferably
an optionally substituted C₁₋₆ alkoxy group (excluding a cyanoethoxy group),
an optionally substituted C₆₋₁₄ aryl-oxy group,
an optionally substituted C₃₋₁₀ cycloalkyl-oxy group,
an optionally substituted 3- to 14-membered non-aromatic heterocyclic-oxy group,
an optionally substituted C₁₋₆ alkyl group,
an optionally substituted C₂₋₆ alkenyl group, or
an optionally substituted C₆₋₁₄ aryl group,
more preferably,
a C₁₋₆ alkoxy group optionally substituted by 1 to 3 substituents selected from a C₁₋₆ alkoxy group, a C₁₋₆ alkoxy-C₁₋₆ alkoxy group, and a C₇₋₁₆ alkoxy group,
a C₆₋₁₄ aryl-oxy group optionally substituted by 1 to 3 substituents selected from a C₁₋₆ alkyl group, a C₁₋₆ alkoxy group, and a protected hydroxy group,
a C₃₋₆ cycloalkyl-oxy group optionally substituted by 1 to 3 substituents selected from a C₁₋₆ alkyl group, a C₁₋₆ alkoxy group, and a protected hydroxy group,
a 5- or 6-membered non-aromatic heterocyclic-oxy group optionally substituted by 1 to 3 substituents selected from a C₁₋₆ alkyl group, a C₁₋₆ alkoxy group, and a protected hydroxy group,
a C₁₋₆ alkyl group optionally substituted by 1 to 3 C₁₋₆ alkoxy groups,
a C₂₋₆ alkenyl group optionally substituted by 1 to 3 substituents selected from a C₁₋₆ alkyl group and a C₆₋₁₄ aryl group, or
a C₆₋₁₄ aryl group optionally substituted by 1 to 3 substituents selected from a C₁₋₆ alkyl group and a C₁₋₆ alkoxy group, further preferably,
a C₁₋₆ alkoxy group,
a C₆₋₁₄ aryl-oxy group,
a C₃₋₆ cycloalkyl-oxy group,
a 5- or 6-membered non-aromatic heterocyclic-oxy group,
a C₁₋₆ alkyl group,
a C₂₋₆ alkenyl group, or
a C₆₋₁₄ aryl group,
most preferably,
a C₁₋₆ alkoxy group,
a phenyl-oxy group, or
a C₁₋₆ alkyl group.

Y is as defined above, and is preferably
a hydrogen atom or an optionally substituted trityl-based protecting group,
more preferably,
a trityl group, a mono methoxytrityl group (e.g., a 4-methoxytrityl group), or a dimethoxytrityl group (e.g., a 4,4'-dimethoxytrityl group),
most preferably,
a dimethoxytrityl group.

Z¹ and Z² are each independently as defined above, and preferably
an optionally substituted C₁₋₆ alkyl group, or
Z¹ and Z² are bonded to each other to form, together with a nitrogen atom bonded thereto, an optionally substituted nitrogen-containing non-aromatic heterocycle,
more preferably,
Z¹ and Z² are each independently a C₁₋₆ alkyl group, or
Z¹ and Z² are bonded to each other to form, together with a nitrogen atom bonded thereto, a 3- to 8-membered monocyclic nitrogen-containing non-aromatic heterocycle.

R¹ is as defined above, and is preferably
a hydrogen atom, a halogen atom, an optionally protected hydroxy group,
an optionally substituted C₁₋₆ alkyl group,
an optionally substituted C₁₋₆ alkoxy group,
an optionally substituted C₂₋₆ alkenyloxy group, or
an optionally substituted C₂₋₆ alkynyloxy group,
more preferably,
a hydrogen atom, a halogen atom, a hydroxy group,
a C₁₋₆ alkyl group optionally substituted by a group selected from a cyano group, a C₁₋₆ alkoxy group, a protected amino group, and an optionally substituted carbamoyl group,
a C₁₋₆ alkoxy group optionally substituted by a group selected from a cyano group, a C₁₋₆ alkoxy group, a protected amino group, and an optionally substituted carbamoyl group,
a C₂₋₆ alkenyloxy group optionally substituted by a group selected from a cyano group, a C₁₋₆ alkoxy group, a protected amino group, and an optionally substituted carbamoyl group, or
a C₂₋₆ alkynyloxy group optionally substituted by a group selected from a cyano group, a C₁₋₆ alkoxy group, a protected amino group, and an optionally substituted carbamoyl group, further preferably,
a hydrogen atom, a halogen atom, a hydroxy group,
a C₁₋₆ alkyl group,
a C₁₋₆ alkoxy group optionally substituted by a group selected from a cyano group, a C₁₋₆ alkoxy group, an acylamino group, a carbamoyl group, and a mono- or di-C₁₋₁₀ alkyl-carbamoyl group (particularly preferably, a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group),
a C₂₋₆ alkenyloxy group, or
a C₂₋₆ alkynyloxy group.

R² is as defined above, and is preferably
a hydrogen atom, a halogen atom, an optionally protected hydroxy group,
an optionally substituted C₁₋₆ alkyl group,
an optionally substituted C₁₋₆ alkoxy group,
an optionally substituted C₂₋₆ alkenyloxy group, or
an optionally substituted C₂₋₆ alkynyloxy group,
more preferably,
a hydrogen atom, a halogen atom, a hydroxy group,
a C₁₋₆ alkyl group optionally substituted by a group selected from a cyano group, a C₁₋₆ alkoxy group, a protected amino group, and an optionally substituted carbamoyl group,
a C₁₋₆ alkoxy group optionally substituted by a group selected from a cyano group, a C₁₋₆ alkoxy group, a protected amino group, and an optionally substituted carbamoyl group,
a C₂₋₆ alkenyloxy group optionally substituted by a group selected from a cyano group, a C₁₋₆ alkoxy group, a protected amino group, and an optionally substituted carbamoyl group, or
a C₂₋₆ alkynyloxy group optionally substituted by a group selected from a cyano group, a C₁₋₆ alkoxy group, a protected amino group, and an optionally substituted carbamoyl group, further preferably,
a hydrogen atom, a halogen atom, a hydroxy group,
a C₁₋₆ alkyl group,
a C₁₋₆ alkoxy group optionally substituted by a group selected from a cyano group, a C₁₋₆ alkoxy group, an acylamino group, a carbamoyl group, and a mono- or di-C₁₋₁₀ alkyl-carbamoyl group (particularly preferably, a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group),
a C₂₋₆ alkenyloxy group, or
a C₂₋₆ alkynyloxy group.

R³ is as defined above, and is preferably
a hydrogen atom, an optionally substituted C₁₋₆ alkyl group, an optionally substituted C₁₋₆ alkoxy group, or an optionally substituted C₁₋₆ alkylthio group,
more preferably,
a hydrogen atom, a C₁₋₆ alkyl group, a C₁₋₆ alkoxy group, or a C₁₋₆ alkylthio group.

R² and R³ are bonded to each other to form a group represented by the following formula (shown in the order of - R²-R³-) :
-O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
in the above-mentioned formulas, Ra to Rj are each independently a hydrogen atom or an optionally substituted alkyl group,
more preferably,
a group represented by the following formula (shown in the order of -R²-R³-) :
-O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
in the above-mentioned formulas, Ra to Rj are each independently a hydrogen atom or an optionally substituted C₁₋₆ alkyl group,
most preferably,
a group represented by the following formula (shown in the order of -R²-R³-) :
   -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
   in the above-mentioned formulas, Ra to Rj are each independently a hydrogen atom or a C₁₋₆ alkyl group.

Base is an optionally modified nucleobase.

In the nucleotide derivative (I) of the present invention, when X is an optionally substituted (C₄-C₅)alkoxy group, the nucleotide derivative (I) is a novel compound.

The nucleotide derivative (I) in the present invention can be produced by appropriately applying the specific methods described in Examples/Production Examples below or methods known in the pertinent technique field. When the nucleotide derivative (I) is commercially available, commercially available products may also be used.

Preferred embodiments of each symbol of the nucleotide derivative residue (II) are the same as those described above for nucleotide derivative (I).

Preferred nucleotide derivatives (I) are described below.

### [Nucleotide derivative (I-A)]

A nucleotide derivative (I), wherein
X is an optionally substituted C₁₋₆ alkoxy group (excluding a cyanoethoxy group),
an optionally substituted C₆₋₁₄ aryl-oxy group,
an optionally substituted C₃₋₁₀ cycloalkyl-oxy group,
an optionally substituted 3- to 14-membered non-aromatic heterocyclic-oxy group,
an optionally substituted C₁₋₆ alkyl group,
an optionally substituted C₂₋₆ alkenyl group, or
an optionally substituted C₆₋₁₄ aryl group;
Y is a hydrogen atom or an optionally substituted trityl-based protecting group;
Z¹ and Z² are each independently an optionally substituted C₁₋₆ alkyl group, or
Z¹ and Z² are bonded to each other to form, together with a nitrogen atom bonded thereto, an optionally substituted nitrogen-containing non-aromatic heterocycle;
R¹ is a hydrogen atom, a halogen atom, an optionally protected hydroxy group, an optionally substituted C₁₋₆ alkyl group, an optionally substituted C₁₋₆ alkoxy group, an optionally substituted C₂₋₆ alkenyloxy group, or an optionally substituted C₂₋₆ alkynyloxy group;
R² is a hydrogen atom, a halogen atom, an optionally protected hydroxy group, an optionally substituted C₁₋₆ alkyl group, an optionally substituted C₁₋₆ alkoxy group, an optionally substituted C₂₋₆ alkenyloxy group, or an optionally substituted C₂₋₆ alkynyloxy group;
R³ is a hydrogen atom, an optionally substituted C₁₋₆ alkyl group, an optionally substituted C₁₋₆ alkoxy group, or an optionally substituted C₁₋₆ alkylthio group, or
R² and R³ are bonded to each other to form a group represented by the following formula (shown in the order of -R²-R³-) :
   -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
   in the above-mentioned formulas, Ra to Rj are each independently a hydrogen atom or an optionally substituted C₁₋₆ alkyl group; and

Base is an optionally modified nucleobase.

### [Nucleotide derivative (I-B)]

A nucleotide derivative (I), wherein
X is a C₁₋₆ alkoxy group optionally substituted by 1 to 3 substituents selected from a C₁₋₆ alkoxy group, a C₁₋₆ alkoxy-C₁₋₆ alkoxy group, and a C₇₋₁₆ alkoxy group,
a C₆₋₁₄ aryl-oxy group optionally substituted by 1 to 3 substituents selected from a C₁₋₆ alkyl group, a C₁₋₆ alkoxy group, and a protected hydroxy group,
a C₃₋₆ cycloalkyl-oxy group optionally substituted by 1 to 3 substituents selected from a C₁₋₆ alkyl group, a C₁₋₆ alkoxy group, and a protected hydroxy group,
a 5- or 6-membered non-aromatic heterocyclic-oxy group optionally substituted by 1 to 3 substituents selected from a C₁₋₆ alkyl group, a C₁₋₆ alkoxy group, and a protected hydroxy group,
a C₁₋₆ alkyl group optionally substituted by 1 to 3 C₁₋₆ alkoxy groups,
a C₂₋₆ alkenyl group optionally substituted by 1 to 3 substituents selected from a C₁₋₆ alkyl group and a C₆₋₁₄ aryl group, or
a C₆₋₁₄ aryl group optionally substituted by 1 to 3 substituents selected from a C₁₋₆ alkyl group and a C₁₋₆ alkoxy group;
Y is a hydrogen atom, a trityl group, a mono methoxytrityl group, or a dimethoxytrityl group;
Z¹ and Z² are each independently a C₁₋₆ alkyl group, or
Z¹ and Z² are bonded to each other to form, together with a nitrogen atom bonded thereto, a 3- to 8-membered monocyclic nitrogen-containing non-aromatic heterocycle;
R¹ is a hydrogen atom, a halogen atom, a hydroxy group,
a C₁₋₆ alkyl group optionally substituted by a group selected from a cyano group, a C₁₋₆ alkoxy group, a protected amino group, and an optionally substituted carbamoyl group (preferably, a C₁₋₆ alkyl group),
a C₁₋₆ alkoxy group optionally substituted by a group selected from a cyano group, a C₁₋₆ alkoxy group, a protected amino group, and an optionally substituted carbamoyl group (preferably, a C₁₋₆ alkoxy group optionally substituted by a group selected from a cyano group, a C₁₋₆ alkoxy group, an acylamino group, a carbamoyl group, and a mono- or di-C₁₋₁₀ alkyl-carbamoyl group, more preferably, a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group),
a C₂₋₆ alkenyloxy group optionally substituted by a group selected from a cyano group, a C₁₋₆ alkoxy group, and a protected amino group, and an optionally substituted carbamoyl group (preferably, a C₂₋₆ alkenyloxy group), or
a C₂₋₆ alkynyloxy group optionally substituted by a group selected from a cyano group, a C₁₋₆ alkoxy group, and a protected amino group, and an optionally substituted carbamoyl group (preferably, a C₂₋₆ alkynyloxy group) ;
R² is a hydrogen atom, a halogen atom, a hydroxy group,
a C₁₋₆ alkyl group optionally substituted by a group selected from a cyano group, a C₁₋₆ alkoxy group, a protected amino group, and an optionally substituted carbamoyl group (preferably, a C₁₋₆ alkyl group),
a C₁₋₆ alkoxy group optionally substituted by a group selected from a cyano group, a C₁₋₆ alkoxy group, a protected amino group, and an optionally substituted carbamoyl group (preferably, a C₁₋₆ alkoxy group optionally substituted by a group selected from a cyano group, a C₁₋₆ alkoxy group, an acylamino group, a carbamoyl group, and a mono- or di-C₁₋₁₀ alkyl-carbamoyl group, more preferably, a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group),
a C₂₋₆ alkenyloxy group optionally substituted by a group selected from a cyano group, C₁₋₆ alkoxy group, and a protected amino group, and an optionally substituted carbamoyl group (preferably, a C₂₋₆ alkenyloxy group), or
a C₂₋₆ alkynyloxy group optionally substituted by a group selected from a cyano group, a C₁₋₆ alkoxy group, and a protected amino group, and an optionally substituted carbamoyl group (preferably, a C₂₋₆ alkynyloxy group) ;
R³ is a hydrogen atom, a C₁₋₆ alkyl group, a C₁₋₆ alkoxy group, or a C₁₋₆ alkylthio group, or
R² and R³ are bonded to each other to form a group represented by the following formula (shown in the order of -R²-R³-) :
   -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
   in the above-mentioned formulas, Ra to Rj are each independently a hydrogen atom or a C₁₋₆ alkyl group; and
   Base is an optionally modified nucleobase.

### [Nucleotide derivative (I-C)]

A nucleotide derivative (I-A) or (I-B), wherein
X is a C₁₋₆ alkoxy group,
   a C₆₋₁₄ aryl-oxy group,
   a C₃₋₆ cycloalkyl-oxy group,
   a 5- or 6-membered non-aromatic heterocyclic-oxy group,
   a C₁₋₆ alkyl group,
   a C₂₋₆ alkenyl group, or
   a C₆₋₁₄ aryl group
   (more preferably, a C₁₋₆ alkoxy group,
   a phenyl-oxy group, or a C₁₋₆ alkyl group); and
Y is a dimethoxytrityl group.

### [Nucleotide derivative (I-D)]

A nucleotide derivative (I-A), (I-B) or (I-C), wherein
R¹ is a hydrogen atom, a halogen atom, a hydroxy group,
a C₁₋₆ alkyl group,
a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group,
a C₂₋₆ alkenyloxy group, or
a C₂₋₆ alkynyloxy group; and
R² is a hydrogen atom, a halogen atom, a hydroxy group,
a C₁₋₆ alkyl group,
a C₁₋₆ alkoxy group optionally substituted by a group selected from a cyano group, a C₁₋₆ alkoxy group, a formylamino group, a C₁₋₁₀ alkyl-carbonylamino group, a carbamoyl group, and a C₁₋₁₀ alkyl-carbamoyl group,
a C₂₋₆ alkenyloxy group, or
a C₂₋₆ alkynyloxy group.

As preferred nucleotide derivative residue (II), reference is made to those mentioned above for nucleotide derivative (I).

### [Step (1) in the present invention]

In practicing the present invention, the above-mentioned nucleotide derivative (I) is carried on a solid phase support via a universal linker, more specifically, the nucleotide derivative (I) is linked onto a solid phase support via a covalent bond in an embodiment shown by (solid phase support (SM))-(spacer (L))-(universal linker (UL))-(nucleotide derivative (I)) (hereinafter to be also denoted as "solid phase support linked-nucleotide derivative (I) ") .
When the universal linker can be directly linked onto the solid phase support, the spacer is not essential.

In the following, the conjugate of (solid phase support (SM))-(spacer (L))-(universal linker (UL)) is sometimes simply denoted as a "solid phase support".

In the present specification, "solid phase support" (to be also denoted as "SM") is not particularly limited as long as it is a carrier for solid phase synthesis from which excess reagents can be easily removed by washing. Examples include a glass-based porous support, a porous polymer support such as polystyrene-based porous support, acrylamide-based porous support, and the like, and the like, and a polystyrene-based porous support and a glass-based porous support are preferred.

In the present specification, the "glass-based porous support" refers to a porous support containing glass as a constituent component. For example, porous glass particles (CPG) in a particle form and the like can be mentioned, but it is not limited thereto. More specifically, as the aforementioned CPG, a CPG solid phase support having a long chain amino alkyl spacer (LCAA-CPG solid phase support) is preferably used. In the case of synthesis of a long chain nucleotide, CPG with pores of 20 to 400 nm, more preferably 50 to 200 nm, further preferably 100 nm, is used.

In the present specification, the "polystyrene-based porous support" is a porous support made of a resin mainly composed of a structural unit of styrene or a derivative thereof, and in particular, a polystyrene-based porous support having amino group and/or hydroxy group is preferred.

Examples of the polystyrene-based porous support include a porous support composed of styrene-hydroxystyrene-divinylbenzene-based copolymer particles (JP-A-2005-097545, JP-A-2005-325272 and JP-A-2006-342245), a porous support composed of a styrene-(meth)acrylonitrile-hydroxystyrene-divinylbenzene-based copolymer (JP-A-2008-074979), and the like.

In the present specification, the "acrylamide-based porous support" is a porous support made of a resin mainly composed of a structural unit of acrylamide or a derivative thereof, and in particular, an acrylamide-based porous support having amino group and/or hydroxy group is preferred and an acrylamide-based porous support having a hydroxy group is preferred.

Examples of the acrylamide-based porous support include a porous support composed of an aromatic mono-vinyl compound-divinyl compound-(meth)acrylamide derivative-based copolymer and the like. When the support is an acrylamide-based solid phase support and the content of the structural unit derived from (meth)acryl amide derivative monomer is too low, the effect of preventing a decrease in the amount of nucleic acid synthesized and a decrease in the synthesis purity cannot be obtained, whereas when the content is too high, the formation of porous resin beads becomes difficult. Accordingly, the content is preferably 0.3 to 4 mmol/g, more preferably 0.4 to 3.5 mmol/g, further preferably 0.6 to 3 mmol/g.

The above-mentioned solid phase support may be any solid phase support having a functional group capable of forming a covalent bond with a spacer (cleavable linker). In particular, a solid phase support having an amino group and/or a hydroxy group is preferred. In this case, the bond between the solid phase support (SM) and the spacer (L) is, for example, an amide bond or an ester bond.

In the present invention, while the content of the functional group in the solid phase support is not particularly limited, when the content of the functional group is too low, the yield of the oligonucleotide (nucleic acid) decreases, whereas when the content of the functional group is too high, the purity of the obtained oligonucleotide (nucleic acid) decreases. Those of ordinary skill in the art can appropriately select preferred contents.

In the present specification, the "spacer" (to be also denoted as "L") refers to a molecule that connects the universal linker and the solid phase support via a covalent bond. In order to efficiently cleave the resultant product from the solid phase support in the final stage of solid phase synthesis, it is preferred that the spacer be cleavable, and more preferably, can be hydrolyzed by an alkaline solution such as aqueous ammonia or a methylamine solution. Known spacers used in the pertinent technique field for solid phase synthesis of oligonucleotides can also be used in practicing the present invention.

The "spacer" is as defined above, and is preferably a divalent group represented by the formula (III): 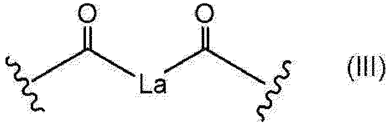
wherein La is an inactive divalent group; and each 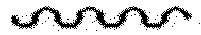
shows a bonding site.

When the structure of the spacer is an asymmetric structure, either the left or right bonding site may be bonded to the nucleotide derivative (I) of the present invention.

The "inactive" in the "inactive divalent group" refers to the absence of a functional group that inhibits solid phase synthesis reactions, such as a hydroxy group, an amino group, a carboxy group, a sulfanyl group, a sulfo group, and the like.

La as the "inactive divalent group" is more preferably an inactive divalent group whose main chain is composed of atoms selected from a carbon atom, an oxygen atom, a sulfur atom, and a nitrogen atom (e.g., 1 to 200 atoms, preferably, 1 to 150 atoms, more preferably, 1 to 100 atoms, further preferably, 1 to 50 atoms, further more preferably, 1 to 10 atoms, and particularly preferably, 1 to 5 atoms).

La is preferably -CH₂OC₆H₄OCH₂-, -CH₂CH₂-, or -CH₂CH₂CH₂-, more preferably -CH₂OC₆H₄OCH₂- or -CH₂CH₂-.

In the present invention, the length of the spacer is not particularly limited. It can be appropriately set according to the chain length of the desired oligonucleotide (nucleic acid) sequence.

In the present invention, the "universal linker" (to be also denoted as "UL") is a linker that is linked to a solid phase support via a covalent bond or a spacer, and is used as a starting point for synthesizing the desired oligonucleotide on the solid phase support. By using this universal linker, the desired oligonucleotide can be obtained, regardless of the type of nucleoside at the 3'-terminus of the desired oligonucleotide, by starting the synthesis by reacting the nucleoside phosphoramidite to be the 3'-terminal in the same step as in a general automated nucleic acid synthesis, synthesizing the desired oligonucleotide, and cleaving same from the solid support by a method similar to the general method.

The universal linker used in the present invention is not particularly limited, and may be the universal linker disclosed or cited in JP-A-2011-088843, the universal linker disclosed in JP-A-2016-204316, or the like. Alternatively, it may be a commercially available universal support in which a universal linker is linked to a solid phase support. More specifically, for example, UnyLinker (trade name) universal support for oligonucleotide synthesis (Chem Genes), Universal Syn Base (trade name) and Universal Q Syn Base (trade name) (Biosearch Technologies), and Universal Support I, II, and III (Glen Research), and the like are used.

In this step (1), the aforementioned "solid phase support linked-nucleotide derivative (I)" can be produced by those of ordinary skill in the art by appropriately applying the specific methods described in Examples/Production Examples below or methods known in the pertinent technique field.

For example, a group represented by HO-L- is reacted with the corresponding acid anhydride to introduce a spacer into a universal linker (UL), and then a reaction with a solid phase support is performed in a solvent in the presence of a condensing agent and a base to obtain a "(solid phase support (SM))-(spacer (L))-(universal linker (UL)) conjugate". The conjugate is then loaded into a reaction column of an oligonucleotide automatic synthesizer and the nucleotide derivative (I) of the present invention, which serves as the starting point for the subsequent nucleotide elongation reaction, is bonded as the first (3'-terminal) nucleotide to the universal linker (UL) by appropriately applying the specific methods described in Examples/Production Examples below or methods known in the pertinent technique field, whereby the "solid phase support linked-nucleotide derivative (I)" can be produced.

When the "(solid phase support (SM))-(spacer (L))-(universal linker (UL)) conjugate" is commercially available, commercially available products may also be used.

### [Step (2) in the present invention]

Step (2) is a step of sequentially condensing nucleic acid monomers according to the desired oligonucleotide sequence, by using the nucleotide derivative (I) of the "solid phase support linked-nucleotide derivative (I)" obtained in step (1) as a starting point, to elongate the nucleotide.

In the "solid phase support linked-nucleotide derivative (I)", the nucleotide derivative (I) specifically takes the form of "nucleotide derivative residue (II)".

The "nucleic acid monomer" here is a nucleoside phosphoramidite appropriately selected according to the oligonucleotide sequence and can be produced by appropriately applying the specific methods described in Examples/Production Examples below or methods known in the pertinent technique field. When the "nucleic acid monomer" is commercially available, commercially available products may also be used. For example, a group of nucleoside phosphoramidites represented by a compound of the following formula (A): 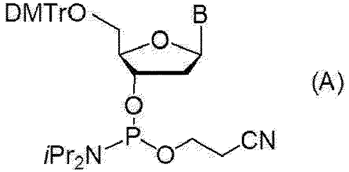 wherein B is an optionally modified nucleobase, can be mentioned.

As the nucleic acid monomer, one or two or more "nucleotide derivatives (I)" of the present invention may also be used.

In the nucleotide elongation reaction in step (2), step A: a step of deprotecting 5' hydroxy group of nucleotide (including nucleotide derivative (I) at the 3'-terminus) - step B: a step of condensing with nucleic acid monomer (phosphoramidite) - step C: a step of oxidizing/sulfurizing nucleotide linked moiety, are successively repeated once or twice or more according to the desired nucleic acid sequence, whereby an oligonucleotide having the desired sequence can be obtained. The respective steps A to C can be performed by those of ordinary skill in the art by appropriately applying the specific methods described in Examples/Production Examples below or methods known in the pertinent technique field.

### [Step (3) in the present invention]

Step (3) is a step of subjecting the obtained oligonucleotide having the desired sequence to a reaction for cleaving from the solid phase support.

The reaction for cleaving in this step can be performed by those of ordinary skill in the art by appropriately applying the specific methods described in Examples/Production Examples below or methods known in the pertinent technique field. For example, it is performed by treating the solid phase support on which the oligonucleotide has been bonded with ammonia, amines and/or base and recovering the oligonucleotide with the desired sequence.

Examples of ammonia include concentrated aqueous ammonia. Examples of amines include alkylamine (methylamine, ethylamine, isopropylamine, ethylenediamine, diethylamine, triethylamine, etc.). Examples of base include metal carbonates such as potassium carbonate and the like. One or more kinds of ammonia, amines, and/or base can be used in combination.

It is desirable to use ammonia, amines, and/or base in a mixture with a solvent. Examples of the solvent include water, alcohols (e.g., methanol, ethanol, etc.) and the like. Two or more kinds of these solvents may be used in a mixture in an appropriate ratio.

More specifically, step (3) is performed by pouring a solution of ammonia, amines, and/or base into a column containing a solid phase support to which an oligonucleotide has been bonded, thereby bringing the solution into contact with the solid phase support to which the oligonucleotide is bonded.

In the reaction for cleaving in step (3), the reaction temperature thereof is appropriately selected according to the reaction conditions and is not particularly limited. The reaction is generally performed in the range of from room temperature to under warming. The reaction time is appropriately selected according to the reaction conditions and is not particularly limited, but the reaction is generally performed within the range of 1 minute to 10 hours.

The reaction for cleaving in step (3) of the present invention is preferably performed by contacting the solid phase support, on which the oligonucleotide has been carried via a universal linker, with
1) concentrated aqueous ammonia at room temperature to under warming,
2) a mixed solution of concentrated alkylamine aqueous solution/concentrated aqueous ammonia at room temperature to under warming,
3) an alcohol solution of potassium carbonate at room temperature to under warming, or
4) a mixed solution of alkylamine/water or alkylamine/water/alcohol at room temperature to under warming.

Further preferably, it is performed by contacting the solid phase support, on which the oligonucleotide has been carried via a universal linker, with
1) 28% aqueous ammonia at room temperature,
2) a 1:1 mixed solution of 40% methylamine aqueous solution/28% aqueous ammonia (AMA solution) at room temperature,
3) a methanol solution of 50 mM potassium carbonate at room temperature, or
4) a mixed solution of t-butylamine/water or t-butylamine/water/methanol under warming.

In the present invention, the nucleotide derivative (I) is used as the 3'-terminal nucleotide in oligonucleotide synthesis. Because of this, the present invention is characterized in that the desired oligonucleotide can be efficiently cleaved not only under general cleavage conditions, but also under cleavage conditions with extremely mild basic conditions. This point is specifically described in the Examples below.

### [Universal linker having a simple structure in the present invention]

As described above, in oligonucleotide synthesis using the nucleotide derivative (I) of the present invention, the desired oligonucleotide can be efficiently cleaved even under extremely mild cleavage conditions. The present inventors have also found that a simple 1,2-diol derivative having a 1,2-diol structure can be used as a universal linker when the nucleotide derivative (I) of the present invention is used.

More specifically, they have found that a 1,2-diol derivative represented by the following formula (III):

Y¹-O-C (X¹) (X²) C (X³) (X⁴) -O-Y² (III)

wherein X¹ to X⁴ are each independently a hydrogen atom or a chain or cyclic substituent, wherein any two groups of X¹ to X⁴ are bonded to each other at a bondable position to form, together with a carbon atom bonded thereto, an optionally substituted ring; and
Y¹ and Y² are each a hydrogen atom or a hydroxy-protecting group
(hereinafter to be also denoted as "1,2-diol derivative (III)") can be used as a universal linker.

Each group in the 1,2-diol derivative (III) is described below.

As the "chain or cyclic substituent", the "alkyl group", "alkenyl group", "alkynyl group", and "alkoxy group" described above for the nucleotide derivative (I) can be mentioned as the "chain substituent", and the aforementioned "cyclic group" can be mentioned as the "cyclic substituent". As used herein, each group may be substituted.

As the ring in the "optionally substituted ring", "heterocycle" can be mentioned.

Examples of the "heterocycle" include an aromatic heterocycle and a non-aromatic heterocycle, each containing, as a ring-constituting atom besides carbon atom, 1 to 4 hetero atoms selected from a nitrogen atom, a sulfur atom, and an oxygen atom.

Examples of the "aromatic heterocycle" include a 5- to 14-membered (preferably, 5- to 10-membered, more preferably, 5-or 6-membered) aromatic heterocycle containing, as a ring-constituting atom besides carbon atom, 1 to 4 hetero atoms selected from a nitrogen atom, a sulfur atom and an oxygen atom. Preferred examples of the "aromatic heterocycle" include 5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine, and the like.

In addition, examples of the "non-aromatic heterocycle" include a 3- to 14-membered (preferably, 4- to 10-membered, more preferably, 5- or 6-membered) non-aromatic heterocycle containing, as a ring-constituting atom besides carbon atom, 1 to 4 hetero atoms selected from a nitrogen atom, a sulfur atom and an oxygen atom. Preferred examples of the "non-aromatic heterocycle" include 3- to 8-membered (more preferably, 5- or 6-membered) monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydroxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepane, diazepane, azepine, azocane, diazocane, oxepane, and the like.

Examples of the "hydroxy-protecting group" include the "hydroxy-protecting group" described above for nucleotide derivative (I).

Specific preferred examples of each group above and the substituent with which each group is optionally substituted include those described for the nucleotide derivative (I).

Preferred embodiments of each group in the 1,2-diol derivative (III) are as follows.
X¹ - X⁴ are each independently a hydrogen atom,
any two groups (preferably, two groups on adjacent carbon atoms) of X¹ to X⁴ are bonded to each other at a bondable position to form, together with a carbon atom bonded thereto, an optionally substituted 5- or 6- membered non-aromatic heterocycle (more preferably, 5- or 6-membered oxygen-containing non-aromatic heterocycle, most preferably, tetrahydrofuran);
Y¹ is a hydrogen atom or a trityl-based protecting group (more preferably, a 4,4'-dimethoxytrityl group); and
Y² is a hydrogen atom.

The usefulness of the 1,2-diol derivative (II) as a universal linker is specifically described in the Examples below.

Another preferred embodiment of the present invention is as follows.
[1-A] A solid phase synthesis method of an oligonucleotide, comprising a step of subjecting an oligonucleotide having, at the 3'-terminus of an oligonucleotide sequence thereof, a nucleotide derivative represented by the formula (II): 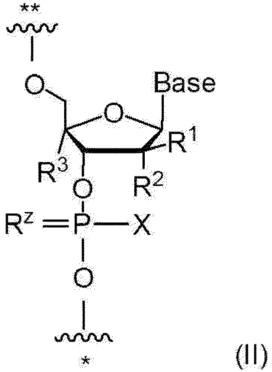
   wherein * is a bonding position to the universal linker;
   ** is a bonding position to the nucleotide or oligonucleotide;
   R^{z} is O or S;
   X is an optionally substituted alkoxy group (excluding a cyanoethoxy group),
   an optionally substituted cyclic group-oxy group,
   an optionally substituted alkyl group,
   an optionally substituted alkenyl group, or
   an optionally substituted aryl group;
   R¹ is a hydrogen atom, a halogen atom, an optionally protected hydroxy group, an optionally substituted alkyl group, or an optionally substituted alkoxy group;
   R² is a hydrogen atom, a halogen atom, an optionally protected hydroxy group, an optionally substituted alkyl group, or an optionally substituted alkoxy group, and
   R³ is a hydrogen atom, an optionally substituted alkyl group, an optionally substituted alkoxy group, or an optionally substituted alkylthio group,
      or
   R² and R³ are bonded to each other to form a group represented by the following formula (shown in the order of -R²-R³-) : -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
   in the above-mentioned formulas, Ra to Rj are each independently a hydrogen atom or an optionally substituted alkyl group; and
   Base is an optionally modified nucleobase,
   which oligonucleotide is carried on a solid phase support via a universal linker and synthesized by a nucleotide elongation reaction in a solid phase synthesis method of an oligonucleotide,
   to a reaction for cleaving the oligonucleotide from the solid phase support and the universal linker.
[2-A] A solid phase synthesis method of an oligonucleotide, comprising
   (1) a step of carrying a nucleotide derivative represented by the formula (I): 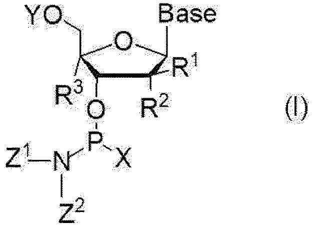
      wherein X is an optionally substituted alkoxy group (excluding a cyanoethoxy group), an optionally substituted cyclic group-oxy group,
      an optionally substituted alkyl group,
      an optionally substituted alkenyl group, or
      an optionally substituted aryl group;
      Y is a hydrogen atom or a hydroxy-protecting group;
      Z¹ and Z² are each independently an optionally substituted alkyl group, or
      Z¹ and Z² are bonded to each other to form, together with a nitrogen atom bonded thereto, an optionally substituted heterocycle;
      R¹ is a hydrogen atom, a halogen atom, an optionally protected hydroxy group, an optionally substituted alkyl group, or an optionally substituted alkoxy group;
      R² is a hydrogen atom, a halogen atom, an optionally protected hydroxy group, an optionally substituted alkyl group, or an optionally substituted alkoxy group, and
      R³ is a hydrogen atom, an optionally substituted alkyl group, an optionally substituted alkoxy group, or an optionally substituted alkylthio group,
         or
      R² and R³ are bonded to each other to form a group represented by the following formula (shown in the order of -R²-R³-) : -O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
      in the above-mentioned formulas, Ra to Rj are each independently a hydrogen atom or an optionally substituted alkyl group; and
      Base is an optionally modified nucleobase, on a solid phase support via a universal linker,
   (2) a step of sequentially condensing nucleic acid monomers according to the desired oligonucleotide sequence to elongate the nucleotide (one or two or more nucleotide derivatives represented by the above-mentioned formula (I) may also be used as the nucleic acid monomers),
   (3) a step of subjecting the obtained oligonucleotide having the desired sequence to a reaction for cleaving from the solid phase support and the universal linker.
[3-A] The solid phase synthesis method of an oligonucleotide of the above-mentioned [1-A] or [2-A], wherein the reaction for cleaving from the solid phase support and the universal linker is performed by contacting the solid phase support, on which the oligonucleotide is carried via a universal linker, with
   1) concentrated aqueous ammonia at room temperature to under warming,
   2) a mixed solution of concentrated alkylamine aqueous solution/concentrated aqueous ammonia at room temperature to under warming,
   3) an alcohol solution of potassium carbonate at room temperature to under warming, or
   4) a mixed solution of alkylamine/water or alkylamine/water/alcohol at room temperature to under warming.
[4-A] The solid phase synthesis method of an oligonucleotide of the above-mentioned [3-A], wherein the reaction for cleaving from the solid phase support is performed by contacting the solid phase support, on which the oligonucleotide is carried via a universal linker, with
   1) 28% aqueous ammonia at room temperature,
   2) a 1:1 mixed solution of 40% methylamine aqueous solution/28% aqueous ammonia (AMA solution) at room temperature,
   3) a methanol solution of 50 mM potassium carbonate at room temperature, or
   4) a mixed solution of t-butylamine/water or t-butylamine/water/methanol under warming.

### [Example]

The present invention is described in detail in the following by referring to specific Examples. However, the present invention is not limited thereto.

### A. Preparation of nucleotide derivative

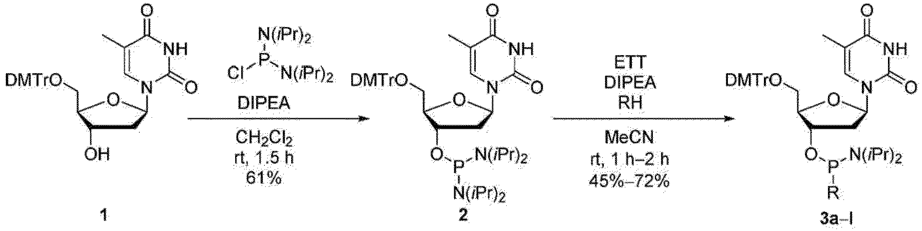

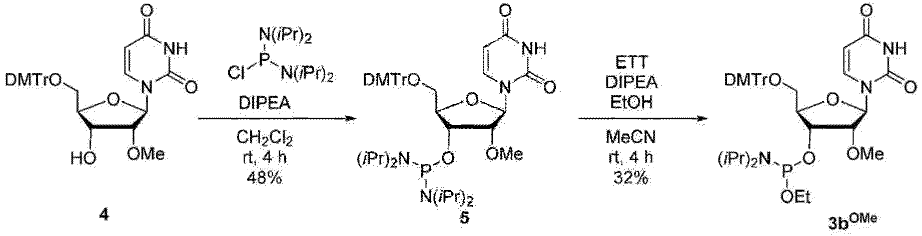

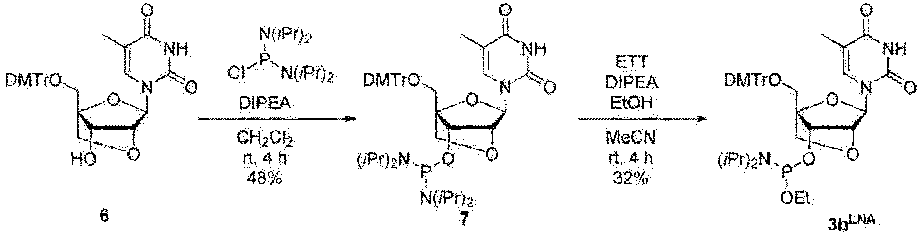

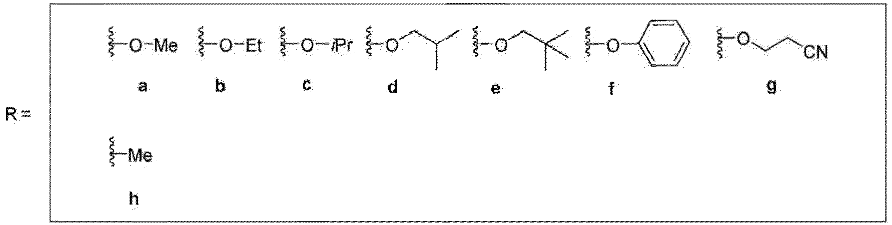

In the above-mentioned formulas, DMTr is a 4,4'-dimethoxytrityl group, Me is a methyl group, Et is an ethyl group, iPr is an isopropyl group, DIPEA is diisopropyl ethylamine, and ETT is ethylthiotetrazole.

In the following, phosphoramidites represented by the above-mentioned formulas 3a to 3f, and 3h are also denoted as nucleotide derivatives 3a to 3f, and 3h, and phosphoramidite represented by the formula 3g is also denoted as compound 3g. Compound 3g is a known phosphoramidite and used as a control for the nucleotide derivative (I) of the present invention.

1. Commercially available products (purchased from Glen Research) were used for nucleotide derivative 3a, compound 3g, and nucleotide derivative 3h.
2. Nucleotide derivatives 3b - 3f were synthesized by the following methods.

### Production Example A1: Synthesis of compound 2

It was synthesized according to a reported literature (Bioconjugate Chem., 2008, 19, 1696-1706). Under an argon stream, to a solution of commercially available compound 1 (3.00 g, 5.51 mmol) in anhydrous dichloromethane (20 mL) was added diisopropyl ethylamine (2.40 mL, 13.2 mmol). At 0°C, bis(diisopropylamino)chlorophosphine (1.56 g, 6.61 mmol) was added, and the mixture was stirred at room temperature for 1.5 hr. After completion of the reaction, the reaction mixture was evaporated under reduced pressure, and a crude product was purified by silica gel column chromatography (hexane/ethyl acetate=2:1) to give compound 2 (2.62 g, yield 61%) as a white solid.

### Example A1: Synthesis of nucleotide derivative 3b

It was synthesized according to a reported literature (Bioorg. Med. Chem. Lett., 2015, 25, 3610-3615.). Under an argon stream, to a solution of compound 2 (150 mg, 0.194 mmol) in anhydrous acetonitrile (2 mL) were added diisopropyl ethylamine (40.5 µL, 0.232 mmol) and ethanol (13.5 µL, 0.232 mmol). Ethylthiotetrazole (30.2 mg, 0.232 mmol) was added, and the mixture was stirred at room temperature for 1 hr. After completion of the reaction, the mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=3:1) to give nucleotide derivative 3b (78.0 mg, yield 56%) as a white solid.

### Example A2: Synthesis of nucleotide derivative 3c

Under an argon stream, to a solution of compound 2 (150 mg, 0.194 mmol) in anhydrous acetonitrile (2 mL) were added diisopropyl ethylamine (40.5 µL, 0.232 mmol) and isopropanol (17.9 µL, 0.232 mmol). Ethylthiotetrazole (30.2 mg, 0.232 mmol) was added, and the mixture was stirred at room temperature for 2 hr. The mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=3:1) to give nucleotide derivative 3c (64.0 mg, yield 45%) as a white solid.
¹H-NMR (500 MHz,CDCl₃) δ7.99(brs,1H), 7.66,7.63(d×2,1H,J=1.0Hz), 7.42-7.39(m,2H), 7.31-7.22(m,6H), 6.84-6.81(m,4H), 6.44-6.39(m,1H), 4.65-4.59(m,1H), 4.21,4.18(d×2,1H,J=2.0Hz), 4.07-3.89(m,1H), 3.79(s,6H), 3.59-3.46(m,3H), 3.36-3.30(m,1H), 2.57-2.45(m,1H), 2.33-2.26(m,1H), 1.39-1.36(m,3H), 1.26-1.04(m,18H) ³¹P-NMR (202 MHz, CDCl₃) δ145.2,144.6
IR(ATR):2966.0,2928,1686,1607,1508cm⁻¹
HRMS (ESI-TOF) :calcd. for C₄₀H₅₂N₃aO₈P
[M+Na]⁺756.3390, found 756.3391

### Example A3: Synthesis of nucleotide derivative 3d

Under an argon stream, to a solution of compound 2 (150 mg, 0.194 mmol) in anhydrous acetonitrile (2 mL) were added diisopropyl ethylamine (40.5 µL, 0.232 mmol) and 2-methylpropanol (21.5 µL, 0.232 mmol). Ethylthiotetrazole (30.2 mg, 0.232 mmol) was added, and the mixture was stirred at room temperature for 1 hr. The mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=3:1) to give nucleotide derivative 3d (68.0 mg, yield 44%) as a white solid.
¹H-NMR (500 MHz, CDCl₃) δ 7.96 (brs, 1H), 7.66,7.62 (brs×2, 1H), 7.41-7.39(m,2H), 7.31-7.22(m,6H), 6.83-6.82(m,4H), 6.43-6.39(m,1H), 4.67-4.62(m,1H), 4.22,4.15(d×2,1H,J=2.0Hz), 3.79(s,6H), 3.61-3.46(m,3H), 3.42-3.16(m,3H), 2.56-2.45(m,1H), 2.33-2.28(m,1H), 1.88-1.66(m,1H), 1.40,1.39(s×2,3H), 1.16-
1.04(m,12H), 0.92-0.81(m,6H)
³¹P-NMR (202 MHz, CDCl₃) δ147.2,146.9
IR(ATR):2963.0,2929,1686,1607,1508cm⁻¹
HRMS(ESI-TOF):calcd. for C₄₁H₅₄N₃NaO₈P
[M+Na]⁺ 770.3546, found 770.3539

### Example A4: Synthesis of nucleotide derivative 3e

Under an argon stream, to a solution of compound 2 (200 mg, 0.258 mmol) in anhydrous acetonitrile (2 mL) were added diisopropyl ethylamine (54.1 µL, 0.310 mmol) and 2,2-dimethyl-1-propanol (27.3 mg, 0.310 mmol). Ethylthiotetrazole (40.4 mg, 0.310 mmol) was added, and the mixture was stirred at room temperature for 2 hr. After completion of the reaction, the mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=5:1) to give nucleotide derivative 3e (94.8 mg, yield 47%) as a white solid. ¹H-NMR (500 MHz,CDCl₃) δ8.03(brs,1H), 7.66,7.61(d×2,1H,J=1.0Hz), 7.40(dd,2H,J=2.0Hz,J=8.0Hz), 7.30-7.22(m,6H), 6.84-6.81(m,4H), 6.43-6.40(m,1H), 4.68-4.62(m,1H), 4.20,4.14(d×2,1H,J=2.0Hz), 3.79(s,6H), 3.60-3.45(m,3H), 3.39-3.26(m,2H), 3.19-3.06(m,1H)2.55-2.45(m,1H), 2.33-2.32(m,1H), 1.40-1.38(m,3H), 1.27-1.25(m,1H), 1.17-1.04(m,12H), 0.91,0.82(s×2,9H)
³¹P-NMR (202 MHz, CDCl₃) δ147.6,147.4
IR(ATR)cm⁻¹:2962,1684,1607,1508
HRMS (ESI-TOF):calcd. for C₄₂H₅₆N₃NaO₈P [M+Na]⁺ 784.3703, found 784.3701

### Example A5: Synthesis of nucleotide derivative 3f

It was synthesized according to a reported literature (J. Org. Chem., 1995, 60, 925-930.). Under an argon stream, to a solution of compound 2 (500 mg, 0.645 mmol) in anhydrous acetonitrile (6 mL) were added diisopropyl ethylamine (270 µL, 1.55 mmol) and phenol (70.0 mg, 0.774 mmol). Ethylthiotetrazole (101 mg, 0.774 mmol) was added, and the mixture was stirred at room temperature for 3 hr. After completion of the reaction, a 5% sodium hydrogen carbonate aqueous solution was added to quench the reaction, and the mixture was diluted with ethyl acetate. The organic layer was washed twice with water and once with saturated brine, and dried over sodium sulfate. The solvent was evaporated under reduced pressure. A crude product was purified by silica gel column chromatography (hexane/ethyl acetate=2:1) to give nucleotide derivative 3f (328 mg, yield 63%) as a white solid.

### Production Example A2: Synthesis of compound 5

Under an argon stream, to a solution of commercially available compound 4 (1.0 g, 1.88 mmol) in anhydrous dichloromethane (10 mL) was added diisopropyl ethylamine (0.788 mL, 4.51 mmol). At 0°C, bis(diisopropylamino)chlorophosphine (534 mg, 2.26 mmol) was added, and the mixture was stirred at room temperature for 1.5 hr. After completion of the reaction, the reaction mixture was evaporated under reduced pressure and a crude product was purified by silica gel column chromatography (hexane/ethyl acetate=2:1) to give compound 5 (1.09 g, yield 73%) as a white solid.
¹H-NMR (500 MHz, CDCl₃) δ 8.34(brs,1H), 8.01(d,2H,J=8.0Hz), 7.40(d,2H,J=7.5Hz), 7.30-7.22(m,7H), 6.84-6.82(m,4H), 6.02(d,J=8.0Hz,1H), 5.25(d,J=8.0Hz,1H), 4.37-4.33(m,1H), 4.26-4.25(m,1H), 3.88(dd,J=4.0,3.5Hz,1H), 3.79(s,6H), 3.59-3.42(m,6H), 3.56(s,3H), 1.17(d,J=2Hz,6H), 1.16(d,J=2Hz,6H), 1.14(d,J=7.0Hz,6H), 1.04(d,J=6.5Hz,6H).
¹³C-NMR (125 MHz,CDCl₃) δ162.9, 158.7, 149.9, 144.3, 140.2, 135.3, 135.1, 130.3, 128.3, 127.9, 127.1, 113.2, 101.9, 87.4, 87.2, 84.0, 83.9, 82.9, 82.8, 70.4, 70.3, 62.1, 58.6, 55.2, 44.7, 44.6, 24.6, 24.5, 24.4, 24.2, 24.1, 24.0.
³¹P-NMR (202 MHz, CDCl₃) δ118.3.
IR(ATR)cm⁻¹:2968,1684,1508.
HRMS (FAB):calcd for C₄₃H₆₀N₄O₈P [M+H]⁺,791.4149, found 791.4147.

### Production Example A3: Synthesis of compound 7

Under an argon stream, to a solution of commercially available compound 6 (310 mg, 0.543 mmol) in anhydrous dichloromethane (5 mL) was added diisopropyl ethylamine (0.227 mL, 1.30 mmol). At 0°C, bis(diisopropylamino)chlorophosphine (154 mg, 0.652 mmol) was added, and the mixture was stirred at room temperature for 4 hr. After completion of the reaction, the reaction mixture was evaporated under reduced pressure, and a crude product was purified by silica gel column chromatography (hexane/ethyl acetate=2:1) to give compound 7 (210 mg, yield 48%) as a white solid.
¹H-NMR (500 MHz,CDCl₃) δ8.30(brs,1H), 7.69(d,1H,J=1.0Hz), 7.49-7.47(m,2H), 7.37-7.34(m,4H), 7.30-7.22(m,5H), 6.84-6.82(m,4H), 4.59 (s, 1H), 4.08(d,1H,J=9.0Hz), 3.89, 3.85(ABq,2H,J=18.0,7.5Hz), 3.79(s,6H), 3.58(d,1H,J=11.0Hz), 3.43-3.36(m,5H), 1.73(s,3H), 1.13(d,J=6.5Hz,6H), 1.10(d,J=7.0Hz,6H), 1.03(d,J=6.5Hz,6H), 0.91(d,J=6.5Hz,6H).
³¹P-NMR (202 MHz, CDCl₃) δ117.6.
IR(ATR)cm⁻¹:2966,1687,1607,1508.
HRMS (ESI-TOF):calcd for C₄₄H₆₀N₄O₈P
[M+H]⁺ 803.4149, found 803.4148.

### Example A6: Synthesis of nucleotide derivative 3b^{OMe}

Under an argon stream, to a solution of compound 5 (600 mg, 0.759 mmol) in anhydrous acetonitrile (5 mL) were added diisopropyl ethylamine (0.16 mL, 0.911 mmol) and ethanol (53 µL, 0.911 mmol). Ethylthiotetrazole (119 mg, 0.911 mmol) was added, and the mixture was stirred at room temperature for 2.5 hr. The mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=4:1) to give compound 3b^{OMe} (219 mg, yield 39%) as a white solid.
¹H-NMR (500 MHz,CDCl₃) δ8.73(brs,1H), 8.08(d,0.3H,J=8.0Hz,1H), 8.04(d,0.7H,J=8.0Hz), 7.86(brs,1H), 7.43-7.36(m,2H), 7.37-7.22(m,7H), 6.85-6.82(m,4H), 5.99(d,0.7H,J=8.0Hz), 5.97(d,0.3H,J=8.0Hz), 5.18-5.15(m,1H), 4.59-4.55(m,0.3H), 4.48-4.44(m,0.7H), 4.24-4.21(m,1H), 3.84-3.82(m,1H), 3.80(s,6H), 3.67-3.47(m,9H), 1.26-1.03(m,16H).
³¹P-NMR (202 MHz, CDCl₃) δ148.3,147.9.
IR(ATR)cm⁻¹:2966,1607,1508.
HRMS (FAB) :calcd for C₃₉H₅₁N₃O₉P [M+H]⁺ 736.3363, found 736.3375.

### Example A7: Synthesis of nucleotide derivative 3b^{LNA}

Under an argon stream, to a solution of compound 7 (110 mg, 0.134 mmol) in anhydrous acetonitrile (2 mL) were added diisopropyl ethylamine (28.6 µL, 0.164 mmol) and ethanol (9.6 µL, 0.164 mmol). Ethylthiotetrazole (21.3 mg, 0.164 mmol) was added, and the mixture was stirred at room temperature for 4 hr. The mixture was diluted with ethyl acetate, and the organic layer was washed twice with water and once with saturated brine, and dried over sodium sulfate. The solvent was evaporated under reduced pressure. A crude product was purified by silica gel column chromatography (hexane/ethyl acetate=3:1) to give nucleotide derivative 3b^{LNA (}33.0 mg, yield 32%) as a white solid.
¹H-NMR (500 MHz,CDCl₃) δ8.21(brs,1H), 7.70(brs,1H), 7.49-7.46(m,2H), 7.37-7.22(m,7H), 6.85-6.82(m,4H), 5.65(s,0.7H), 5.64(s,0.3H), 4.57(s,0.7H), 4.51(s,0.3H), 4.35(d,0.3H,J=9.0Hz), 4.26(d,0.7H,J=6.5Hz), 3.87 (d, 1H, J=7 . 5Hz), 3.80-3.79(m,7H),
3.71-3.47(m,5H), 3.45-3.41(m,1H), 1.61(s,3H), 1.22-0.99(m,15H). ³¹P-NMR (202 MHz, CDCl₃) δ147.2.
IR(ATR)cm⁻¹:2966,1687,1607,1508.
HRMS (ESI-TOF):calcd for C₄₀H₅₀N₃NaO₉P
[M+Na]⁺ 770.3182, found 770.3182.

### B. Preparation of resin

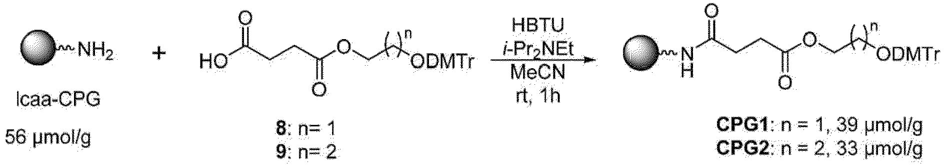

wherein DMTr is a 4,4'-dimethoxytrityl group, CPG is a porous glass particle (controlled pore glass), and HBTU is 2-(1H-benzotriazol-1-yl)-1,1,3,3-tetramethyluronium hexafluorophosphate.

### Example B1: Preparation of CPG1

Under an argon stream, to a suspension of solid phase support lcaa-CPG (Amino-SynBase^{™} CPG 1000/110 (LCAA), manufactured by Biosearch Technologies) (200 mg, amino group content: 11.2 µmol) in anhydrous acetonitrile (1 mL) were added diisopropyl ethylamine (3.80 µL, 22.4 µmol), HBTU (4.25 mg, 11.2 µmol) and compound 8 (5.20 mg, 11.2 µmol) synthesized according to a literature (Bioorganic & Med. Chem., 1997, 5, 2235-2243.). After stirring at room temperature for 1 hr, the reaction mixture was filtered, and a solid phase support was washed with acetonitrile and further vacuum dried overnight. The obtained solid phase support was suspended in CapA solution (acetonitrile solution of 1-methylimidazole, 0.5 mL) and CapB solution (acetonitrile solution of acetic anhydride, 0.5 mL) and the suspension was stirred for 30 min. The reaction mixture was filtered again, and the solid phase support was washed with acetonitrile and vacuum dried overnight to give CPG1 solid phase support. The amount of compound 8 supported by CPG1 was quantified by the DMTr assay method (*) and found to be 39±2.8 mol/g.

### Example B2: Preparation of CPG2

By a method similar to that used for CPG1 and using compound 9 (5.36 mg, 11.2 µmol) synthesized according to a literature (Nucleic Acids Res., 1987, 15, 3113-3129.), CPG2 solid phase support was synthesized. The amount of compound 9 supported by CPG2 was quantified by the DMTr assay method and found to be 33±0.9 mol/g.
(*) DMTr assay method: a method for indirectly quantifying the supported amount by treating solid phase support with a deblocking solution (3 w/v% dichloromethane solution of trichloroacetic acid) and performing absorbance measurement (504 nm) of the amount of deprotected DMTr (dimethoxytrityl) group.

### C. Synthesis of oligonucleotide

Oligonucleotide was synthesized using a GeneDesign nS-8II Oligonucleotides Synthesizer (manufactured by GeneDesign, Inc.) and according to the general phosphoramidite method. The synthesis scale was set to 0.2 pmol, and the synthesis was performed under trityl OFF conditions. Commercially available phosphoramidite of T (same as compound 3g), commercially available phosphoramidite of ^{Ac}C, commercially available phosphoramidite of ^{Pac}A, and commercially available phosphoramidite of ^{iPrPac}G were prepared as 0.1 M solutions in anhydrous acetonitrile and used. As the activator, 5-ethylthio-1H-tetrazole (0.25 M anhydrous acetonitrile solution) was used, and the condensation time was 10 minutes for nucleotide derivatives 3a-3f, 3h, and compound 3g (all in the case of condensation with universal linker), and 25 seconds for commercially available phosphoramidite of T, commercially available phosphoramidite of ^{Ac}C, commercially available phosphoramidite of ^{Pac}A, and commercially available phosphoramidite of ^{iPrPac}G (all in the case of condensation with nucleotide other than universal linker).

The outline of the synthesis scheme and the structures of the synthesized oligonucleotides are shown in the following. In the present specification, ^{Ac}C, ^{Pac}A, and ^{iPrPac}G are respectively also denoted as C^{Ac}, A^{Pac}, and G^{iPrPac}.

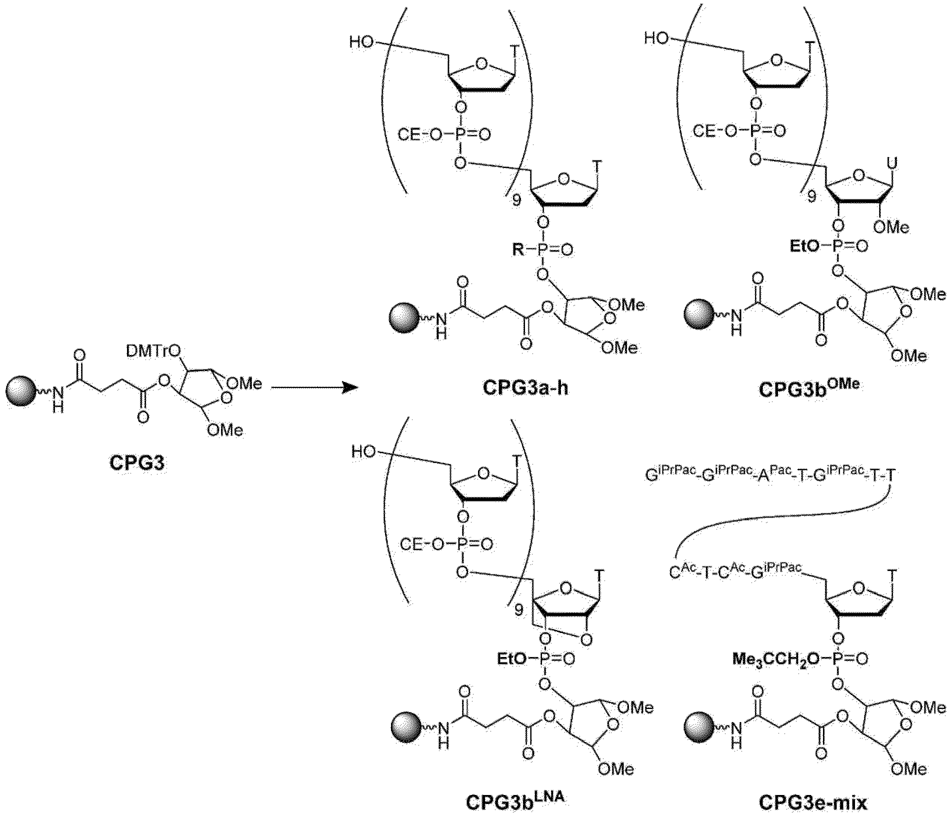

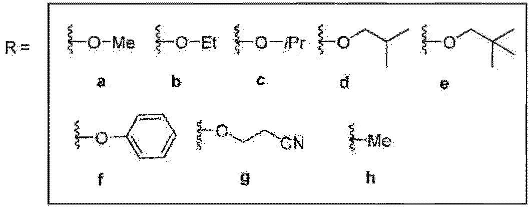

In the above-mentioned formulas, DMTr, Me and Et are each as defined above, CE is a 2-cyanoethyl group, T is a thyminyl group, U is a uracil group, C^{Ac} is a N-acetylcytosyl group, A^{Pac} is a N-phenoxyacetyladenyl group, and G^{iPrPac} is a N-(4-isopropylphenoxyacetyl)guanyl group (here, thyminyl group, uracil group, cytosyl group, adenyl group, and guanyl group respectively show thymine monovalent group, uracil monovalent group, cytosine monovalent group, adenine monovalent group, and guanine monovalent group).

When T, C^{Ac}, A^{Pac}, and G^{iPrPac} are described as nucleic acid monomers constituting oligonucleotide sequences, each nucleobase means a deoxyribonucleotide bonded to deoxy-D-ribose. When U is described as a nucleic acid monomer constituting an oligonucleotide sequence, the nucleobase means a ribonucleotide bonded to D-ribose.

### Example C1: Synthesis of CPG3a-h, CPG3b^{OMe}, CPG3b^{LNA}, and CPG3e-mix

Using commercially available CPG3 (Universal Support manufactured by Glen Research) as a solid phase support and a DNA automatic synthesizer, T-10 mer oligonucleotides (CPG3a-h) into which the aforementioned nucleotide derivatives 3a-3f, 3h, 3b^{OMe}, 3b^{LNA}, compound 3g were introduced at the 3'-terminus, T-9 mer-U^{OMe} oligonucleotide (CPG3b^{OMe}), T-9 mer-T^{LNA} oligonucleotide (CPG3b^{LNA}), and (11 mer constituted of plural kinds of nucleic acid monomers)-T oligonucleotide (CPG3e-mix) were synthesized.

### Example C2: Synthesis of CPG1a and g/CPG2a and g

The outline of the synthesis scheme and the structures of the synthesized oligonucleotides are shown in the following.

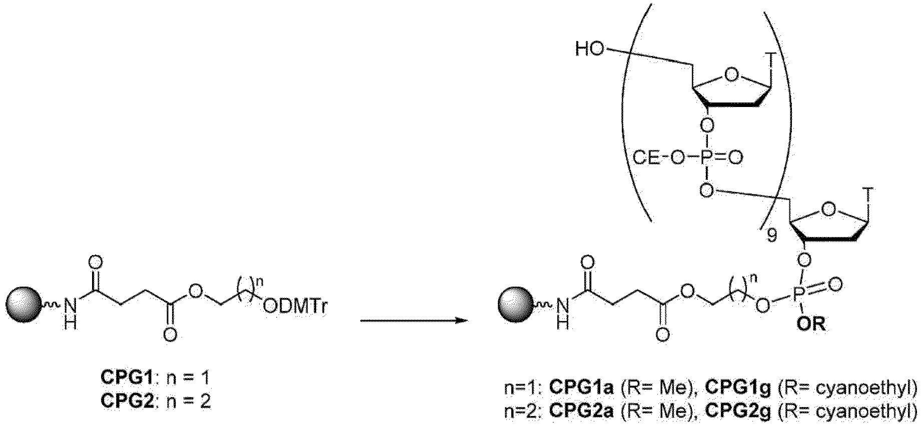

In the above-mentioned formulas, DMTr, CE, and T are each as defined above.

(1) CPG1a and g:
   Using CPG1 synthesized according to Example B1 as a solid phase support and a DNA automatic synthesizer, T-10 mer oligonucleotides (CPG1a and CPG1g) into which nucleotide derivative 3a or compound 3g was introduced at the 3'-terminus were synthesized.
(2) CPG2a and g:
   Using CPG2 synthesized according to Example B2 as a solid phase support, and according to a method similar to that for CPG1a, T-10 mer oligonucleotides (CPG2a and CPG2g) were synthesized.

### D. Evaluation of cleavage efficiency of oligonucleotide from solid phase (CPG)

### Example D1: Cleavage from CPG3a-h, CPG3b^{OMe}, CPG3b^{LNA}, and CPG3e-mix

The outline of the cleavage of oligonucleotide is shown in the following.

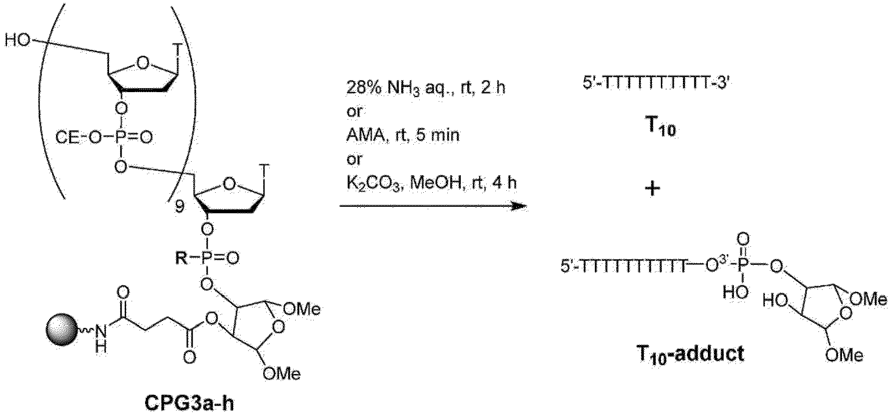

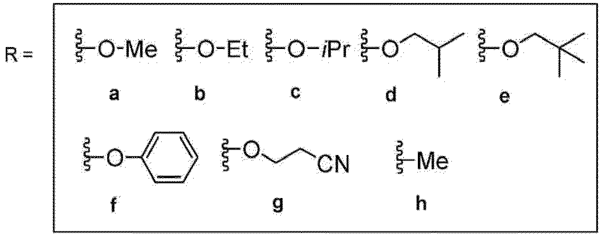

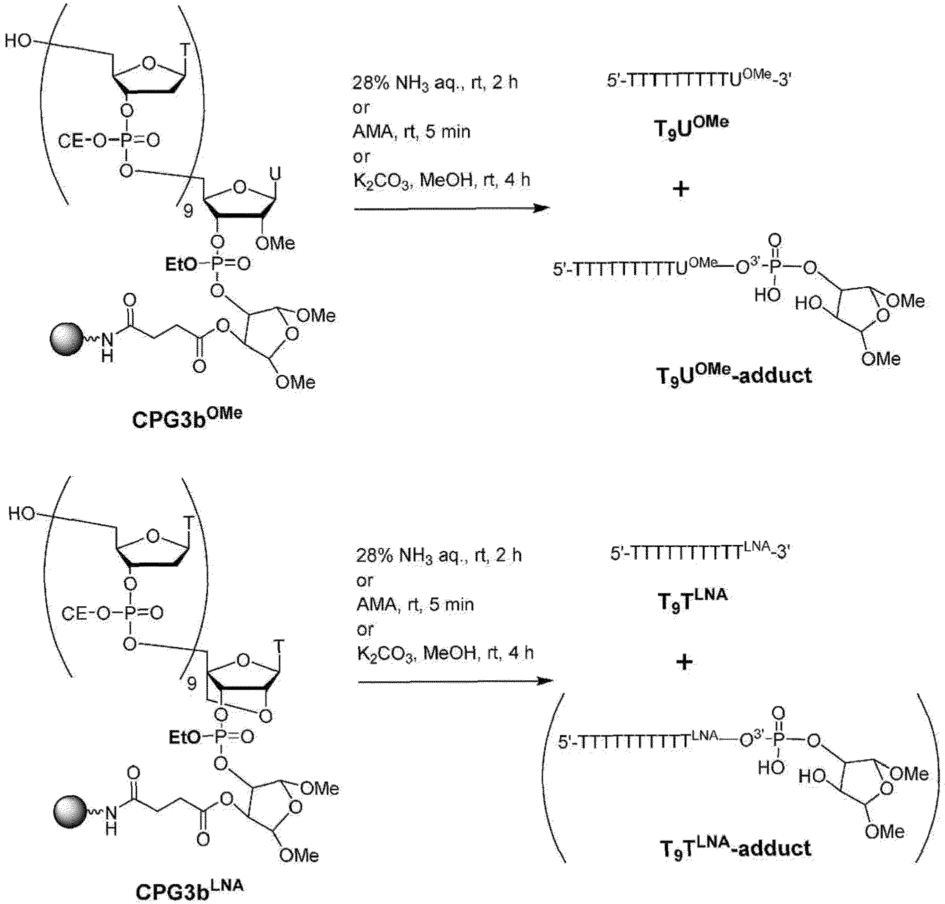

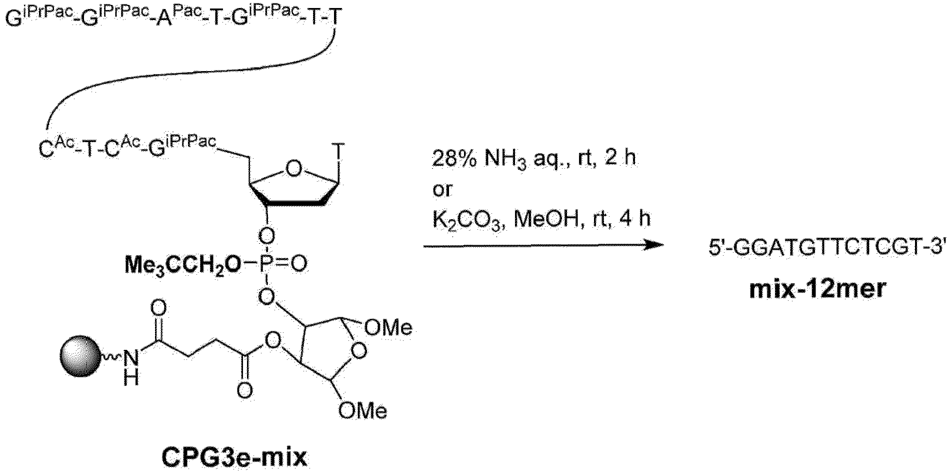

In the above-mentioned formulas, CE, T, Me, Et, U, C^{Ac}, A^{Pac}, and G^{iPrPac} are each as defined above,
T₁₀ is T-10 mer oligonucleotide (SEQ ID NO: 1),
T₁₀-adduct is an oligonucleotide in which a universal linker is bonded to a T-10 mer oligonucleotide without being cleaved, T₉U^{OMe} is T-9 mer-U^{OMe} oligonucleotide (SEQ ID NO: 2),
T₉U^{OMe}-adduct is an oligonucleotide in which a universal linker is bonded to a T-9 mer-U^{OMe} oligonucleotide without being cleaved,
T₉T^{LNA} is T-9 mer-T^{LNA} oligonucleotide (SEQ ID NO: 3), and
mix-12 mer is a 12 mer oligonucleotide (SEQ ID NO: 4) constituted of plural kinds of nucleic acid monomers with a base sequence of GGATGTTCTCGT.
(*1) T₉T^{LNA}-adduct was not observed.

Oligonucleotides were cleaved from CPG3a-h, CPG3b^{OMe}, and CPG3b^{LNA} under the following conditions 1) to 3).
1) treatment with 28% aqueous ammonia at room temperature for 2 hr
2) treatment with 40% methylamine aqueous solution/28% aqueous ammonia=1:1 (AMA) solution at room temperature for 5 min
3) treatment with 50 mM methanol solution of potassium carbonate at room temperature for 4 hr

In addition, oligonucleotide was cleaved from CPG3e-mix under the above-mentioned conditions 1) and 3).

After treatment under the above-mentioned respective conditions, the cleavage results were analyzed by reversed-phase HPLC. The HPLC analysis conditions are shown in the following Table 1.

**[Table 1]**

| | |
|---|---|
| eluent | SOLUTION A: 0.1 M acetic acid triethylammonium buffer (pH 7.0) |
| | SOLUTION B: acetonitrile |
| gradient | SOLUTION B: 5-15% (30 min) |
| column | Waters Xbridge^{™} Shield RP18 2.5 mm (4.6×50 mm) |
| flow rate | 1.0 mL/min |
| column temperature | 40°C |
| detection | UV (254 nm) |

HPLC charts showing the cleavage results of oligonucleotides from CPG3a, CPG3e, CPG3g, CPG3b^{OMe}, and CPG3b^{LNA} are shown in Fig. 1.

HPLC charts showing the cleavage results of oligonucleotides from CPG3e-mix are shown in Fig. 2.

A table summarizing the production ratio of the oligonucleotide (T₁₀) of interest and the by-produced oligonucleotide (T₁₀-adduct) which are released upon cleavage of each CPG3a-h is also shown (Table 2). Here, the structure of the T₁₀-adduct was identified by comparison with the description in a literature (Tetrahedron, 2021, 92, 132261.)

Similarly, tables summarizing the production ratio of the oligonucleotides (T₉U^{OMe} and T₉T^{LNA}) of interest and the by-produced oligonucleotides (T₉U^{OMe}-adduct and T₉T^{LNA}-adduct) which are released upon cleavage of CPG3b^{OMe} and CPG3b^{LNA} are also shown (Table 3 and Table 4).

**[Table 2]**

| HPLC peak area ratio (T₁₀:T₁₀-adduct) | | | | |
|---|---|---|---|---|
| CPG3 | R | condition 1) | condition 2) | condition 3) |
| a | -OMe | 49:51 | 66:34 | 100:0 |
| b | -OE t | 80:20 | 90:10 | 100:0 |
| c | -O*i*Pr | 91:9 | 95:5 | 100:0 |
| d | -OCH₂CH (CH₃)₂ | 86:14 | 94:6 | 100:0 |
| e | -OCH₂C(C H₃)₃ | 91:9 | 97:3 | 100:0 |
| f | -OPh | 15:85 | 22:78 | 100:0 |
| 9 | -OCH₂CH₂ CN | 11:89 | 14:86 | 13:87 |
| h | -Me | 100:0* | 100:0* | 100:0* |

| | | | | |
|---|---|---|---|---|
| * when cleaved from CPG3h wherein R is -Me, about 6% in total of T₉ and T₉-adduct was by-produced. | | | | |

**[Table 3]**

| HPLC peak area ratio (T₉:T^{OMe}:T₉:T^{OMe}-adduct) | | | | |
|---|---|---|---|---|
| b^{OMe} | -OEt | 95:5 | 100:0 | 100:0 |

**[Table 4]**

| HPLC peak area ratio (T₉:T^{LNA}:T₉:T^{LNA}-adduct) | | | | |
|---|---|---|---|---|
| b^{LNA} | -OEt | 100:0 | 100:0 | 100:0 |

Furthermore, CPG3e and CPG3g were subjected to cleavage by 4) treatment with 28% aqueous ammonia at 80°C or 5) AMA solution at 55°C, and monitored over time by HPLC (HPLC gradient; SOLUTION B: 8-18% (30 min)).

HPLC charts showing the cleavage results of oligonucleotides from CPG3e and CPG3g are shown in Fig. 3 [cleavage under conditions 4)] and Fig. 4 [cleavage under conditions 5; same conditions as in the above-mentioned Table 1] .

Tables summarizing the production ratio of the oligonucleotide (T₁₀) of interest and the by-produced oligonucleotide (T₁₀-adduct) (T₁₀:T₁₀-adduct) which are released under conditions 4) and conditions 5) are shown in Table 5 and Table 6.

**[Table 5]**

| Time | CPG3 e | CPG3 g |
|---|---|---|
| 1 h | 91:9 | 26:74 |
| 3 h | 97:3 | 60:40 |

**[Table 6]**

| Time | CPG3e | CPG3g |
|---|---|---|
| 4 h | 99:1 | 59:41 |

In oligonucleotide synthesis, phosphoramidites (compound 3g) having a cyanoethoxy group as a protecting group for the phosphoric acid moiety have been widely used. However, when synthesizing oligonucleotides by using a universal support conventionally used widely, the use of the nucleotide derivatives (I) of the present invention (corresponding to nucleotide derivatives 3a-3f, 3h), having a more stable substituent such as an alkoxy group as a protecting group for the phosphoric acid moiety, as a phosphoramidite strikingly increased the production of T10 oligonucleotides, and it was confirmed that the oligonucleotides can be released from the solid phase support under mild base conditions. This superior effect was also confirmed when OMe or LNA having modified sugar moieties was used.

### Example D2: Cleavage from CPG1a and CPG1g/CPG2a and CPG2g

The outline of the cleavage of oligonucleotide is shown in the following.

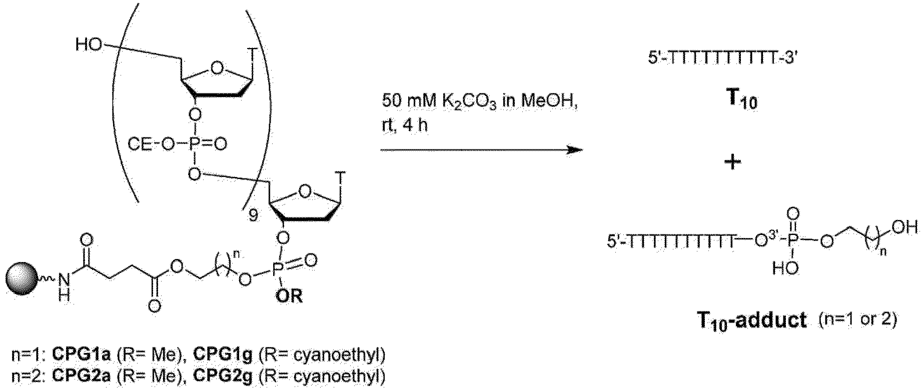

In the above-mentioned formulas, CE, T, T₁₀, and T₁₀-adduct are each as defined above.

Cleavage of oligonucleotides from CPG1a and CPG1g/CPG2a and CPG2g was performed by a treatment with a methanol solution of 50 mM potassium carbonate at room temperature for 4 hr. The results thereof were analyzed by reversed-phase HPLC. HPLC analysis conditions are shown in the following Table 7.

**[Table 7]**

| | |
|---|---|
| eluent | SOLUTION A: 0.1 M acetic acid triethylammonium buffer (pH 7.0) |
| | SOLUTION B: acetonitrile |
| gradient | SOLUTION B: 5-15% (30 min) |
| column | Waters Xbridge^{™} Shield RP18 2.5 mm (4.6×50 mm) |
| flow rate | 1.0 mL/min |
| column temperature | 40°C |
| detection | UV (254 nm) |

HPLC charts showing the cleavage results of oligonucleotides from CPG1a and CPG1g/CPG2a and CPG2g are shown in Fig. 5.

The production ratio of the oligonucleotide (T₁₀) (SEQ ID NO: 1) of interest and the by-produced oligonucleotide (T₁₀-adduct) which are released upon cleavage of each CPG is shown in Table 8 as values calculated from peak area ratio on HPLC chart.

**[Table 8]**

| CPG | area ratio (T₁₀:T₁₀-adduct) |
|---|---|
| 1 a | 90:10 |
| 1 g | 0:100 |
| 2 a | 5:20:75* |
| 2 g | 0:100 |

In the Table, * is a Triester form represented by the following formula.

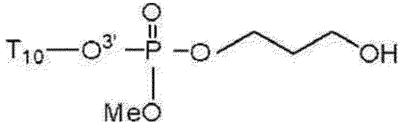

T₁₀-adduct (n=1 or 2) and Triester form as by products were each identified by ESI-MS analysis. The results thereof are shown in the following Table 9.

**[Table 9]**

| | molecular formula | theoretical value | measured value |
|---|---|---|---|
| T₁₀-adduct (n=1) | C₁₀₂H₁₃₆N₂₀O₇₂P₁₀ | 3104.0156 | 3104.3000 |
| T₁₀-adduct (n=2) | C₁₀₃H₁₃₈N₂₀O₇₂P₁₀ | 3118.5002 | 3118.0426 |
| Triester | C₁₀₄H₁₄₀N₂₀O₇₂P₁₀ | 3132.6001 | 3132.0696 |

It was clarified that, by using the nucleotide derivative (I) of the present invention that is a phosphoramidite having stabler substituents, oligonucleotides can be cleaved sufficiently rapidly even when an ethyleneglycol resin (CPG1) with a "1,2-diol structure" which is the same as a universal support with a simpler structure than the universal support is used as a solid phase support.

In this way, one of the features of the production method of oligonucleotides of the present invention is that a 1,2-diol derivative having a simple structure can be used as a universal linker.

### E. Preparation 2 of nucleotide derivative

In each formula below, DMTr is a 4,4'-dimethoxytrityl group, Me is a methyl group, iPr is an isopropyl group, tBu is a t-butyl group, and Ac is an acetyl group.

### Production Example E1: Synthesis of compound 10

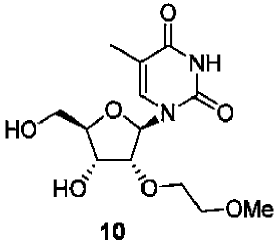

According to the procedures in a known literature (Nucleosides, Nucleotides, and Nucleic Acids, 2005, 24, 815-818.), compound 10 was synthesized.

### Production Example E2: Synthesis of compound 11

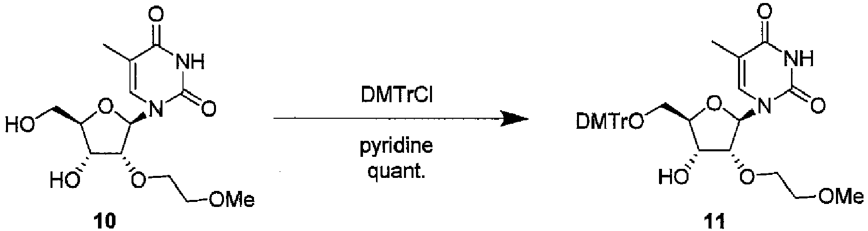

Compound 10 (10.0 g, 31.6 mmol) was subjected 3 times to azeotropic distillation with dehydration with pyridine. The Compound was successively dissolved in pyridine (80 mL), 4,4'-dimethoxytritylchloride (13.9 g, 41.1 mmol) was added and the mixture was stirred for 1.5 hr. After completion of the reaction, the reaction was quenched with saturated aqueous sodium hydrogen carbonate and the mixture was extracted with ethyl acetate. The organic layer was dried over magnesium sulfate and the solvent was evaporated under reduced pressure. The obtained residue was purified by silica gel column chromatography (ethyl acetate/n-hexane=3/7→7/3) to give compound 11 (19.6 g, quant.).
¹H-NMR(CDCl₃, 400 MHz); δ7.94(1H,brs), 7.65(1H,d,J=0.8Hz), 7.42-7.39(2H,m), 7.31-7.24(7H,m), 6.85-6.82(4H,m), 6.01(1H,d,J=4.0Hz), 4.43(1H,dd,J=10.8,5.6Hz), 4.15-4.11(2H,m), 4.08-4.03(1H,m), 3.79(6H,s), 3.78-3.73(1H,m), 3.65-3.60(1H,m), 3.56-3.51(3H,m), 3.42-3.39(1H,m), 3.39(3H,s), 1.37(3H,s). LRMS(ESI-QTOF):m/z 641.2360[M+Na]⁺

### Example E1: Synthesis of nucleotide derivative 12c

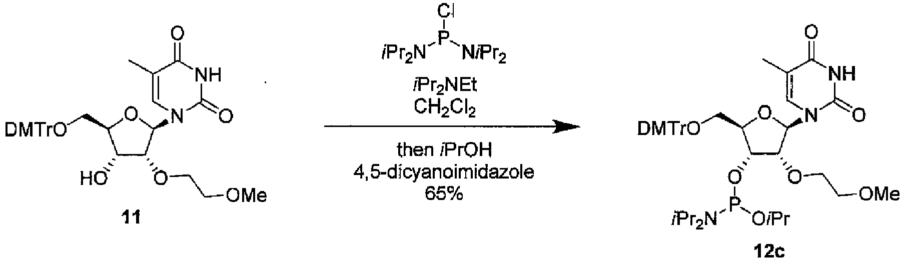

Compound 11 (4.00 g, 7.50 mmol) was dissolved in dichloromethane (33.0 mL), N,N-diisopropyl ethylamine (3.40 mL, 19.0 mmol) and bis(diisopropylamino)chlorophosphine (2.60 g, 9.70 mmol) were successively added, and the mixture was stirred for 2 hr. Then, 2-propanol (1.50 mL, 19.0 mmol) and 4,5-dicyanoimidazole (150 mg, 1.30 mmol) were successively added, and the mixture was stirred for 22 hr. After completion of the reaction, the reaction was quenched with saturated aqueous sodium hydrogen carbonate and the mixture was extracted with ethyl acetate. The organic layer was dried over magnesium sulfate and the solvent was evaporated under reduced pressure, and the residue was purified by silica gel column chromatography (n-hexane→ethyl acetate/n-hexane=1/1) to give nucleotide derivative 12c (3.37 g, 65%).
¹H-NMR(CD₃CN, 400 MHz); δ9.13(1H,brs), 7.53-7.44(3H,m), 7.36-7.23(7H,m), 6.89-6.86(4H,m), 5.92-5.90(1H,m), 4.46-4.38(1H,m), 4.21-3.91(3H,m), 3.84-3.72(8H,m), 3.64-3.49(4H,m), 3.42-
3.27(5H,m), 1.39-1.35(3H,m), 1.22-1.13(12H,m), 1.09-1.03(6H,m). ³¹P-NMR(CD₃CN, 162 MHz); δ147.6,147.1.
LRMS(ESI-QTOF):m/z 808.4109[M+H]⁺

### Example E2: Synthesis of nucleotide derivative 12e

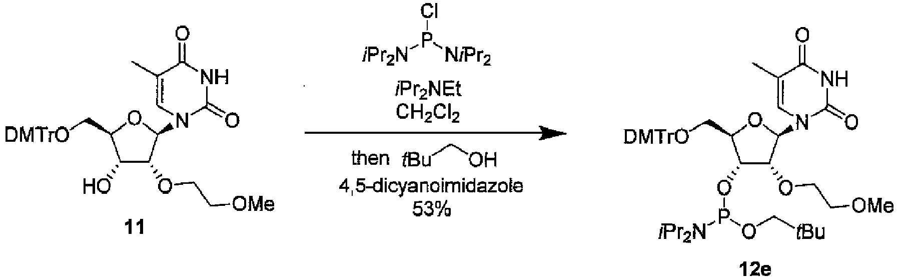

Compound 11 (8.00 g, 13.0 mmol) was dissolved in dichloromethane (65.0 mL), N,N-diisopropyl ethylamine (6.80 mL, 39.0 mmol) and bis(diisopropylamino)chlorophosphine (5.20 g, 19.0 mmol) were successively added, and the mixture was stirred for 2 hr. Then, neopentyl alcohol (5.70 g, 65.0 mmol) and 4,5-dicyanoimidazole (310 mg, 2.60 mmol) were successively added, and the mixture was stirred for 6 hr. After completion of the reaction, the reaction was quenched with saturated aqueous sodium hydrogen carbonate and the mixture was extracted with ethyl acetate. The organic layer was dried over magnesium sulfate and the solvent was evaporated under reduced pressure, and the residue was purified by silica gel column chromatography (ethyl acetate/n-hexane=3/7→1/1) to give nucleotide derivative 12e (5.69 g, 53%).
¹H-NMR(CD₃CN, 400 MHz); δ9.11(1H,brs), 7.53-7.44(3H,m), 7.35-7.23(7H,m), 6.88-6.86(4H,m), 5.93-5.90(1H,m), 4.50-4.42(1H,m), 4.22-4.13(2H,m), 3.86-3.72(8H,m), 3.65-3.24(10H,m), 3.20-3.06(1H,m), 1.38-1.35(3H,m), 1.24-1.14(9H,m), 1.04-1.03(3H,m), 0.90-0.80(9H,m).
³¹P-NMR(CD₃CN, 162 MHz); δ149.8,149.5.
LRMS(ESI-QTOF):m/z 836.4615[M+H]⁺

### Production Example E3: Synthesis of compound 13

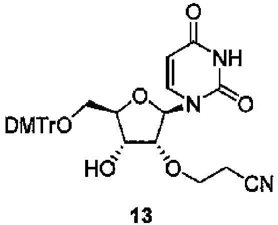

According to the procedures in a known literature (J. Org. Chem. 2005, 70, 10453-10460.), compound 13 was synthesized.

### Example E3: Synthesis of nucleotide derivative 14g

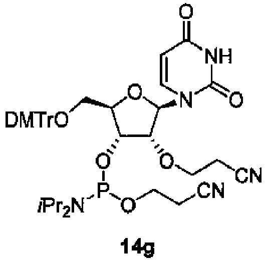

According to the procedures in a known literature (J. Org. Chem. 2005, 70, 10453-10460.), nucleotide derivative 14g was synthesized.

### Example E4: Synthesis of nucleotide derivative 14c

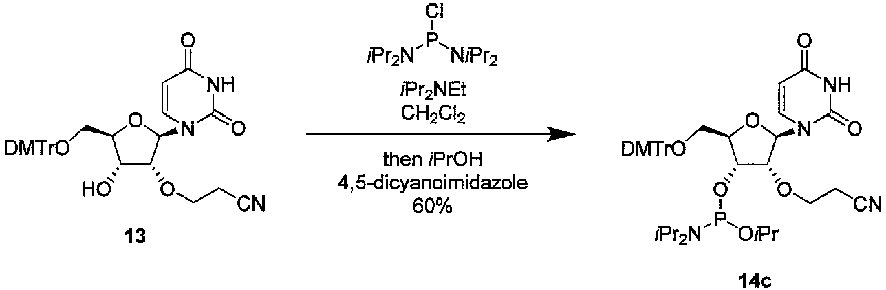

Compound 13 (4.5 g, 7.50 mmol) was dissolved in dichloromethane (37.5 mL), N,N-diisopropyl ethylamine (3.92 mL, 22.5 mmol) and bis(diisopropylamino)chlorophosphine (3.0 g, 11.3 mmol) were successively added, and the mixture was stirred for 3 hr. Then, 2-propanol (1.72 mL, 22.5 mmol) and 4,5-dicyanoimidazole (177 mg, 1.50 mmol) were successively added, and the mixture was stirred for 21 hr. After completion of the reaction, the reaction was quenched with saturated aqueous sodium hydrogen carbonate and the mixture was extracted with ethyl acetate. The organic layer was dried over magnesium sulfate and the solvent was evaporated under reduced pressure, and the residue was purified by silica gel column chromatography (ethyl acetate/n-hexane=1/1) to give nucleotide derivative 14c (3.57 g, 60%).
¹H-NMR(CD₃CN, 400 MHz); δ9.01(1H,brs), 7.86-7.75(1H,m), 7.48-7.43(2H,m), 7.36-7.24(7H,m), 6.89-6.86(4H,m), 5.84-5.83(1H,m), 5.22-5.17(1H,m), 4.47-4.32(1H,m), 4.17-3.86(5H,m), 3.77(6H,m), 3.67-3.54(2H,m), 3.49-3.36(2H,m), 2.74-2.60(2H,m), 1.23-1.10(15H,m), 1.06-1.05(3H,m)
³¹P-NMR(CD₃CN, 162 MHz); δ147.9,146.9.
LRMS(ESI-QTOF):m/z 789.3420[M+H]⁺

### Production Example E4: Synthesis of compound 15

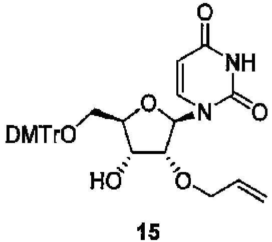

According to the procedures in a known literature (Nucleosides and Nucleotides, 1992, 11, 1263-1273.), compound 15 was synthesized.

### Example E5: Synthesis of nucleotide derivative 16g

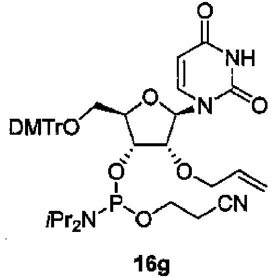

According to the procedures in a known literature (Nucleosides and Nucleotides, 1992, 11, 1263-1273.), nucleotide derivative 16g was synthesized.

### Example E6: Synthesis of nucleotide derivative 16c

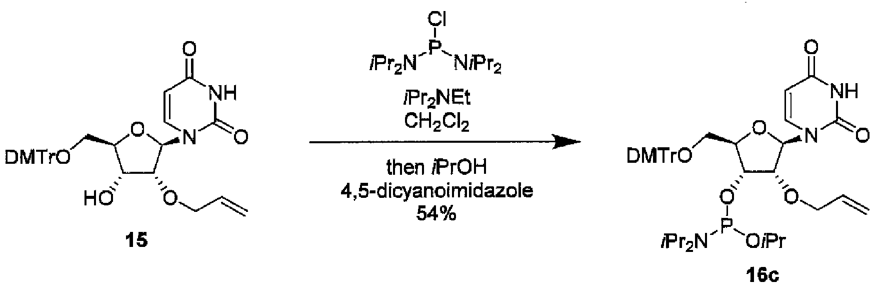

Compound 15 (4.5 g, 7.67 mmol) was dissolved in dichloromethane (38.4 mL), N,N-diisopropylethylamine (4.01 mL, 23.0 mmol) and bis(diisopropylamino)chlorophosphine (3.07 g, 11.5 mmol) were successively added, and the mixture was stirred for 2 hr. Then, 2-propanol (1.76 mL, 23.0 mmol) and 4,5-dicyanoimidazole (181 mg, 1.53 mmol) were successively added, and the mixture was stirred for 21 hr. After completion of the reaction, saturated aqueous sodium hydrogen carbonate the reaction was quenched with and the mixture was extracted with ethyl acetate. The organic layer was dried over magnesium sulfate and the solvent was evaporated under reduced pressure, and the residue was purified by silica gel column chromatography (ethyl acetate/n-hexane=1/4) to give nucleotide derivative 16c (3.23 g, 54%).
¹H-NMR(CD₃CN, 400 MHz); δ9.03(1H,brs), 7.84-7.73(1H,m), 7.47-7.42(2H,m), 7.36-7.23(7H,m), 6.90-6.85(4H,m), 5.99-5.87(2H,m), 5.33-5.27(1H,m), 5.24-5.14(2H,m), 4.47-4.35(1H,m), 4.28-3.94(5H,m), 3.77(6H,m), 3.65-3.53(2H,m), 3.44-3.34(2H,m), 1.22-1.13(12H,m), 1.11-1.09(3H,m), 1.06-1.04(3H,m).
³¹P-NMR(CD₃CN, 162 MHz); δ147.5,147.2.
LRMS(ESI-QTOF):m/z 776.3499[M+H]⁺

### Production Example E5: Synthesis of compound 17

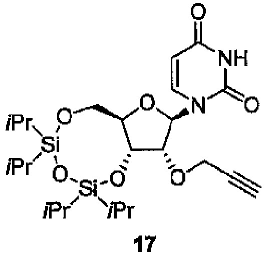

According to the procedures in a known literature (J. Med. Chem. 2008, 51, 4957-4967.), compound 17 was synthesized.

### Production Example E6: Synthesis of compound 19

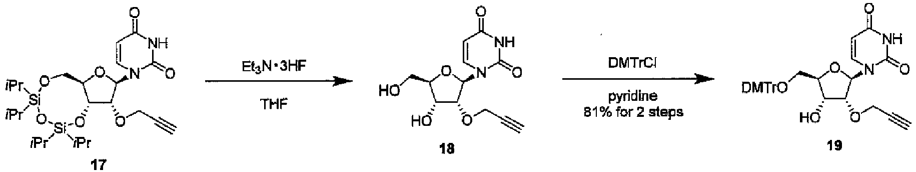

(1) Compound 17 (14.1 g, 27.0 mmol) was dissolved in tetrahydrofuran (135 mL), triethylamine 3 hydrofluoric acid salt (6.59 mL, 40.5 mmol) was added, and the mixture was stirred for 3.5 hr. After completion of the reaction, the reaction was quenched with saturated aqueous sodium hydrogen carbonate, sodium chloride (10 g) was added, and the mixture was extracted with tetrahydrofuran. Then, the organic layer was dried over magnesium sulfate and the solvent was evaporated under reduced pressure to give crudely purified compound 18 (7.62 g, <27.0 mmol).
(2) The crudely purified compound 18 (7.62 g, <27.0 mmol) was azeotropically distilled three times with pyridine. The obtained residue was dissolved in pyridine (90 mL), 4,4'-dimethoxytritylchloride (11.0 g, 32.4 mmol) was added and the mixture was stirred for 15 hr. After completion of the reaction, the reaction was quenched with saturated aqueous sodium hydrogen carbonate and the mixture was extracted with ethyl acetate. The organic layer was dried over magnesium sulfate and the solvent was evaporated under reduced pressure, and the mixture was azeotropically distilled three times with toluene. The residue was purified by silica gel column chromatography (ethyl acetate/n-hexane=1/1) to give compound 19 (12.8 g, 81%).
   ¹H-NMR(CDCl₃, 400 MHz); δ8.00-7.98(2H,m), 7.41-7.37(2H,m), 7.32-7.25(7H,m), 6.87-6.83(4H,m), 5.97(1H,d,J=2.4Hz), 5.28(1H,dd,J=8.0,2.4Hz), 4.55-4.44(3H,m),
   4.24(1H,dd,J=4.0,2.8Hz), 4.06(1H,dt,J=6.8,2.0Hz), 3.80(6H,s), 3.57-3.50(2H,m), 2.53(1H,d,J=8.0Hz), 2.51(1H,t,J=2.4). LRMS(ESI-QTOF):m/z 607.1871[M+Na]⁺

### Example E7: Synthesis of nucleotide derivative 20g

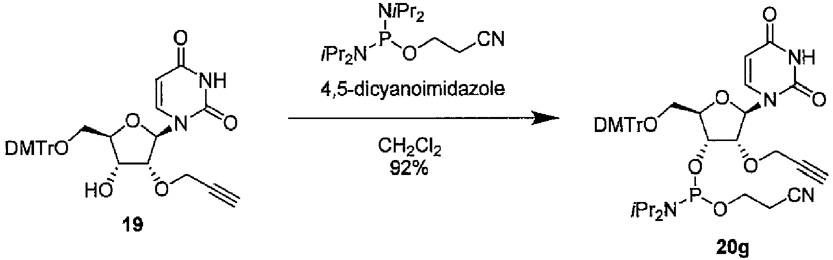

Compound 19 (3.50 g, 5.99 mmol) was dissolved in dichloromethane (30 mL), 2-cyanoethyl-N,N,N',N'-tetraisopropylphosphorodiamidite (2.80 mL, 8.98 mmol) and 4,5-dicyanoimidazole (70.7 mg, 0.599 mmol) were successively added, and the mixture was stirred for 15.5 hr. After completion of the reaction, the reaction was quenched with saturated aqueous sodium hydrogen carbonate. The organic layer was separated, and the aqueous layer was extracted with ethyl acetate. Then, the organic layer was dried over magnesium sulfate and the solvent was evaporated under reduced pressure, and the residue was purified by silica gel column chromatography (ethyl acetate/n-hexane=7/10→ethyl acetate) to give compound 20g (4.33 g, 92%).
¹H-NMR (400 MHz,CD₃CN)δ9.07(1H,brs), 7.79-7.69(1H,m), 7.44-7.39(m,2H), 7.33-7.20(7H,m), 6.80-6.93(4H,m), 5.86-5.84(1H,m), 5.24-5.22(1H,m), 4.51-4.41(1H,m), 4.37-4.32(2H,m), 4.28-4.23(1H,m), 4.17-4.10(1H,m), 4.06-4.00(1H,m), 3.92-3.53(8H,m), 3.43-3.31(2H,m), 2.72-2.63(2H,m), 2.50-2.47(1H,m), 1.92-1.89(1H,m), 0.97-1.27(12H,m).
³¹P-NMR (162 MHz, CD₃CN)δ150.7,150.3.
LRMS(ESI-QTOF):m/z 807.3075[M+Na]⁺

### Example E8: Synthesis of nucleotide derivative 20c

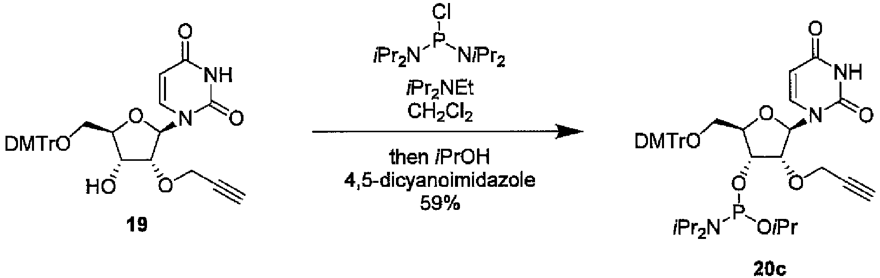

Compound 19 (4.50 g, 7.70 mmol) was dissolved in dichloromethane (39 mL), N,N-diisopropyl ethylamine (4.02 mL, 23.1 mmol) and bis(diisopropylamino)chlorophosphine (3.08 g, 11.5 mmol) were successively added, and the mixture was stirred for 2 hr. Then, 2-propanol (3.53 mL, 46.2 mmol) and 4,5-dicyanoimidazole (182 mg, 1.54 mmol) were successively added, and the mixture was stirred for 5 hr. After completion of the reaction, the reaction was quenched with saturated aqueous sodium hydrogen carbonate and the mixture was extracted with ethyl acetate. Then, the organic layer was dried over magnesium sulfate and the solvent was evaporated under reduced pressure, and the residue was purified by silica gel column chromatography (ethyl acetate/n-hexane=9/13) to give nucleotide derivative 20c (3.53 g, 59%).
¹H-NMR (400 MHz,CD₃CN)δ9.05(brs,1H), 7.79-7.69(1H,m), 7.48-7.40(2H,m), 7.32-7.20(7H,m), 6.86-6.82(4H,m), 5.86(1H,m), 5.26-5.22(1H,m), 4.44-4.32(2H,m), 4.24-4.18(1H, m), 4.15-3.88(2H,m), 3.73(6H,s), 3.64-3.51(2H,m), 3.41-3.29(2H,m), 2.70-2.68(1H,m), 1.92-1.89(1H,m), 1.19-1.01(18H,m).
³¹P-NMR (162 MHz, CD₃CN)δ147.7,147.6.
LRMS(ESI-QTOF):m/z 774.3848[M+H]⁺

### Production Example E7: Synthesis of compound 21

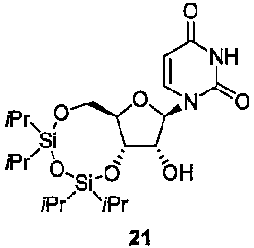

According to the procedures in a known literature (Tetrahedron. 2021, 72, 153066.), compound 21 was synthesized.

### Production Example E8: Synthesis of compound 22

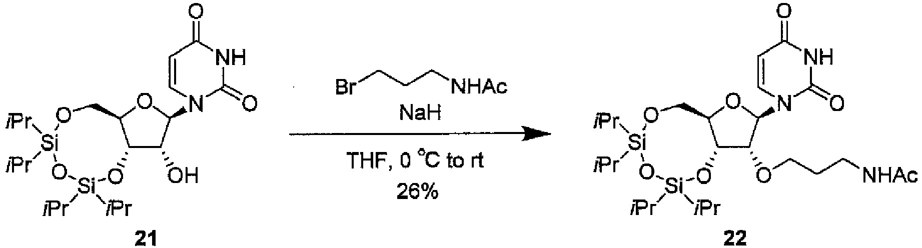

Compound 21 (10.6 g, 21.7 mmol) was dissolved in tetrahydrofuran (80 mL), under ice-cooling, sodium hydride (60 wt%, oily) (2.60 g, 65.0 mmol) was added over 10 min, and the mixture was stirred for 15 min. Then, N-(3-bromopropyl)acetamide (5.85 g, 32.5 mmol) was dissolved in 6 mL of tetrahydrofuran. The mixture was stirred at room temperature for 15 hr. After completion of the reaction, the reaction was quenched with saturated ammonium chloride aqueous solution, the organic layer was separated, and the aqueous layer was extracted with ethyl acetate. Then, the organic layer was dried over magnesium sulfate, the solvent was evaporated under reduced pressure, and the residue was purified by silica gel column chromatography (ethyl acetate/n-hexane=1/4 →ethyl acetate→methanol/chloroform=1/19) to give compound 22 (3.25 g, 26%).
¹H-NMR (400 MHz,DMSO-D6)δ7.96(1H,brs), 7.76(1H,t,J=5.3Hz), 7.65(1H,d,J=8.2Hz), 5.58(1H,s), 5.53(1H,d,J=8.2Hz), 4.23(1H,dd,J=8.0,4.4Hz), 4.13(1H,d,J=12.3Hz),
4.03(1H,d,J=4.6Hz), 3.99-3.90(2H,m), 3.78-3.65(2H,m), 3.14-3.07(2H,m), 1.77(3H,s), 1.69-1.63(2H,m), 1.06-0.98 (28H,m). LRMS(ESI-QTOF) :m/z 608.3365[M+Na]⁺

### Production Example E9: Synthesis of compound 23

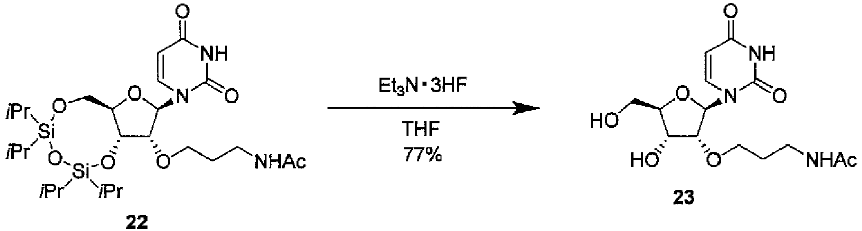

Compound 22 (3.25 g, 5.55 mmol) was dissolved in tetrahydrofuran (28 mL), triethylamine 3 hydrofluoric acid salt (1.36 mL, 8.32 mmol) was added, and the mixture was stirred for 2.5 hr. After completion of the reaction, under ice-cooling, potassium carbonate (1.53 g, 11.1 mmol) and silica gel (6.0 g) were successively added, and tetrahydrofuran was evaporated. Purification by silica gel column chromatography (methanol/chloroform=3/22→1/4) gave compound 23 (1.46 g, 77%). ¹H-NMR (400 MHz, DMSO-D6)δ11.32(1H,brs), 7.93(1H,d,J=8.2Hz), 7.77(1H,t,J=5.7Hz), 5.83(1H,d,J=5.0Hz), 5.64(1H,d,J=7.8Hz), 5.15-5.13(2H,m), 4.09(1H,qd,J=5.2,1.8Hz), 3.88-3.84(2H,m), 3.65(1H,dq,J=12.3,2.7Hz), 3.58-3.47(2H,m), 3.17(1H,d,J=5.0Hz), 3.12-3.02(2H,m), 1.78(3H,s), 1.65-1.58(2H,m).
LRMS(ESI-QTOF):m/z 366.1188[M+Na]⁺

### Production Example E10: Synthesis of compound 24

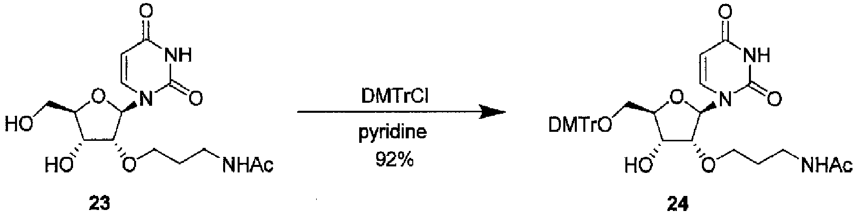

Compound 23 (1.46 g, 4.25 mmol) was azeotropically distilled three times with pyridine. The obtained residue was dissolved in pyridine (14 mL), 4,4'-dimethoxytritylchloride (1.73 g, 5.10 mmol) was added, and the mixture was stirred for 15 hr. After completion of the reaction, the reaction was quenched with saturated aqueous sodium hydrogen carbonate, and the mixture was extracted with ethyl acetate. The organic layer was dried over magnesium sulfate and the solvent was evaporated under reduced pressure, and the mixture was azeotropically distilled twice with toluene. The residue was purified by silica gel column chromatography
(methanol/chloroform=1/49→1/19) to give compound 24 (2.53 g, 92%) .
¹H-NMR (400 MHz, DMSO-D6)δ11.38(1H,brs), 7.79(1H,t,J=5.3Hz), 7.72(1H,d,J=8.2Hz), 7.39-7.23(9H,m), 6.90(4H,d,J=8.5Hz), 5.79(1H,d,J=3.7Hz), 5.28(1H,d,J=8.2Hz), 5.20(1H,d,J=6.4Hz), 4.21-4.16(1H,m), 3.98-3.94(1H,m), 3.90(1H,t,J=4.1Hz),
3.74(6H,s), 3.59(2H,t,J=6.2Hz), 3.25-3.20(1H,m), 3.15-3.05(2H,m), 1.78(3H,s), 1.67-1.61(2H,m).
LRMS(ESI-QTOF):m/z 668.2421[M+Na]⁺

### Example E9: Synthesis of nucleotide derivative 25g

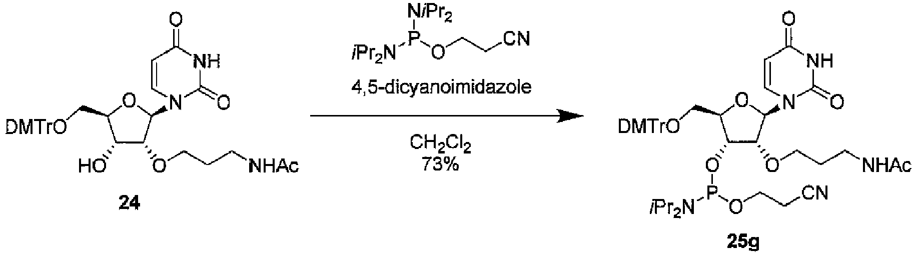

Compound 24 (1.10 g, 1.70 mmol) was dissolved in dichloromethane (8.5 mL), 2-cyanoethyl-N,N,N',N'-tetraisopropylphosphorodiamidite (0.810 mL, 2.56 mmol) and 4,5-dicyanoimidazole (20.1 mg, 0.170 mmol) were successively added, and the mixture was stirred for 18 hr. After completion of the reaction, the reaction was quenched with saturated aqueous sodium hydrogen carbonate. The organic layer was separated, and the aqueous layer was extracted with ethyl acetate. The organic layer was dried over magnesium sulfate and the solvent was evaporated under reduced pressure. The residue was purified by silica gel column chromatography (methanol/(1% triethylamine-containing ethyl acetate)=1/99→1/19) to give nucleotide derivative 25g (1.05 g, 73%).
¹H-NMR (400 MHz,CD₃CN)δ9.09(1H,brs), 7.82-7.70(1H,m), 7.41(2H,m), 7.33-7.20(7H,m), 6.85(4H,m), 6.44(1H,brs), 5.84-5.82(1H,m), 5.21-5.17(1H,m), 4.50-4.36(1H,m), 4.15-4.09(1H,m), 4.05-3.97(2H,m), 3.87-3.52(11H,m), 3.44-3.32(2H,m), 3.21-3.14(2H,m), 2.67-2.45(2H,m), 1.80-1.79(3H,m), 1.61-1.75(2H,m), 0.99-1.23(12H,m).
³¹P-NMR (162 MHz, CD₃CN)δ150.2,149.8.
LRMS(ESI-QTOF):m/z 868.3463[M+Na]⁺

### Example E10: Synthesis of nucleotide derivative 25c

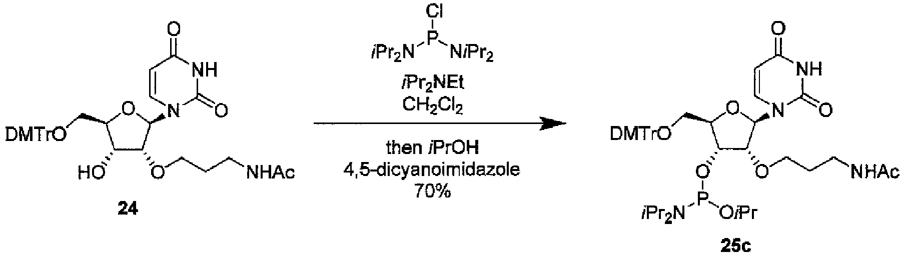

Compound 24 (2.50 g, 3.87 mmol) was dissolved in dichloromethane (19 mL), N,N-diisopropyl ethylamine (2.02 mL, 11.6 mmol) and bis(diisopropylamino)chlorophosphine (1.55 g, 5.81 mmol) were successively added, and the mixture was stirred for 2 hr. Then, 2-propanol (1.78 mL, 23.2 mmol) and 4,5-dicyanoimidazole (91.4 mg, 0.774 mmol) were successively added, and the mixture was stirred for 6 hr. After completion of the reaction, the reaction was quenched with saturated aqueous sodium hydrogen carbonate. The organic layer was separated, and the aqueous layer was extracted with ethyl acetate. Then, the organic layer was dried over magnesium sulfate and the solvent was evaporated under reduced pressure. The residue was purified by silica gel column chromatography (methanol/(1% triethylamine-containing ethyl acetate)=1/99→1/19) to give nucleotide derivative 25c (2.27 g, 70%).
¹H-NMR (400 MHz,CD₃CN)δ9.03(1H,brs), 7.82-7.69(1H,m), 7.48-7.39(2H,m), 7.32-7.21(7H,m), 6.89-6.83(4H,m), 6.45(1H,brs), 5.84-5.82(1H,m), 5.23-5.16(1H,m), 4.43-4.27(1H,m), 4.13-3.89(3H,m), 3.77-3.47(10H,m), 3.44-3.28(2H,m), 3.25-3.07(2H,m), 1.80-1.79(3H,m), 1.73-1.63(2H,m), 1.23-1.01(18H,m).
³¹P-NMR (162 MHz, CD₃CN)δ147.3,147.1.
LRMS(ESI-QTOF):m/z 835.3854[M+H]⁺

### F. Synthesis 2 of oligonucleotide

Oligonucleotide was synthesized using Oligpilot 100 (Cytiva) and according to the general phosphoramidite method. As a support, the following Primer Support 5G Unylinker 350(PS1) was used and the synthesis scale was set to 50 pmol, and the synthesis was performed under trityl OFF conditions.

In each formula below, DMTr is a 4,4'-dimethoxytrityl group, Ph is a phenyl group, Me is a methyl group, iPr is an isopropyl group, Ac is an acetyl group, and CE is a 2-cyanoethyl group.

### PSI:Primer Support 5G Unylinker 350

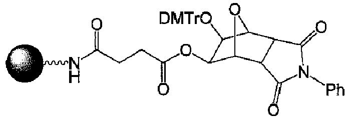

Commercially available phosphoramidite of T (same as compound 3g) and commercially available MOE-T amidite (nucleotide derivative 12g shown by the following formula, manufactured by Hongene) were prepared as 0.15 mol/L solutions in anhydrous acetonitrile and used.

As the activator, 5-benzylthio-1H-tetrazole was used and the condensation time was 3 min for coupling of natural thymidine amidite and 12 min for coupling of 2'-modified nucleoside amidite.

The outline of the synthesis scheme and the structures of the synthesized oligonucleotides are shown in the following.

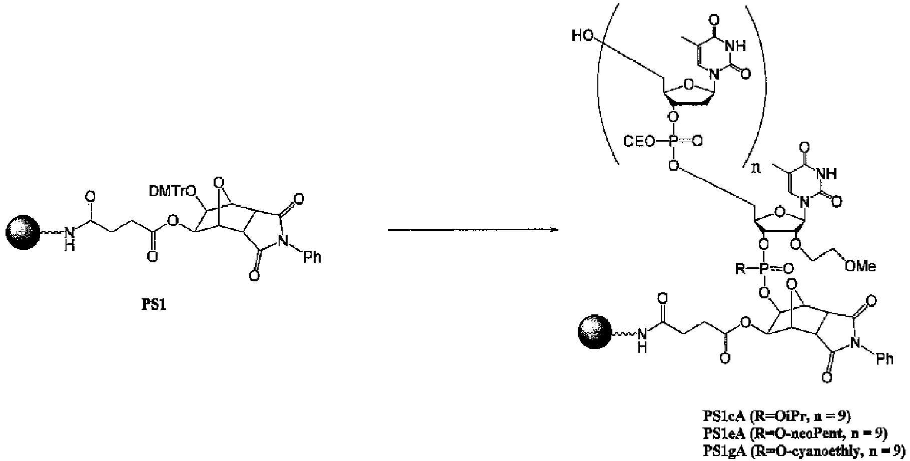

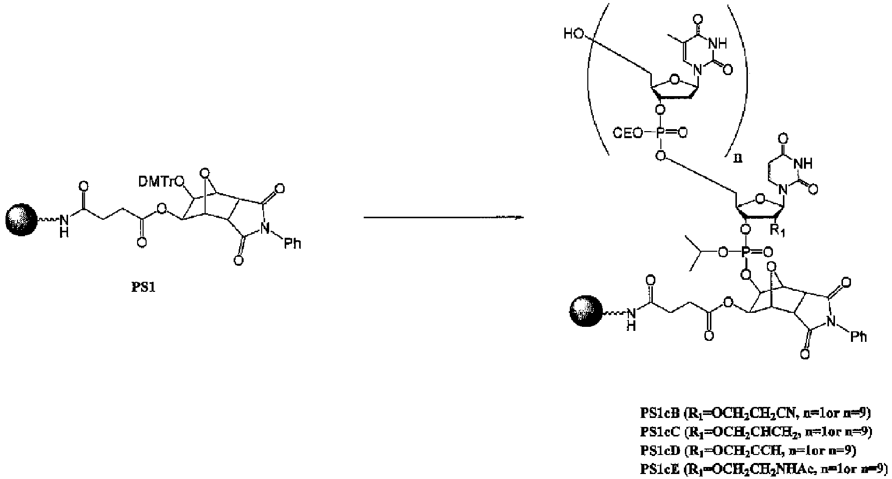

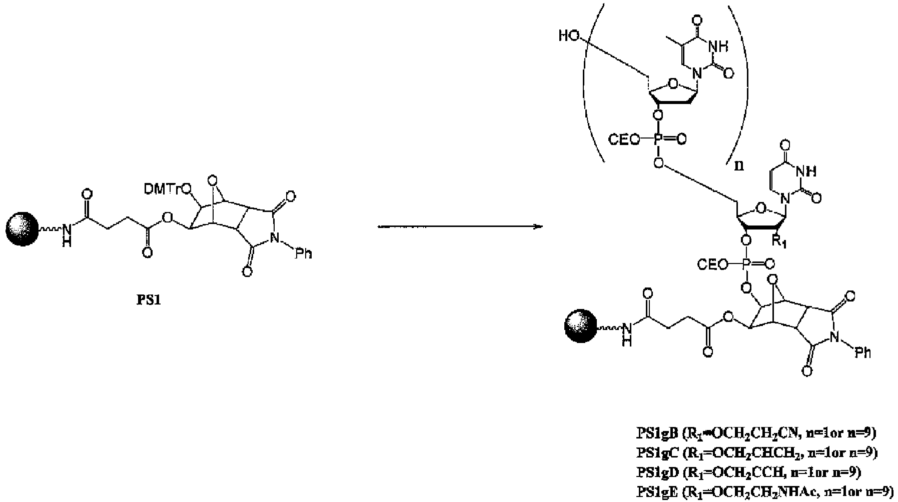

### Example F1: Synthesis of PS1cA (n=9), PS1eA (n=9), PS1gA (n=9)

Using commercially available PS1 (Primer Support 5G Unylinker 350 manufactured by Cytiva) as a solid phase support and a DNA automatic synthesizer, T-9 mer-(MOE)T oligonucleotides (PS1cA (n=9), PS1eA (n=9), PS1gA (n=9)) into which the aforementioned nucleotide derivatives 12c, 12e, 12g were introduced at the 3'-terminus were synthesized.

### Example F2: Synthesis of PS1cB (n=1), PS1gB (n=1)

Using commercially available PS1 (Primer Support 5G Unylinker 350 manufactured by Cytiva) as a solid phase support and a DNA automatic synthesizer, T-1 mer-(2'-OCE)U oligonucleotides (PS1cB (n=1), PS1gB (n=1)) into which the aforementioned nucleotide derivatives 14c, 14g were introduced at the 3'-terminus were synthesized.

### Example F3: Synthesis of PS1cB (n=9), PS1gB (n=9)

Using commercially available PS1 (Primer Support 5G Unylinker 350 manufactured by Cytiva) as a solid phase support and a DNA automatic synthesizer, T-9 mer-(2'-OCE)U oligonucleotides (PS1cB (n=9), PS1gB (n=9)) into which the aforementioned nucleotide derivatives 14c, 14g were introduced at the 3'-terminus were synthesized.

### Example F4: Synthesis of PS1cC (n=1), PS1gC (n=1)

Using commercially available PS1 (Primer Support 5G Unylinker 350 manufactured by Cytiva) as a solid phase support and a DNA automatic synthesizer, T-1 mer-(2'-O-allyl)U oligonucleotides (PS1cC (n=1), PS1gC (n=1)) into which the aforementioned nucleotide derivatives 16c, 16g were introduced at the 3'-terminus were synthesized.

### Example F5: Synthesis of PS1cC (n=9), PS1gC (n=9)

Using commercially available PS1 (Primer Support 5G Unylinker 350 manufactured by Cytiva) as a solid phase support and a DNA automatic synthesizer, T-9 mer-(2'-O-allyl)U oligonucleotides (PS1cC (n=9), PS1gC (n=9)) into which the aforementioned nucleotide derivatives 16c, 16g were introduced at the 3'-terminus were synthesized.

### Example F6: Synthesis of PS1cD (n=1), PS1gD (n=1)

Using commercially available PS1 (Primer Support 5G Unylinker 350 manufactured by Cytiva) as a solid phase support and a DNA automatic synthesizer, T-1 mer-(2'-O-propargyl)U oligonucleotides (PS1cD (n=1), PS1gD (n=1)) into which the aforementioned nucleotide derivatives 20c, 20g were introduced at the 3'-terminus were synthesized.

### Example F7: Synthesis of PS1cD (n=9), PS1gD (n=9)

Using commercially available PS1 (Primer Support 5G Unylinker 350 manufactured by Cytiva) as a solid phase support and a DNA automatic synthesizer, T-9 mer-(2'-O-propargyl)U oligonucleotides (PS1cD (n=9), PS1gD (n=9)) into which the aforementioned nucleotide derivatives 20c, 20g were introduced at the 3'-terminus were synthesized.

### Example F8: Synthesis of PS1cE (n=1), PS1gE (n=1)

Using commercially available PS1 (Primer Support 5G Unylinker 350 manufactured by Cytiva) as a solid phase support and a DNA automatic synthesizer, T-1 mer-(2'-O-(3-acetamide)propyl)U oligonucleotides (PS1cE (n=1), PS1gE (n=1)) into which the aforementioned nucleotide derivatives 25c, 25g were introduced at the 3'-terminus were synthesized.

### Example F9: Synthesis of PS1cE (n=9), PS1gE (n=9)

Using commercially available PS1 (Primer Support 5G Unylinker 350 manufactured by Cytiva) as a solid phase support and a DNA automatic synthesizer, T-9 mer-(2'-O-(3-acetamide)propyl)U oligonucleotides (PS1cE (n=9), PS1gE (n=9)) into which the aforementioned nucleotide derivatives 25c, 25g were introduced at the 3'-terminus were synthesized.

### G. Evaluation of oligonucleotide cleavage efficiency from solid phase (PS)

In each formula below, Ph is a phenyl group, Me is a methyl group, iPr is an isopropyl group, Ac is an acetyl group, and CE is a 2-cyanoethyl group.

T is a thyminyl group and U is a uracil group (wherein thyminyl group and uracil group respectively indicate thymine monovalent group and uracil monovalent group).

When T is described as a nucleic acid monomer constituting an oligonucleotide sequence, the nucleobase means a deoxyribonucleotide bonded to deoxy-D-ribose. When U is described as a nucleic acid monomer constituting an oligonucleotide sequence, the nucleobase means a ribonucleotide bonded to D-ribose.

### Example G1: Cleavage from PS1cA (n=9), PS1eA (n=9) and PS1gA (n=9)

The outline of the cleavage of oligonucleotide is shown in the following.

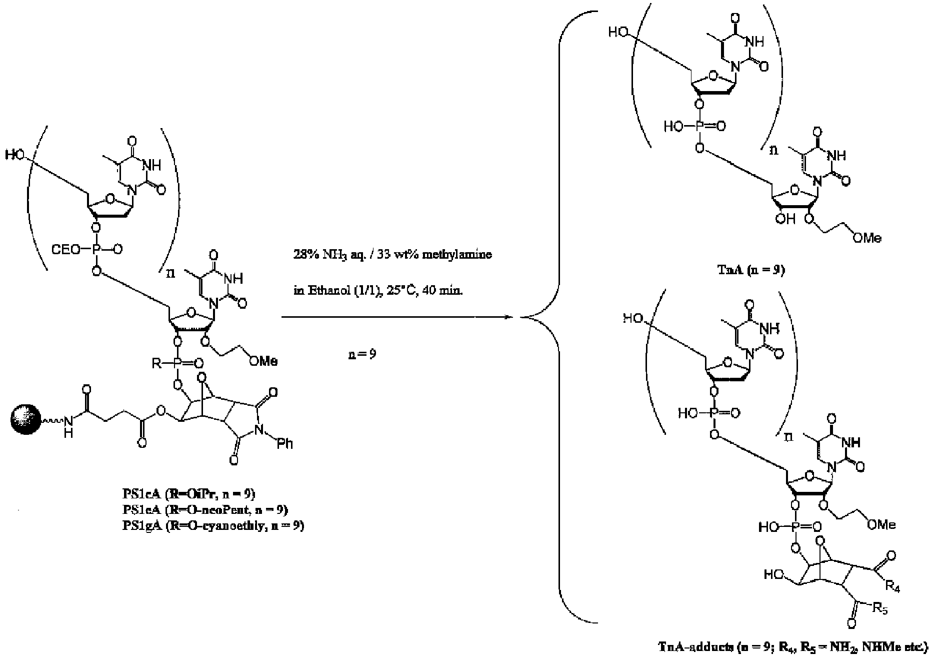

In the above-mentioned formulas, TnA (n=9) is T9-(MOE)T oligonucleotide (SEQ ID NO: 5), TnA-adduct (n=9) is an oligonucleotide in which a universal linker is bonded to the aforementioned each oligonucleotide without being cleaved.

Cleavage from PS1cA (n=9), PS1eA (n=9) and PS1gA (n=9) was performed under the following conditions. conditions: treatment with 28% aqueous ammonia/33 wt% methylamine-ethanol solution=1:1, 25°C, 40 min

After treatment under the above-mentioned respective conditions, the cleavage results were analyzed by reversed-phase HPLC. The HPLC analysis conditions are shown in the following Table 10.

**[Table 10]**

| | |
|---|---|
| eluent | SOLUTION A: 400 mmol/L HFIP, 15 mmol/L TEA |
| | SOLUTION B: MeOH |
| gradient | SOLUTION B: 15 - 35% (10 min) |
| column | ACQUITY UPLC Oligonucleotide BEH C18 (1.7 µm, 2.1×50 mm) |
| flow rate | 0.2 mL/min |
| column temperature | 40°C |
| detection | UV (260 nm) |

HPLC charts showing the cleavage results of oligonucleotides from PS1cA (n=9), PS1eA (n=9) and PS1gA (n=9) are shown in Fig. 6.

The structure of the oligonucleotide (TnA) of interest which is released upon cleavage from each solid phase was confirmed by LC-MS analysis. The results of the MS analysis are shown in the following Table 11.

**[Table 11]**

| TnA | molecular formula | theoretical value | measured value |
|---|---|---|---|
| cleaved from PS1cA (n=9) | C₁₀₃H₁₃₇N₂₀O₇₀P₉ | 3051.53 | 3051.55 |
| cleaved from PS1eA (n=9) | C₁₀₃H₁₃₇N₂₀O₇₀P₉ | 3051.53 | 3051.54 |
| cleaved from PS1gA (n=9) | C₁₀₃H₁₃₇N₂₀O₇₀P₉ | 3051.53 | 3051.54 |

In addition, the production ratio (HPLC area ratio; TnA:TnA-adducts) of the oligonucleotide (TnA) of interest and the by-produced oligonucleotide (TnA-adducts) which are released upon cleavage from each solid phase is shown in the following Table 12.

**[Table 12]**

| | R | HPLC area ratio |
|---|---|---|
| PS1cA (n=9) | -OiPr | 98:2 |
| PS1eA (n=9) | -OCH₂C(CH₃)₃ | 97:3 |
| PS1gA (n=9) | -OCH₂CH₂CN | 38:62 |

In oligonucleotide synthesis, phosphoramidites (compound: MOE-T amidite commercially available product, nucleotide derivative 12g) having a cyanoethoxy group as a protecting group for the phosphoric acid moiety have been widely used. However, when synthesizing oligonucleotides by using a universal support conventionally used widely, the use of the nucleotide derivatives (I) of the present invention (corresponding to nucleotide derivatives 12c, 12e), having a more stable substituent such as an alkoxy group as a protecting group for the phosphoric acid moiety, as a phosphoramidite strikingly increased the production of TnA oligonucleotides, and a superior effect was also confirmed that the oligonucleotides can be released from the solid phase support under mild base conditions even when MOE having modified 2' moiety of sugar moiety was used.

### Example G2: Cleavage from PS1cB - E (n=1), PS1cB - E (n=9), PS1gB - E (n=1) and PS1gB - E (n=9)

The outline of the cleavage of oligonucleotide is shown in the following.

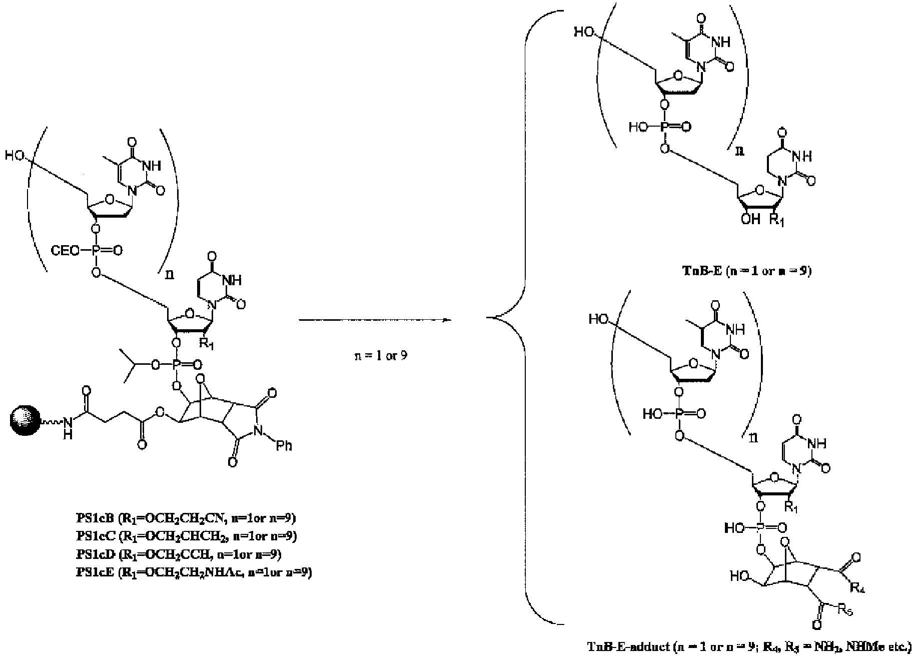

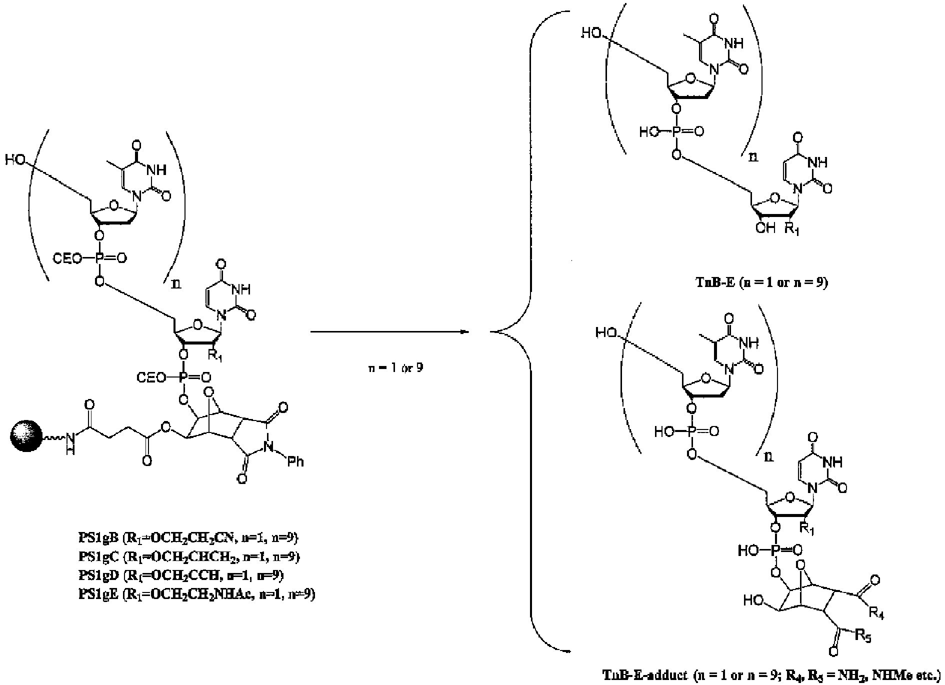

In the above-mentioned formulas, TnB - E (n=1) is T1-(2'-alkoxy)U oligonucleotide,
TnB - E (n=9) is T9-(2'-alkoxy)U oligonucleotide (SEQ ID NO: 6 - 9), and
TnB - E-adduct (n=1, or n=9) is an oligonucleotide in which a universal linker is bonded to the aforementioned each oligonucleotide without being cleaved.

Cleavage from PS1cB - E (n=1), PS1cB - E (n=9), PS1gB - E (n=1), and PS1gB - E (n=9) was performed under the following conditions.
conditions:
treatment with 28% aqueous ammonia/33 wt% methylamine-ethanol solution=1:1, 25°C, 40 min

After treatment under the above-mentioned respective conditions, the cleavage results were analyzed by reversed-phase HPLC. The HPLC analysis conditions are shown in the following Table 13 (HPLC analysis conditions for TnB - D (n=1)), Table 14 (HPLC analysis conditions for TnE (n=1)), and Table 15 (HPLC analysis conditions for TnB - E (n=9)).

**[Table 13]**

| | |
|---|---|
| eluent | SOLUTION A: 5 mmol/L TEAA (pH 7.0) |
| | SOLUTION B: 50% MeCN in 5 mmol/L TEAA |
| gradient | SOLUTION B: 0 - 100% (20 min) |
| column | ACQUITY UPLC Oligonucleotide BEH C18 (1.7 µm, 2.1×50 mm) |
| flow rate | 0.2 mL/min |
| column temperature | 40°C |
| detection | UV (260 nm) |

**[Table 14]**

| | |
|---|---|
| eluent | SOLUTION A: 5 mmol/L TEAA (pH 7.0) |
| | SOLUTION B: 50% MeCN in 5 mmol/L TEAA |
| gradient | SOLUTION B: 0 - 30% (20 min) |
| column | ACQUITY UPLC Oligonucleotide BEH C18 (1.7 µm, 2.1×50 mm) |
| flow rate | 0.2 mL/min |
| column temperature | 40°C |
| detection | UV (260 nm) |

**[Table 15]**

| | |
|---|---|
| eluent | SOLUTION A: 400 mmol/L HFIP, 15 mmol/L TEA |
| | SOLUTION B: MeOH |
| gradient | SOLUTION B: 15 - 35% (10 min) |
| column | ACQUITY UPLC Oligonucleotide BEH C18 (1.7 µm, 2.1×50 mm) |
| flow rate | 0.2 mL/min |
| column temperature | 40°C |
| detection | UV (260 nm) |

HPLC charts showing the cleavage results from 1) PS1cB (n=1) and PS1gB (n=1), 2) PS1cC (n=1) and PS1gC (n=1), 3) PS1cD (n=1) and PS1gD (n=1), 4) PS1cE (n=1), and PS1gE (n=1), 5) PS1cB (n=9) and PS1gB (n=9), 6) PS1cC (n=9) and PS1gC (n=9), 7) PS1cD (n=9) and PS1gD (n=9), and 8) PS1cE (n=9) and PS1gE (n=9) are respectively shown in 1) Fig. 7, 2) Fig. 8, 3) Fig. 9, 4) Fig. 10, 5) Fig. 11, 6) Fig. 12, 7) Fig. 13, and 8) Fig. 14.

The structures of the oligonucleotides (TnB - E) of interest which are released upon cleavage from respective solid phases were confirmed by LC-MS analysis. The results of the MS analysis are shown in Table 16.

**[Table 16]**

| TnB~E | molecular formula | theoretical value | measured value |
|---|---|---|---|
| cleaved from PS1cB (n=1) | C₂₂H₂₈N₅O₁₃P | 600.13 | 600.13 |
| cleaved from PS1gB(n=1) | C₂₂H₂₈N₅O₁₃P | 600.13 | 600.13 |
| cleaved from PS1cC(n=1) | C₂₂H₂₉N₄O₁₃P | 587.14 | 587.15 |
| cleaved from PS1gC(n=1) | C₂₂H₂₉N₄O₁₃P | 587.14 | 587.15 |
| cleaved from PS1cD(n=1) | C₂₂H₂₇N₄O₁₃P | 585.12 | 585.12 |
| cleaved from PS1gD(n=1) | C₂₂H₂₇N₄O₁₃P | 585.12 | 585.12 |
| cleaved from PS1cE(n=1) | C₂₄H₃₄N₅O₁₄P | 646.18 | 646.19 |
| cleaved from PS1gE(n=1) | C₂₄H₃₄N₅O₁₄P | 646.18 | 646.19 |
| cleaved from PS1cB (n=9) | C₁₀₂H₁₃₂N₂₁O₆₉P₉ | 3032.50 | 3032.60 |
| cleaved from PS1gB (n=9) | C₁₀₂H₁₃₂N₂₁O₆₉P₉ | 3032.50 | 3032.50 |
| cleaved from PS1cC (n=9) | C₁₀₂H₁₃₃N₂₀O₆₉P₉ | 3019.51 | 3019.59 |
| cleaved from PS1gC (n=9) | C₁₀₂H₁₃₃N₂₀O₆₉P₉ | 3019.51 | 3019.60 |
| cleaved from PS1cD (n=9) | C₁₀₂H₁₃₁N₂₀O₆₉P₉ | 3017.49 | 3017.47 |
| cleaved from PS1gD (n=9) | C₁₀₂H₁₃₁N₂₀O₆₉P₉ | 3017.49 | 3017.47 |
| cleaved from PS1cE (n=9) | C₁₀₄H₁₃₈N₂₁O₇₀P₉ | 3078.55 | 3078.66 |
| cleaved from PS1gE (n=9) | C₁₀₄H₁₃₈N₂₁O₇₀P₉ | 3078.55 | 3078.66 |

In addition, the production ratio (HPLC area ratio; TnB - E:TnB - E-adduct) of the oligonucleotide (TnB - E) of interest and the by-produced oligonucleotide (TnB - E-adduct) which are released upon cleavage from each solid phase is shown in the following Table 17.

**[Table 17]**

| | R | R₁ | HPLC area ratio |
|---|---|---|---|
| PS1cB (n=1) | -OiPr | -OCH₂CH₂CN | 100:0 |
| PS1gB (n=1) | -OCH₂CH₂CN | -OCH₂CH₂CN | 64:36 |
| PS1cC (n=1) | -OiPr | -OCH₂CHCH₂ | 100:0 |
| PS1gC (n=1) | -OCH₂CH₂CN | -OCH₂CHCH₂ | 53:47 |
| PS1cD (n=1) | -OiPr | -OCH₂CCH | 100:0 |
| PS1gD (n=1) | -OCH₂CH₂CN | -OCH₂CCH | 73:27 |
| PS1cE (n=1) | -OiPr | -OCH₂CH₂CH₂NHAc | 100:0 |
| PS1gE (n=1) | -OCH₂CH₂CN | -OCH₂CH₂CH₂NHAc | 16:84 |
| PS1cB (n=9) | -OiPr | -OCH₂CH₂CN | 97:3 |
| PS1gB (n=9) | -OCH₂CH₂CN | -OCH₂CH₂CN | 61:39 |
| PS1cC (n=9) | -OiPr | -OCH₂CHCH₂ | 99:1 |
| PS1gC (n=9) | -OCH₂CH₂CN | -OCH₂CHCH₂ | 34:66 |
| PS1cD (n=9) | -OiPr | -OCH₂CCH | 96:4 |
| PS1gD (n=9) | -OCH₂CH₂CN | -OCH₂CCH | 81:19 |
| PS1cE (n=9) | -OiPr | -OCH₂CH₂CH₂NHAc | 100:0 |
| PS1gE (n=9) | -OCH₂CH₂CN | -OCH₂CH₂CH₂NHAc | 33:67 |

In oligonucleotide synthesis, phosphoramidites (compound: corresponding to nucleotide derivatives 14g, 16g, 20g, 25g) having a cyanoethoxy group as a protecting group for the phosphoric acid moiety have been widely used. However, when synthesizing oligonucleotides by using a universal support conventionally used widely, the use of the nucleotide derivatives (I) of the present invention (corresponding to nucleotide derivatives 14c, 16c, 20c, 25c), having a more stable substituent such as an alkoxy group as a protecting group for the phosphoric acid moiety, as a phosphoramidite strikingly increased the production of TnB - E oligonucleotides, and a superior effect was also confirmed that the oligonucleotides can be released from the solid phase support under mild base conditions even when nucleotides having 2' moiety of sugar moiety modified with various functional groups were used.

The corresponding Table between the sequence Nos and the base sequences described in the above-mentioned Examples is shown below.

**[Table 18]**

| sequence No. | base sequence |
|---|---|
| 1 | T T T T T T T T T T |
| 2 | T T T T T T T T T U^{OMe} |
| 3 | T T T T T T T T T T^{LNA} |
| 4 | G G A T G T T C T C G T |
| 5 | T T T T T T T T T T^{0-(methoxyethyl)} |
| 6 | T T T T T T T T T U^{0-(cyanoethyl)} |
| 7 | T T T T T T T T T U^{0-(allyl)} |
| 8 | T T T T T T T T T U^{0-(propargyl)} |
| 9 | T T T T T T T T T U^{0-(3-acetamidopropyl)} |

### [Industrial Applicability]

The present invention relates to a solid phase synthesis method of an oligonucleotide that enables cleavage of a synthesized oligonucleotide even under mild conditions by using a nucleoside phosphoramidite with a specific structure, and the like, and is useful in the technique fields requiring oligonucleotide synthesis (e.g., in the field of research and development of nucleic acid drugs).

This application is based on a patent application No. 2021-205491 filed in Japan (filing date: December 17, 2021), the contents of which are incorporated in full herein.

## Claims

1. A solid phase synthesis method of an oligonucleotide, comprising a step of subjecting an oligonucleotide having, at the 3'-terminus of an oligonucleotide sequence thereof, a nucleotide derivative represented by the formula (II): 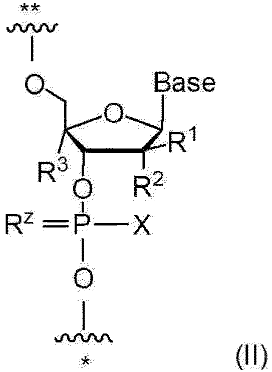
wherein * is a bonding position to the universal linker;
** is a bonding position to the nucleotide or oligonucleotide;
R^{Z} is O or S;
X is an optionally substituted alkoxy group (excluding a cyanoethoxy group),
an optionally substituted cyclic group-oxy group,
an optionally substituted alkyl group,
an optionally substituted alkenyl group, or
an optionally substituted aryl group;
R¹ is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R² is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R³ is a hydrogen atom, an optionally substituted alkyl group,
an optionally substituted alkoxy group, or an optionally substituted alkylthio group, or
R² and R³ are bonded to each other to form a group represented by the following formula (shown in the order of -R²-R³-) :
-O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-in the above-mentioned formulas, Ra to Rj are each independently a hydrogen atom or an optionally substituted alkyl group; and
Base is an optionally modified nucleobase,
which oligonucleotide is carried on a solid phase support via a universal linker and synthesized by a nucleotide elongation reaction in a solid phase synthesis method of an oligonucleotide,
to a reaction for cleaving the oligonucleotide from the solid phase support and the universal linker.

2. A solid phase synthesis method of an oligonucleotide, comprising
(1) a step of carrying a nucleotide derivative represented by the formula (I): 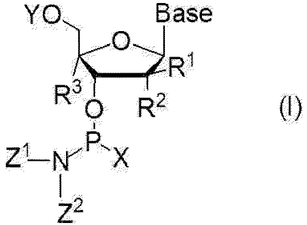
wherein X is an optionally substituted alkoxy group (excluding a cyanoethoxy group),
an optionally substituted cyclic group-oxy group,
an optionally substituted alkyl group,
an optionally substituted alkenyl group, or
an optionally substituted aryl group;
Y is a hydrogen atom or a hydroxy-protecting group;
Z¹ and Z² are each independently an optionally substituted alkyl group, or
Z¹ and Z² are bonded to each other to form, together with a nitrogen atom bonded thereto, an optionally substituted heterocycle;
R¹ is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R² is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R³ is a hydrogen atom, an optionally substituted alkyl group,
an optionally substituted alkoxy group, or an optionally substituted alkylthio group, or
R² and R³ are bonded to each other to form a group represented by the following formula (shown in the order of -R²-R³-) :
-O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
in the above-mentioned formulas, Ra to Rj are each independently a hydrogen atom or an optionally substituted alkyl group; and
Base is an optionally modified nucleobase,
on a solid phase support via a universal linker,
(2) a step of sequentially condensing nucleic acid monomers according to the desired oligonucleotide sequence to elongate the nucleotide (one or two or more nucleotide derivatives represented by the above-mentioned formula (I) may also be used as the nucleic acid monomers),
(3) a step of subjecting the obtained oligonucleotide having the desired sequence to a reaction for cleaving from the solid phase support and the universal linker.

3. The solid phase synthesis method of an oligonucleotide according to claim 1 or 2, wherein the reaction for cleaving from the solid phase support and the universal linker is performed by contacting the solid phase support, on which the oligonucleotide is carried via a universal linker, with
1) concentrated aqueous ammonia at room temperature to under warming,
2) a mixed solution of concentrated alkylamine aqueous solution/concentrated aqueous ammonia at room temperature to under warming,
3) an alcohol solution of potassium carbonate at room temperature to under warming, or
4) a mixed solution of alkylamine/water or alkylamine/water/alcohol at room temperature to under warming.

4. The solid phase synthesis method of an oligonucleotide according to claim 3, wherein the reaction for cleaving from the solid phase support is performed by contacting the solid phase support, on which the oligonucleotide is carried via a universal linker, with
1) 28% aqueous ammonia at room temperature,
2) a 1:1 mixed solution of 40% methylamine aqueous solution/28% aqueous ammonia (AMA solution) at room temperature,
3) a methanol solution of 50 mM potassium carbonate at room temperature, or
4) a mixed solution of t-butylamine/water or t-butylamine/water/methanol under warming.

5. A nucleotide derivative represented by the formula (Ia): 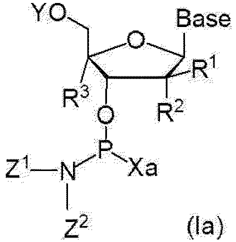
wherein Xa is an optionally substituted (C₄-C₅)alkoxy group;
Y is a hydrogen atom or a hydroxy-protecting group;
Z¹ and Z² are each independently an optionally substituted alkyl group, or
Z¹ and Z² are bonded to each other to form, together with a nitrogen atom bonded thereto, an optionally substituted heterocycle;
R¹ is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R² is a hydrogen atom, a halogen atom, an optionally protected hydroxy group, an optionally substituted alkyl group, an optionally substituted alkoxy group, an optionally substituted alkenyloxy group, or an optionally substituted alkynyloxy group;
R³ is a hydrogen atom, an optionally substituted alkyl group, an optionally substituted alkoxy group, or an optionally substituted alkylthio group, or
R² and R³ are bonded to each other to form a group represented by the following formula (shown in the order of -R²-R³-):
-O-C(Ra)₂-, -O-C(Rb)₂-C(Rc)₂-, -O-C(Rd)₂-O-, -N(Re)-C(Rf)₂-, - N(Rg)-CO-, -S-C(Rh)₂-, or -C(Ri)₂-C(Rj)₂-
in the above-mentioned formulas, Ra to Rj are each independently a hydrogen atom or an optionally substituted alkyl group; and
Base is an optionally modified nucleobase.

6. Use of a 1,2-diol derivative represented by the formula (III) :
Y¹-O-C (X¹) (X²) C (X³) (X⁴) -O-Y² (III)
wherein X¹ - X⁴ are each independently a hydrogen atom or a chain or cyclic substituent, wherein any two groups of X¹ to X⁴ are optionally bonded to each other at a bondable position to form, together with a carbon atom bonded thereto, an optionally substituted ring; and
Y¹ and Y² are each a hydrogen atom or a hydroxy-protecting group,
as a universal linker for oligonucleotide solid phase synthesis.
